(19) Europäisches Patentamt
European Patent Office
Office européen des brevets

(11) Veröffentlichungsnummer: **0 083 315**
**B1**

(12) **EUROPÄISCHE PATENTSCHRIFT**

(45) Veröffentlichungstag der Patentschrift: **29.07.87**

(21) Anmeldenummer: **82810572.6**

(22) Anmeldetag: **30.12.82**

(51) Int. Cl.[4]: **C 07 D 401/04,**
C 07 D 491/04, A 61 K 31/445
// C07D213/89 ,(C07D401/04,
213:89, 211:90),(C07D491/04,
307:00, 211:00)

(54) **Pyridyl-N-oxid-Verbindungen, Verfahren zu deren Herstellung und diese enthaltende pharmazeutische Zusammensetzungen.**

(30) Priorität: **30.12.81 CH 8359/81**
**14.04.82 CH 2255/82**

(43) Veröffentlichungstag der Anmeldung:
**06.07.83 Patentblatt 83/27**

(45) Bekanntmachung des Hinweises auf die Patenterteilung:
**29.07.87 Patentblatt 87/31**

(84) Benannte Vertragsstaaten:
**AT BE CH DE FR IT LI LU NL SE**

(56) Entgegenhaltungen:
**DE-A-1 545 873**
**DE-A-1 792 765**
**DE-A-2 616 991**
**DE-A-2 629 892**
**DE-A-2 635 916**
**FR-A-2 187 349**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Klybeckstrasse 141**
**CH-4002 Basel (CH)**

(72) Erfinder: **Zimmermann, Markus, Dr.**
**Steinbrecheweg 8**
**CH-4125 Riehen (CH)**
Erfinder: **Kühnis, Hans, Dr.**
**Lange Gasse 26**
**CH-4052 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 083 315 B1

**Beschreibung**

Die Erfindung betrifft neue Pyridyl-N-Verbindungen und deren Salze und Verfahren zu ihrer Herstellung sowie diese enthaltende pharmazeutische Zusammensetzungen und deren Verwendung.

Die erfindungsgemässen Verbindungen entsprechen der Formel

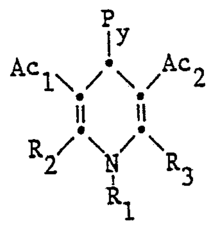

(I),

in welcher Py unsubstituiertes oder durch ($C_1$—$C_7$)-Niederalkyl, ($C_1$—$C_7$)-Niederalkoxy, ($C_1$—$C_7$)-Niederalkylthio, ($C_1$—$C_7$)-Niederalkylsulfinyl, ($C_1$—$C_7$)-Niederalkylsulfonyl und/oder Halogen substituiertes N-Oxidopyridyl bedeutet, $R_1$ Wasserstoff, ($C_1$—$C_7$)-Niederalkyl, Di-($C_1$—$C_7$)-niederalkylamino-($C_1$—$C_7$)-niederalkyl, ($C_4$—$C_6$)-Niederalkylenamino-($C_1$—$C_7$)-niederalkyl, Morpholino-($C_1$—$C_7$)-niederalkyl, Thiomorpholino-$C_1$—$C_7$)-niederalkyl, Piperazino-($C_1$—$C_7$)-niederalkyl oder 4-($C_1$—$C_7$)-Niederalkyl-piperazino-($C_1$—$C_7$)-niederalkyl darstellt, einer der Reste $R_2$ und $R_3$ ($C_1$—$C_7$)-Niederalkyl bedeutet, und der andere für Wasserstoff, ($C_1$—$C_7$)-Niederalkyl, Hydroxy-($C_1$—$C_7$)-niederalkyl, ($C_1$-$C_7$)-Niederalkoxy-($C_1$—$C_7$)-niederalkyl, gem-Di-($C_1$—$C_7$)-niederalkoxy-($C_1$—$C_7$)-niederalkyl, Halogen-($C_1$—$C_7$)-niederalkyl, Oxo-($C_1$—$C_7$)-niederalkyl, ($C_1$—$C_7$)-Niederalkoxycarbonyl-($C_1$—$C_7$)-niederalkyl, Carbamoyl-($C_1$—$C_7$)-niederalkyl, N-($C_1$—$C_7$)-Niederalkylcarbamoyl-($C_1$—$C_7$)-niederalkyl, N,N,-Di-($C_1$—$C_7$)-niederalkylcarbamoyl-($C_1$—$C_7$)-niederalkyl, Cyan-($C_1$—$C_7$)-niederalkyl, Amino-($C_1$—$C_7$)-niederalkyl, ($C_1$—$C_7$)-Niederalkylamino-($C_1$—$C_7$)-niederalkyl, Di-($C_1$—$C_7$)-niederalkylamino-($C_1$—$C_7$)-niederalkyl, ($C_4$—$C_6$)-Niederalkylenamino-($C_1$—$C_7$)-niederalkyl, Morpholino-($C_1$—$C_7$)-niederalkyl, Thiomorpholino-($C_1$—$C_7$)-niederalkyl, Piperazino-($C_1$—$C_7$)-niederalkyl, 4-($C_1$—$C_7$)-Niederalkyl-piperazino-($C_1$—$C_7$)-niederalkyl, ($C_1$—$C_7$)-Niederalkoxyimino-($C_1$—$C_7$)-niederalkyl, worin ($C_1$—$C_7$)-Niederalkoxy und Imino das gleiche Kohlenstoffatom substituierten, Hydroxyimino-($C_1$—$C_7$)-niederalkyl, ($C_1$—$C_7$)-Niederalkoxyimino-($C_1$—$C_7$)-niederalkyl, ($C_1$—$C_7$)-Niederalkoxycarbonyl, Carbamoyl, N-($C_1$—$C_7$)-Niederalkylcarbamoyl, N,N-Di($C_1$—$C_7$)-niederalkylcarbamoyl, Cyan, Amino, ($C_1$—$C_7$)-Niederalkylamino, Di-($C_1$—$C_7$)-niederalkylamino-($C_1$—$C_7$)-niederalkylamino, ($C_4$—$C_6$)-Niederalkylenamino-($C_1$—$C_7$)-niederalkylamino, Morpholino-($C_1$—$C_7$)-niederalkylamino, Di-($C_1$—$C_7$)-niederalkylamino, ($C_4$—$C_6$)-Niederalkylenamino, (2-Oxo-1-imidazolidinyl)-($C_4$—$C_6$)-niederalkylenamino, Morpholino, Thiomorpholino, Piperazino, 4-($C_1$—$C_7$)-Niederalkyl-piperazino, 4-($C_1$—$C_7$)-Niederalkanoyl-piperazino, 4-Benzoyl-piperazino, 4-Furoyl-piperazino oder 4-Thienoyl-piperazino bedeutet, wobei eine Aminogruppe $R_2$ bzw. $R_3$ auch mit einem ($C_1$—$C_7$)-Niederalkylrest $R_1$ verbunden sein und mit diesem zusammen einem 1-Aza-($C_3$—$C_4$)-niederalkylenrest bilden kann, dessen Stickstoffatom mit dem 2- bzw. 6-Ringkohlenstoffatom des 1,4-Dihydro-pyridinrings verbunden ist, oder, falls $R_2$ bzw. $R_3$ für ($C_1$—$C_7$)-Niederalkyl steht, dieses zusammen mit dem benachbarten Acylrest $Ac_1$ bzw. $Ac_2$ einen 2-Oxa-1-oxo-($C_2$—$C_3$)-niederalkylenrest bilden kann, dessen Carbonylgruppe mit dem 3- bzw. 5-Ringkohlenstoffatom des 1,4-Dihydro-pyridinrings verbunden ist, und jeder der Reste $Ac_1$ und $Ac_2$ unabhängig voneinander für ($C_1$—$C_7$)-Niederalkanoyl, unsubstituiertes oder durch ($C_1$—$C_7$)-Niederalkyl, ($C_1$—$C_7$)-Niederalkoxy und/oder Halogen substituiertes Benzoyl, ($C_1$—$C_7$)-Niederalkylsulfonyl, unsubstituiertes oder durch ($C_1$—$C_7$)-Niederalkyl, ($C_1$—$C_7$-Niederalkoxy und/oder Halogen substituiertes Phenylsulfonyl, ($C_1$—$C_7$)-Niederalkoxycarbonyl, Hydroxy-($C_1$—$C_7$)-niederalkoxycarbonyl, ($C_1$—$C_7$)-Niederalkoxy-($C_1$-$C_7$)-niederalkoxycarbonyl, Amino-($C_1$—$C_7$)-niederalkoxycarbonyl, ($C_1$—$C_7$)-Niederalkylamino-($C_1$—$C_7$)-niederalkoxycarbonyl, Di-($C_1$—$C_7$)-niederalkylamino-($C_1$—$C_7$)-niederalkoxycarbonyl, N-($C_1$—$C_7$)-Niederalkyl-N-phenyl-($C_1$—$C_7$)-niederalkyl-amino-($C_1$—$C_7$)-niederalkoxycarbonyl, ($C_4$—$C_6$)-Niederalkylenamino-($C_1$-$C_7$)-niederalkoxycarbonyl, Morpholino-($C_1$—$C_7$)-niederalkoxycarbonyl, Thiomorpholino-($C_1$—$C_7$)-niederalkoxycarbonyl, Piperazino-($C_1$—$C_7$)-niederalkoxycarbonyl, 4-($C_1$—$C_7$)-Niederalkyl-piperazino-($C_1$—$C_7$)-niederalkoxycarbonyl, unsubstituiertes oder durch ($C_1$—$C_7$)-Niederalkyl, ($C_1$—$C_7$)-Niederalkoxy und/oder Halogen substituiertes Phenyl-($C_1$—$C_7$)-niederalkoxycarbonyl, Carbamoyl, N-($C_1$—$C_7$)-Niederalkyl-carbamoyl, N,N-Di-($C_1$—$C_7$)-niederalkyl-carbamoyl, N,N-($C_4$—$C_5$)-Niederalkylencarbamoyl, Morpholino-carbonyl, Thiomorpholinocarbonyl, Piperazinocarbonyl oder 4-($C_1$—$C_7$)-Niederalkyl-piperazinocarbonyl steht, oder Salze von solchen Verbindungen mit salzbildenden Gruppen.

N-Oxido-pyridyl ist N-Oxido-2-pyridyl oder N-Oxido-4-pyridyl, in erster Linie N-Oxido-3-pyridyl, wobei diese Reste unsubstituiert oder z.B. durch ($C_1$—$C_7$)-Niederalkyl, ($C_1$—$C_7$)-Niederalkoxy, ($C_1$—$C_7$)-Niederalkylthio, ($C_1$—$C_7$)-Niederalkylsulfinyl, ($C_1$—$C_7$)-Niederalkylsulfonyl oder Halogen substituiert sein können.

Ein substituierter ($C_1$—$C_7$)-Niederalkylrest $R_1$ enthält als Substituenten in erster Linie unsubstituiertes oder insbesondere mono- oder disubstituiertes Amino, wie Di-($C_1$—$C_7$)-niederalkylamino, N-($C_1$—$C_7$)-Niederalkyl-N-phenyl-($C_1$—$C_7$)-niederalkyl-amino oder gegebenenfalls durch Sauerstoff, Schwefel oder unsubstituierten oder z.B. durch ($C_1$—$C_7$)-Niederalkyl, substituierten Stickstoff unterbrochenes ($C_4$—$C_6$)-Niederalkylenamino, wobei ein solcher Substituent vorzugsweise durch mindestens 2 Kohlenstoffatome vom Ringstickstoffatom getrennt ist.

Veräthertes Hydroxy als Substituent eines ($C_1$—$C_7$)-Niederalkylrestes $R_2$ bzw. $R_3$ ist in erster Linie ($C_1$—$C_7$)-Niederalkoxy, während verestertes Hydroxy insbesondere Halogen oder ($C_1$—$C_7$)-Niederalkanoyloxy bedeutet. Funktionell abgewandeltes Carboxy als entsprechender Substituent einer ($C_1$—$C_7$)-Niederalkylgruppe $R_2$ bzw. $R_3$ ist insbesondere verestertes Carboxy, wie ($C_1$—$C_7$)-Niederalkoxycarbonyl, amidiertes Carboxy, wie Carbamoyl, N-($C_1$—$C_7$)-Niederalkyl-carbamoyl oder N,N-Di-($C_1$—$C_7$)-niederalkylcarbamoyl, oder Cyan. Substituiertes Amino als Substituent von ($C_1$—$C_7$)-Niederalkyl $R_2$ bzw. $R_3$ ist monosubstituiertes Amino, wie ($C_1$—$C_7$)-Niederalkylamino, oder vorzugsweise disubstituiertes Amino, wie Di-($C_1$—$C_7$)-niederalkylamino, N-($C_1$—$C_7$)-Niederalkyl-N-phenyl-($C_1$—$C_7$)-niederalkyl-amino oder gegebenenfalls, z.B. durch ($C_1$—$C_7$)-Niederalkyl oder 2-Oxo-1-imidazolidinyl, substituiertes und/oder durch Sauerstoff, Schwefel oder gegebenenfalls, z.B. durch ($C_1$—$C_7$)-Niederalkyl oder Acyl, substituierten Stickstoff unterbrochenes ($C_4$—$C_6$)-Niederalkylenamino, während substituiertes Imino z.B. ($C_1$—$C_7$)-Niederalkylimino, Hydroxyimino oder O-substituiertes Hydroxyimino, z.B. ($C_1$—$C_7$)-Niederalkoxyimino, darstellt. Dabei kann ein substituierter ($C_1$—$C_7$)-Niederalkylrest $R_2$ bzw. $R_3$ einen oder mehrere, gleiche oder verschiedene Substituenten enthalten.

Funktionell abgewandeltes Carboxy und substituiertes Amino als Gruppe $R_2$ bzw. $R_3$ haben z.B. die oben gegebenen Bedeutungen und sind in erster Linie ($C_1$—$C_7$)-Niederalkoxycarbonyl oder Cyan, bzw. ($C_1$—$C_7$)-Niederalkylamino, disubstituiertes Amino-($C_1$—$C_7$)-niederalkoxycarbonyl wie Di-($C_1$—$C_7$)-niederalkylamino-($C_1$—$C_7$)-niederalkylamino oder gegebenenfalls im ($C_4$—$C_6$)-Niederalkylenteil, z.B. durch ($C_1$—$C_7$)-Niederalkyl oder 2-Oxo-1-imidazolidinyl, substituiertes und/oder durch Sauerstoff, Schwefel oder gegebenenfalls, z.B. durch ($C_1$—$C_7$)-Niederalkyl oder Acyl, substituierten Stickstoff unterbrochenes ($C_4$—$C_6$)-Niederalkylenamino-($C_1$—$C_7$)-niederalkylamino, ferner Di-($C_1$—$C_7$)-niederalkylamino, oder, gegebenenfalls im ($C_4$—$C_6$)-Niederalkylenteil, z.B. durch ($C_1$—$C_7$)-Niederalkyl, substituiertes und/oder durch Sauerstoff, Schwefel oder gegebenenfalls, z.B. durch ($C_1$—$C_7$)-Niederalkyl oder Acyl, substituierten Stickstoff unterbrochenes ($C_4$—$C_6$)-Niederalkylenamino.

Eine mit einem ($C_1$—$C_7$)-Niederalkylrest $R_1$ verbundene Aminogruppe bildet mit diesem zusammen einen, über das Stickstoffatom mit den Ringkohlenstoff verbundenen 1-Aza-($C_3$—$C_4$)-niederalkylenrest.

Ein Acylrest $Ac_1$ bzw. $Ac_2$ kann den entsprechenden Rest einer Carbonsäure, insbesondere ($C_1$—$C_7$)-Niederalkanoyl, ferner unsubstituiertes oder substituiertes, z.B. ($C_1$—$C_7$)-Niederalkyl, ($C_1$—$C_7$)-Niederalkoxy und/oder Halogen enthaltendes Benzoyl, oder einer organischen Sulfosäure, insbesondere ($C_1$—$C_7$)-Niederalkylsulfonyl oder unsubstituiertes oder substituiertes, z.B. ($C_1$—$C_7$)-Niederalkyl, ($C_1$—$C_7$)-Niederalkoxy und/oder Halogen enthaltendes Phenylsulfonyl darstellen. Acylreste $Ac_1$ und $Ac_2$ stehen jedoch in erster Linie für Acylreste von Monoestern, ferner von Monoamiden der Kohlensäure, wie unsubstituiertes oder substituiertes, z.B. freies oder veräthertes Hydroxy, wie ($C_1$—$C_7$)-Niederalkoxy, oder unsubstituiertes oder substituiertes Amino, wie z.B. oben beschriebenes Amino, und in erster Linie disubstituiertes Amino, wie Di-($C_1$—$C_7$)-niederalkylamino, N-($C_1$—$C_7$)-Niederalkyl-N-phenyl-($C_1$—$C_7$)-niederalkyl-amino, oder gegebenenfalls durch Sauerstoff, Schwefel oder unsubstituierten oder, z.B. durch ($C_1$—$C_7$)-Niederalkyl, substituierten Stickstoff unterbrochenes ($C_4$—$C_6$)-Niederalkylenamino, oder unsubstituiertes oder, z.B. durch ($C_1$—$C_7$)-Niederalkyl, ($C_1$—$C_7$)-Niederalkoxy und/oder Halogen, substituiertes Phenyl enthaltendes ($C_1$—$C_7$)-Niederalkoxycarbonyl, ferner N-substituiertes oder N-mono oder N,N-disubstituiertes Carbamoyl, wie N-($C_1$—$C_7$)-Niederalkylcarbamoyl, N,N-Di-($C_1$—$C_7$)-niederalkyl-carbamoyl, oder gegebenenfalls im Niederalkylenteil durch Sauerstoff, Schwefel oder unsubstituierten oder, z.B. durch Niederalkyl, substituierten Stickstoff unterbrochenes N,N-($C_5$—$C_6$)-Niederalkylen-carbamoyl.

Die vor-, sowie nachstehend verwendeten Allgemeinbegriffe haben, so fern nicht besonders definiert, die folgenden Bedeutungen:

Der Ausdruck "nieder" bedeutet, dass entsprechend definierte Gruppen oder Verbindungen bis und mit 7, vorzugsweise bis und mit 4 Kohlenstoffatome enthalten.

Substituierte Reste, insbesondere entsprechende Phenyl, Benzoyl, Furoyl- oder Thienoylreste, können einen oder mehrere, gleiche oder verschiedene Substituenten enthalten.

($C_1$—$C_7$)-Niederalkyl ist z.B. Methyl, Aethyl, n-Propyl, Isopropyl, n-Butyl, Isobutyl oder tert.-Butyl, ferner n-Pentyl, n-Hexyl oder n-Heptyl.

($C_1$—$C_7$)-Niederalkoxy steht in erster Linie für Methoxy, Aethoxy, n-Propyloxy, Isopropyloxy, n-Butyloxy, Isobutyloxy oder tert.-Butyloxy.

($C_1$—$C_7$)-Niederalkylthio ist z.B. Methylthio oder Aethylthio.

($C_1$—$C_7$)-Niederalkylsulfinyl ist z.B. Methylsulfinyl oder Aethylsulfinyl.

($C_1$—$C_7$)-Niederalkylsulfonyl ist z.B. Methylsulfonyl oder Aethylsulfonyl.

Halogen hat vorzugsweise eine Atomnummer bis 35 und ist insbesondere Chlor, ferner Fluor oder Brom, kann jedoch auch Iod sein.

Di-($C_1$—$C_7$)-niederalkylamino ist z.B. Dimethylamino, N-Aethyl-N-methyl-amino oder Diäthylamino.

Phenyl-($C_1$—$C_7$)-niederalkyl ist z.B. Benzyl oder -1 oder 2-Phenyläthyl. Somit ist N-($C_1$—$C_7$)-Niederalkyl-N-phenyl-($C_1$—$C_7$)-niederalkyl-amino z.B. N-Benzyl-N-methyl-amino oder N-methyl-N-(2-phenyläthyl)-amino.

($C_4$—$C_6$)-Niederalkylenamino enthält vorzugsweise 4 bis 6 Ringkohlenstoffatome und ist z.B. Pyrrolidino oder Piperidino, während durch Sauerstoff unterbrochenes ($C_4$—$C_6$)-Niederalkylenamino z.B. Morpholino, wie 4-Morpholino, durch Schwefel unterbrochenes ($C_4$—$C_6$)-Niederalkylenamino z.B. Thiomorpholino, wie 4-Thiomorpholino, und durch gegebenenfalls, z.B. durch ($C_1$—$C_7$)-Niederalkyl,

substituierten Stickstoff unterbrochenes ($C_4$—$C_6$)-Niederalkylenamino z.B. Piperazino oder 4-Methylpiperazino darstellen kann.

($C_1$—$C_7$)-Niederalkylimino ist z.B. Methylimino oder Aethylimino, während ($C_1$—$C_7$)-Niederalkoxyimino z.B. Methoxyimino, Aethoxyimino oder tert.-Butyloxyimino darstellt.

($C_1$—$C_7$)-Aminoniederalkylgruppen $R_1$ sind in erster Linie 2-disubstituiertes ($C_1$—$C_7$)-Aminp-niederalkyl, wie 2-Di-($C_1$—$C_7$)-niederalkylaminoäthyl, z.B. 2-Dimethylaminoäthyl oder 2-Diäthylaminoäthyl, 2-($C_4$—$C_6$)-Niederalkylenaminoäthyl, z.B. 2-Pyrrolidino-äthyl oder 2-Piperidino-äthyl, 2-(4-Morpholino)-äthyl, oder 2-(4-Niederalkyl-piperazino)-äthyl, z.B. 2-(4-Methylpiperazino)-äthyl.

($C_1$—$C_7$)-Niederalkanoyloxy ist z.B. Acetyloxy oder Propionyloxy.

($C_1$—$C_7$)-Niederalkoxycarbonyl ist z.B. Methoxycarbonyl, Aethoxycarbonyl, n-Propyloxycarbonyl, Isopropyloxycarbonyl, n-Butyloxycarbonyl oder tert.-Butyloxycarbonyl.

Phenyl-($C_1$—$C_7$)-niederalkoxycarbonyl ist z.B. Benzyloxycarbonyl oder 2-Phenyl-äthoxycarbonyl.

N-($C_1$—$C_7$)-Niederalkyl-carbamoyl ist z.B. N-Methyl-carbamoyl oder N-Aethyl-carbamoyl, während N,N-Di-($C_1$—$C_7$)-niederalkyl-carbamoyl z.B. N,N-Dimethyl-carbamoyl oder N,N-Diäthyl-carbamoyl ist.

($C_1$—$C_7$)-Niederalkylamino ist z.B. Methylamino, Aethylamino, n-Propylamino oder Isopropylamino.

Durch 2-Oxo-1-imidazolidinyl substituiertes ($C_4$—$C_6$)-Niederalkylenamino ist z.B. 4-(2-Oxo-1-imidazolidinyl)-piperidino.

Acyl als Substituent des, einen ($C_4$—$C_6$)-Niederalkylenaminorest unterbrechenden Stickstoffatoms ist in erster Linie der Acylrest einer Carbonsäure, wie ($C_1$—$C_7$)-Niederalkanoyl, z.B. Acetyl oder Propionyl, Benzoyl, Furoyl, z.B. 2-Furoyl, oder Thienoyl, z.B. 2-Thienoyl. Ein entsprechender Aza-($C_4$—$C_6$)-niederalkylenaminorest ist z.B. 4-($C_1$—$C_7$)-Niederalkanoyl-piperazino, 4-Benzoyl-piperazino, 4-Furoyl-piperazino oder 4-Thienoyl-piperazino.

Substituierte ($C_1$—$C_7$)-Niederalkylreste $R_2$ bzw. $R_3$ sind in erster Linie entsprechend substituierte Methylrest, z.B. Hydroxymethyl, ($C_1$—$C_7$)-Niederalkoxymethyl, Halogenmethyl, ($C_1$—$C_7$)-Niederalkoxycarbonylmethyl oder Cyanmethyl.

Di-($C_1$—$C_7$)-niederalkylamino-($C_1$—$C_7$)-niederalkylamino ist z.B. 2-Dimethylaminoäthyl-amino, 2-Diäthylaminoäthyl-amino oder 3-Dimethylaminopropyl-amino, während entsprechend durch Sauerstoff, Schwefel oder gegebenenfalls substituierten Stickstoff unterbrochenes ($C_4$—$C_6$)-Niederalkylenamino-($C_1$—$C_7$)-niederalkylamino z.B. 2-Pyrrolidinoäthyl-amino, 2-Piperidinoäthyl-amino, 2-(4-Morpholino)-äthylamino oder 2-(4-Methyl-piperazino)-äthyl-amino darstellt.

Ein 1-Aza-($C_3$—$C_4$)-niederalkylenrest, den eine Aminogruppe $R_2$ bzw. $R_3$ zusammen mit einem Niederalkylenrest $R_1$ bilden kann, ist in erster Linie 1-Aza-1,3-propylen, ferner 1-Aza-1,4-butylen, wobei in einem solchen Rest das Aza-Stickstoffatom mit dem Ringkohlenstoffatom und das Verknüfungskohlenstoffatom mit dem Ringstickstoffatom des 1,4-Dihydro-pyridinrings verbunden ist.

Ein durch einen ($C_1$—$C_7$)-Niederalkylrest $R_2$ bzw. $R_3$ zusammen mit dem benachbarten Acylrest $Ac_1$ bzw. $Ac_2$ gebildetes 2-Oxa-1-oxo-($C_2$—$C_3$)-niederalkylen ist in erster Linie 2-Oxa-1-oxo-1,3-propylen, wobei dessen 3-Kohlenstoffatom mit dem 2- bzw. 6-Ringkohlenstoffatom und dessen Carbonylgruppe mit dem 3- bzw. 5-Ringkohlenstoffatom des 1,4-Dihydro-pyridinrings verbunden ist.

In einem substituierten ($C_1$—$C_7$)-Niederalkoxycarbonyl ist der Substituent üblicherweise durch mindestens 2, vorzugsweise durch 2 oder 3 Kohlenstoffatome vom Sauerstoff getrennt; solche Reste sind z.B. Hydroxy-($C_1$—$C_7$)-niederalkoxycarbonyl, wie 2-Hydroxyäthoxycarbonyl oder 2,3-Dihydroxy-propyloxycarbonyl, ($C_1$—$C_7$)-Niederalkoxy-($C_1$—$C_7$)-niederalkoxycarbonyl, z.B. 2-Methoxy-äthoxycarbonyl, Di-($C_1$—$C_7$)-niederalkylamino-($C_1$—$C_7$)-niederalkoxycarbonyl, z.B. 2-Di-methylamino-äthoxycarbonyl, 2-Diäthylaminoäthoxycarbonyl oder 3-Di-methylaminopropyloxycarbonyl, ($C_4$—$C_6$)-Niederalkylenamino-($C_1$—$C_7$)-niederalkoxycarbonyl, z.B. 2-Pyrrolidinoäthoxycarbonyl oder 2-piperidinoäthoxycarbonyl, Morpholino-($C_1$—$C_7$)-niederalkoxycarbonyl, z.B. 2-(4-Morpholino)-äthoxycarbonyl, oder N-($C_1$—$C_7$)-Niederalkyl-piperazino-($C_1$—$C_7$)-niederalkoxycarbonyl, z.B. 2-(4-Methyl-piperazino)-äthoxy-carbonyl.

N,N-($C_4$—$C_5$)-Niederalkylen-carbamoyl ist z.B. Pyrrolidinocarbonyl oder Piperidinocarbonyl, wobei entsprechende Reste, in welchen der ($C_4$—$C_5$)-Niederalkylenteil durch Sauerstoff, Schwefel oder unsubstituierten oder substituierten Stickstoff unterbrocken ist, z.B. 4-Morpholino-carbonyl, 4-Thiomorpholinocarbonyl, 1-Piperazinocarbonyl oder 4-Methyl-1-piperazino-carbonyl bedeutet.

Verbindungen der Formel I mit salzbildenden Gruppen, insbesondere entsprechenden basischen Gruppen, können in Form von Salzen, insbesondere von Säureadditionssalzen, in erster Linie entsprechenden pharmazeutisch verwendbaren Säureadditionssalzen vorliegen. Solche Salze sind z.B. diejenigen mit Halogenwasserstoffsäuren, z.B. Chlorwasserstoffsäure oder Bromwasserstoffsäure, ferner Salpetersäure, Schwefelsäure oder Phosphorsäure, oder organischen Säuren, wie Carbonsäuren, z.B. Essigsäure, Propionsäure, Glykolsäure, Bernsteinsäure, Meleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Amino-salicylsäure, 2-Phenoxybenzoesäure, 2-Acetoxybenzoesäure, Embonsäure, Nicotinsäure oder Isonicotinsäure, ferner Aminosäuren, oder organischen Sulfonsäuren, wie gegebenenfalls Hydroxy enthaltende Niederalkansulfonsäuren, z.B. Methansulfonsäure, Aethansulfonsäure, 2-Hydroxyäthansulfonsäure oder Aethan-1,2-disulfonsäure, oder Arylsulfonsäure, z.B. Bensolsulfonsäure, 4-Methylbenzolsulfonsäure oder Naphthalin-2-sulfonsäure, oder anderen sauren organischen Substanzen, wie Ascorbinsäure.

Je nach Art der Substitution bzw. Substituenten können die Verbindungen der Formel I in Form von

Gemischen von Racematen, von Racematen oder von optisch aktiven Antipoden vorliegen.

Die Verbindungen der Formel I und Salze von solchen Verbindungen mit salzbildenden Eigenschaften besitzen wertvolle pharmakologische Eigenschaften, vor allem im cardiovasculären Bereich. So wirken solche Verbindungen mit Ausnahme derer, worin $R_2$ bzw. $R_3$ für ($C_1$—$C_7$)-Niederalkyl steht, und dieses zusammen mit dem benachbarten Acylrest $Ac_1$ bzw. $Ac_2$ einen 2-Oxa-1-oxo-($C_2$—$C_3$)-niederalkylenrest bildet, wobei dessen Carbonylgruppe mit dem 3- bzw. 5-Ringkohlenstoffatom des 1,4-Dihydropyridinrings verbunden ist, antihypertensiv, wie sich beispielsweise an der renalhypertonischen Ratte in Anlehnung an die von Byrom und Wilson, J. Physiol. (London), Bd. 93, S. 301 (1938), Gerold et al., Helv. Physiol, Pharmacol. Acta, Bd. 24, S. 58 (1966), und Goldblatt et al., J. Exptl. Med., Bd. 59, S. 347 (1934) beschriebene Testanordnung in Dosen ab etwa 20 mg/kg p.o. zeigen lässt.

So bewirkt z.B. die über 4 Tage erfolgende orale Verabreichung einer Tagesdosis von jeweils 20 mg/kg, 2,6-Dimethyl-4-(1-oxido-2-methyl-2-pyridyl)-1,4-dihydro-pyridin-3,5-dicarbonsäure-diäthylester an der renalhypertonischen. Ratte eine Blutdrucksenkung von 53mm Hg. Die blutdrucksenkende Wirksamkeit der neuen Verbindungen lässt sich ebenfalls am renalhypertonischen Hund in der Versuchsanordnung von Goldblatt et al (loc. cit.) in oralen Tagesdosen von etwa 1mg/kg an, sowie an der narkotisierten Katze bei intravenöser Verabreichung von Dosen ab etwa 0,01 mg/kg nachweisen. Die neuen Verbindungen der Formel I zeichnen sich ferner dadurch aus, dass sie im Gegensatz zu bekannten Substanzen gleicher Wirkungsrichtung und ähnlicher Struktur als unerwünschte Nebenwirkung einen höchstens marginalen negativ inotropen Effekt aufweisen. Ein solcher lässt sich beispielsweise als eine, erst bei relativ hohen Konzentrationen, etwa einer $EC_{50}$ von über 20 µMol auftretende, Hemmung der Kontraktionskraft des isolierten Meerschweinchenvorhofs nachweisen.

Die neuen Verbindungen zeichnen sich deshalb in erster Linie durch das praktische Fehlen von unerwünschten Nebenwirkungen, wie einer verminderten Herzleistung, ferner durch verhältnismässig raschen Eintritt der pharmakologischen Wirkungen, durch chemische Beständigkeit, gute Löslichkeit und, im Vergleich zu den cardiovasculären Wirkungen, durch niedrige Toxizitäten aus.

Die Verbindungen der Formel I und Salze von solchen Verbindungen mit salzbildenden Eigenschaften können daher, z.B. als Antihypertensiva und als Coronardilatoren, zur Behandlung von cardiovasculären Krankheitszuständen, wie hohen Blutdruck, Gefässpasmen, Angina pectoris und ihre Folgen und Herzinsuffizienz Verwendung finden. Verbindungen der Formel I, worin $R_2$ bzw. $R_3$ für ($C_1$—$C_7$)-Niederalkyl steht, und dieses zusammen mit dem benachbarten Acylrest $Ac_1$ bzw. $Ac_2$ einen 2-Oxa-1-oxo-($C_2$—$C_3$)-niederalkylenrest bildet, wobei dessen Carbonylgruppe mit dem 3- bzw. 5-Ringkohlenstoffatom des 1,4-Dihydropyridinrings verbunden ist, bewirken eine Steigerung der myokardialen Kontraktilität. Diese kann z.B. an elektrisch gereizten Vorhöfen des Meerschweinchens in vitro nachgewiesen werden, wo diese Präparate im Konzentrations-bereich ab etwa 10 µmol/L die Kontraktionskraft steigern (allgemeine Angaben zur verwendeten Methodik: J. V. Levy; Isolated atrial preparations: Methods in Pharmacology, Vol. 1, Editor: H. Schwartz, p.p. 77—104, Appleton-Century-Crofts, New York, 1971). Aufgrund dieser Wirkungen können solche Verbindungen zur Behandlung der Herzinsuffizienz verwendet werden. Die neuen Verbindungen sind aber auch wertvolle Zwischenprodukte zur Herstellung anderer, insbesondere pharmazeutisch wirksamer Verbindungen.

Aus der DE—OS 1.545.873 sind 1,4-Dihydropyridin-3,5-dicarbonsäureester bekannt, die in 4-Stellung durch einen gegebenenfalls substituierten Pyridinring substituiert sind. Verbindungen dieser Art bewirken eine lang anhaltende Coronarerweiterung.

Die aus der DE—OS 1.792.765 bekannten Substanzen mit im wesentlichen gleicher Struktur, wie bei den in der DE—SO 1.545.873 beschriebenen Verbindungen angegeben, zeigen eine deutliche und lang anhaltende Coronarerweiterung und wirken gefässpasmolytisch durch $Ca^{++}$-antagonistische Hemmung der elektromechanischen Koppelung bei der Gefässmuskelkontraktion.

Verbindungen mit im wesentlich gleicher Struktur, wie vorhin angegeben, sind ferner in der DE—OS 2.635.916 beschrieben, wobei solche Stoffe eine starke Wirkung auf das kardiovasculäre System, ausüben und eine sehr starke antihypertonische Wirkung bei sehr schwachen Dosen zu Folge haben.

1,4-Dihydropyridinverbindungen, die in 3- und 5-Stellung verestertes Carboxy tragen und in 4-Stellung u.a. durch Pyridyl substituiert sind, werden in der DE—OS 2.629.892 als vasodilatorisch und antihypertensiv wirksame Verbindungen beschrieben. Verbindungen dieser Art sind ferner solche die mit der in 3-Stellung stehenden veresterten Carboxygruppe mit einem in 2-Stellung stehenden geeigneten Substituenten *zusammen* eine Lactongruppe der Formel

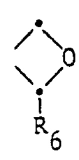

bilden, wobei solche Stoffe ebenfalls die angegebene vasodilatorisch und antihypertensive Wirkung haben sollen.

Aus der DE—OS 2.616.991 sind ferner als Coronarmittel und Antihypertensiva-verwendbare 1,4-Dihydropyridinverbindungen bekannt, die in 4-Stellung einen Pyridylrest, in 3-Stellung verestertes Carboxy und in 5-Stellung einen Schwefel-enthaltenden Substituenten tragen.

In der französischen Patentanmeldung Nr. 2.187.349 schliesslich werden intensiv kreislaufwirksame 1,4-Dihydropyridinverbindungen beschrieben, die in 3- bzw. 5-Stellung eine Ester-Keton- oder Amidgruppierung und in 4-Stellung einen Pyridylrest tragen können.

Demgegenüber zeigen die anmeldungsgemässen Verbindungen ein andersartiges Wirbungsprofil.

Die Erfindung betrifft insbesondere Verbindungen der Formel I, worin Py, $R_1$, $Ac_1$ und $Ac_2$ die unter der Formel I angegebenen Bedeutungen haben, und einer der Reste $R_2$ und $R_3$ ($C_1$—$C_7$)-Niederalkyl bedeutet, und der andere für Wasserstoff, ($C_1$—$C_7$)-Niederalkyl, Hydroxy-($C_1$—$C_7$)-niederalkyl, ($C_1$—$C_7$)-Niederalkoxy-($C_1$—$C_7$)-niederalkyl, Halogen-($C_1$—$C_7$)-niederalkyl, ($C_1$—$C_7$)-Niederalkoxycarbonyl-($C_1$—$C_7$)-niederalkyl, Carbamoyl-($C_1$—$C_7$)-niederalkyl, N-($C_1$—$C_7$)-Niederalkylcarbamoyl-($C_1$—$C_7$)-niederalkyl, N,N-Di-($C_1$—$C_7$)-niederalkylcarbamoyl-($C_1$—$C_7$)-niederalkyl, Cyan-($C_1$—$C_7$)-niederalkyl, ($C_1$—$C_7$)-Aminoniederalkyl, ($C_1$—$C_7$)-Niederalkylamino-($C_1$—$C_7$)-niederalkyl, Di-($C_1$—$C_7$)-niederalkylamino-($C_1$—$C_7$)-niederalkyl, ($C_4$—$C_6$)-Niederalkylenamino-($C_1$—$C_7$)-niederalkyl, Morpholino-($C_1$—$C_7$)-niederalkyl, Thiomorpholino-($C_1$—$C_7$)-niederalkyl, Piperazino-($C_1$—$C_7$)-niederalkyl, 4-($C_1$—$C_7$)-Niederalkyl-piperazino-($C_1$—$C_7$)-niederalkyl, ($C_1$—$C_7$)-Niederalkoxycarbonyl, Carbamoyl, N-($C_1$—$C_7$)-Niederalkylcarbamoyl, N,N-Di-($C_1$—$C_7$)-niederalkylcarbamoyl, Cyan, Amino, ($C_1$—$C_7$)-Niederalkylamino, Di-($C_1$—$C_7$)-niederalkylamino-($C_1$—$C_7$)-niederalkylamino, ($C_4$—$C_6$)-Niederalkylenamino-($C_1$—$C_7$)-niederalkylamino, Morpholino-($C_1$—$C_7$)-niederalkylamino, Di-($C_1$—$C_7$)-niederalkylamino, ($C_4$—$C_6$)-Niederalkylenamino, (2-Oxo-1-imidazolidinyl)-piperidino, Morpholino, Thiomorpholino, Piperazino, 4-($C_1$—$C_7$)-Niederalkyl-piperazino, 4-($C_1$—$C_7$)-Niederalkanoyl-piperazino, 4-Benzoyl-piperazino, 4-Furoyl-piperazino oder 4-Thienoyl-piperazino bedeutet, wobei eine Aminogruppe $R_2$ bzw. $R_3$ auch mit einem ($C_1$—$C_7$)-niederalkylrest $R_1$ verbunden sein und mit diesem zusammen einen 1-Aza-($C_3$—$C_4$)-niederalkylenrest bilden kann, dessen Stickstoffatom mit dem 2- bzw. 6-Ringkohlenstoffatom des 1,4-Dihydro-pyridinrings verbunden ist, oder, falls $R_2$ bzw. $R_3$ für ($C_1$—$C_7$)-Niederalkyl steht, dieses zusammen mit dem benachbarten Acylrest $Ac_1$ bzw. $Ac_2$ einen 2-Oxa-1-oxo-($C_2$—$C_3$)-niederalkylenrest bilden kann, dessen Carbonylgruppe mit dem 3- bzw. 5-Ringkohlenstoffatom des 1,4-Dihydro-pyridinrings verbunden ist, oder Salue von solchen verbindungen mit salzbildenden Gruppen.

Die Erfindung betrifft insbesondere Verbindungen der Formel I worin Py unsubstituiertes oder durch ($C_1$—$C_4$)-Niederalkyl oder ($C_1$—$C_7$)-Niederalkylsulfinyl substituiertes N-Oxido-pyridyl bedeutet, $R_1$ Wasserstoff, ($C_1$—$C_4$)-Niederalkyl, 2-Di-($C_1$—$C_7$)-niederalkylamino-($C_1$—$C_4$)-niederalkyl, 2-($C_4$—$C_6$)-Niederalkylenamino-($C_1$—$C_7$)-niederalkyl oder 2-(4-Morpholino)-($C_1$—$C_4$)-niederalkyl darstellt, einer der Reste $R_2$ und $R_3$ für ($C_1$—$C_4$)-Niederalkyl steht, und der andere Wasserstoff, ($C_1$—$C_4$)-Niederalkyl, Hydroxy-($C_1$—$C_7$)-niederalkyl, Halogen-($C_1$—$C_7$)-niederalkyl, ($C_1$—$C_7$)-Niederalkoxycarbonyl-($C_1$—$C_4$)-niederalkyl, ($C_1$—$C_7$)-Cyanniederalkyl, Di-($C_1$—$C_7$)-niederalkylamino-($C_1$—$C_7$)-niederalkyl, ($C_1$—$C_7$)-Niederalkoxycarbonyl, Cyan, Amino, (4-Morpholino)-($C_1$—$C_7$)-niederalkylamino, ($C_4$—$C_6$)-Niederalkylenamino, (2-Oxo-1-imidazolidinyl)-piperazino, oder 4-(2-Furoyl)-piperazino bedeutet, wobei eine Aminogruppe $R_2$ bzw. $R_3$ mit einem ($C_1$—$C_4$)-Niederalkylrest $R_1$ verbunden sein und mit diesem zusammen einen 1-Aza-Niederalkylenrest bilden kann, dessen Azastickstoffatom mit dem 2-bzw. 6-Ringkohlenstoffatom des 1,4-Dihydro-pyridinrings verbunden ist, oder, falls $R_2$ bzw. $R_3$ für ($C_1$—$C_4$)-Niederalkyl steht, dieses zusammen mit dem benachbarten Acylrest $Ac_1$ bzw. $Ac_2$ einen 2-Oxa-1-oxo-niederalkylenrest bilden kann, dessen Carbonylgruppe mit dem 3- bzw. 5-Ringkohlenstoffatom des 1,4-Diydro-pyridinrings verbunden ist, und jeder der Reste $Ac_1$ und $Ac_2$ unabhängig voneinander für Niederalkanoyl, ($C_1$—$C_7$)-Niederalkylsulfonyl, ($C_1$—$C_7$)-Niederalkoxycarbonyl, Hydroxy-($C_1$—$C_7$)-niederalkoxycarbonyl, ($C_1$—$C_4$)-Niederalkoxy-($C_1$—$C_7$)-niederalkoxy-carbonyl, Di-($C_1$—$C_7$)-niederalkylamino-($C_1$—$C_7$)-niederalkoxycarbonyl, N-($C_1$—$C_4$)-Niederalkyl-N-phenyl-($C_1$—$C_7$)-niederalkyl-amino-($C_1$—$C_7$)-niederalkoxycarbonyl, ($C_4$—$C_6$))-Niederalkylenamino-($C_1$—$C_7$)-niederalkoxycarbonyl, (4-Morpholino)-($C_1$—$C_7$)-niederalkoxycarbonyl, Carbamoyl, N-($C_1$—$C_7$)-Niederalkyl-carbamoyl, N,N-Di-($C_1$—$C_7$)-niederalkyl-carbamoyl, N,N-($C_4$—$C_5$)-Niederalkylen-carbamoyl oder 4- Morpholinocarbonyl steht, wobei Niederalkyl, Niederalkoxy und Niederalkanoyl bis und mit 4 Kohlenstoffatome, Niederalkylen 4 oder 5 Kettenkohlenstoffatome, 1-Aza-niederalkylen 2 oder 3 Kettenkohlenstoffatome und 2-Oxa-1-oxo-neideralkylen 2 Kettenkohlenstoffatome enthalten, oder Salze von solchen Verbindungen mit salzbildenden Gruppen.

Die Erfindung betrifft insbesondere Verbindungen der Formel I, worin Py für N-Oxido-pyridyl steht, $R_1$ Wasserstoff, ($C_1$—$C_4$)-Niederalkyl, 2-(Di-($C_1$—$C_7$)-niederalkylamino)-($C_1$—$C_4$)-niederalkyl, 2-($C_4$—$C_6$)-Niederalkylenamino)-($C_1$—$C_4$)-niederalkyl, oder 2-(4-Morpholino)-($C_1$—$C_4$)-niederalkyl bedeutet, einer der Reste $R_2$ und $R_3$ ($C_1$—$C_4$)-Niederalkyl darstellt, und der andere ($C_1$—$C_4$)-Niederalkyl, ($C_1$—$C_7$)-Hydroxyniederalkyl, ($C_1$—$C_7$)-Halogenniederalkyl, 2-(Di-$C_1$—$C_7$)-niederalkylamino-($C_1$—$C_4$)-niederalkyl, ($C_1$—$C_7$)-Niederalkoxycarbonyl, Cyan, Amino, (4-Morpholino)-($C_1$—$C_7$)-niederalkylamino, ($C_4$—$C_6$)-Niederalkylenamino, (2-Oxo-1-imidazolidinyl)-($C_4$—$C_6$)-niederalkylenamino oder 4-(2-Furoyl)-piperazino bedeutet, wobei eine Aminogruppe $R_2$ bzw. $R_3$ mit einem ($C_1$—$C_4$)-Niederalkylrest $R_1$ verbunden sein und zusammen mit diesem einen 1-Aza-($C_2$—$C_3$)-niederalkylenrest bilden kann, dessen Azastickstoffatom mit dem 2- bzw. 6-Ringkohlenstoffatom des 1,4-Dihydro-pyridinrings verbunden ist, oder, falls $R_2$ bzw. $R_3$ für ($C_1$—$C_4$)-Niederalkyl steht, dieses zusammen mit dem benachbarten Acylrest $Ac_1$ bzw. $Ac_2$ einen 2-Oxo-1-oxo-($C_2$)-niederalkylenrest bilden kann, dessen Carbonylgruppe mit dem 3- bzw. 5-Ringkohlenstoffatom des 1,4-Dihydro-pyridinrings verbunden ist, und jeder der Reste $Ac_1$ und $Ac_2$ unabhängig voneinander ($C_1$—$C_4$)-Niederalkanoyl, ($C_1$—$C_7$)-Niederalkylsulfonyl, ($C_1$—$C_7$)-Niederalkoxycarbonyl, 2-($C_1$—$C_4$)-Niederalkoxy-($C_1$—$C_4$)-niederalkoxy-

carbonyl, Di-($C_1$—$C_7$)-niederalkylamino-($C_1$—$C_7$)-niederalkoxy-carbonyl oder N-($C_1$—$C_4$)-Niederalkyl-N-phenyl-($C_1$—$C_4$)-niederalkyl-amino-($C_1$—$C_7$)-niederalkoxycarbonyl, Carbamoyl, N-($C_1$—$C_4$)-Niederalkyl-carbamoyl, N,N-Di-($C_1$—$C_7$)-niederalkyl-carbamoyl oder 4-Morpholino-carbonyl bedeutet, wobei Niederalkyl, Niederalkoxy und Niederalkanoyl bis und mit 4 Kohlenstoffatome, Niederalkylen 4 oder 5 Kettenkohlenstoffatome, 1-Aza-niederalkylen 2 oder 3 Kettenkohlenstoffatome und 2-Oxa-1-oxo-niederalkylen 2 Kettenkohlenstoffatome enthalten, oder Salze von solchen Verbindungen mit salzbildenden basischen Gruppen.

Die Erfindung betrifft insbesondere Verbindungen der Formel I, worin Py für N-Oxido-pyridyl steht, $R_1$ Wasserstoff oder 2-(4-Morpholino)-äthyl bedeutet, einer der Reste $R_2$ und $R_3$ Methyl darstellt, und der andere Methyl, Hydroxymethyl, ($C_1$—$C_4$)-Niederalkoxycarbonyl, x.B. Methoxycarbonyl, Aethoxycarbonyl, n-Propyloxycarbonyl, Isopropyloxycarbonyl, n-Butyloxycarbonyl, oder Isobutyloxycarbonyl, Cyan oder Amino bedeutet, und jeder der Reste $Ac_1$ und $Ac_2$ unabhängig voneinander ($C_1$—$C_4$)-Niederalkanoyl, z.B. Acetyl, ($C_1$—$C_7$)-Niederalkylsulfonyl, z.B. Aethylsulfonyl, Niederalkoxycarbonyl, z.B. Methoxycarbonyl, Aethoxycarbonyl, N-Propyloxycarbonyl, Isopropyloxycarbonyl, n-Butyloxycarbonyl oder Isobutyloxycarbonyl, Carbamoyl, N-($C_1$—$C_4$)-Niederalkyl-carbamoyl, z.B. N-Methylcarbamoyl, oder N,N-Di($C_1$—$C_4$)-niederalkyl-carbamoyl, z.B. N,N-Dimethylcarbamoyl, darstellt, wobei Niederalkyl, Niederalkoxy und Niederalkanoyl bis und mit 4 Kohlenstoffatome enthalten, oder Salze von solchen Verbindungen mit salzbildenden basischen Gruppen.

Die Erfindung betrifft insbesondere die in den Beispielen beschriebenen spezifischen Verbindungen.

Die Verbindungen der Formel I und Salze von solchen Verbindungen mit salzbildenden Eigenschaften können in an sich bekannter Weise hergestellt werden, indem man z.B.

a) eine Verbindung der Formel

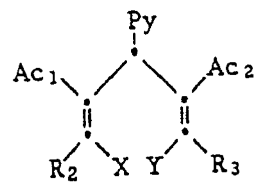

(II)

worin einer der Reste X und Y für die Gruppe der Formel —NH—$R_1$ steht und der andere Hydroxy oder die Gruppe der Formel —NH—$R_1$ bedeutet, oder ein Tautomeres davon oder ein entsprechendes Tautomerengemisch ringschliesst, oder

b) eine Verbindung der Formel Py—CHO (III) oder ein reaktionsfähiges funktionelles Derivat davon mit einer Verbindung der Formel

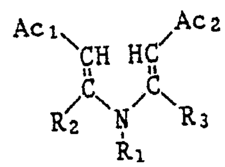

(IV)

oder einem Tautomeren davon oder einem entsprechenden Tautomerengemisch umsetzt, oder

c) in einer Verbindung der Formel

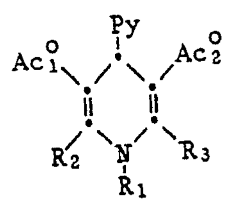

(V)

in welcher einer der Reste $Ac_1^0$ und $Ac_2^0$ einen in den Rest $Ac_1$ bzw. $Ac_2$ überführbaren Rest bedeutet, und der andere $Ac_1$ bzw. $Ac_2$ oder einen in einen $Ac_1$ bzw. $Ac_2$ überführbaren Rest bedeutet, den Rest $Ac_1^0$ und/oder $Ac_2^0$ in einen Rest $Ac_1$ und/oder $Ac_2$ überführt, oder

d) in einer Verbindung der Formel

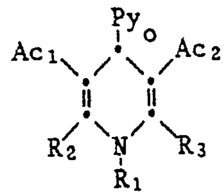

(VI)

worin $Py_o$ für einen unsubstituierten oder substituierten Pyridylrest steht, den Rest $Py_o$ zum ensprechenden N-Oxido-pyridylrest oxidiert, wobei die Bildung von Verbindungen der Formel I, worin $R_2$ bzw. $R_3$ als ($C_1$—$C_7$)-Niederalkyl zusammen mit dem benachbarten Acylrest $Ac_1$ bzw. $Ac_2$ einen 2-Oxo-1-oxo-($C_2$—$C_3$)-niederalkylenrest darstellen, dessen Carbonylgruppe mit dem 3- bzw. 5-Ringkohlenstoffatom des 1,4-Dihydropyrimidinrings verbunden ist, aus Ausgangsstoffen der Formeln II, IV, V und VI, worin $R_2$ bzw. $R_3$ freies oder verestertes Hydroxy enthaltendes ($C_1$—$C_7$)-Niederalkyl bedeutet, und $Ac_1$ bzw. $Ac_2$ oder $Ac_1^o$ bzw. $Ac_2^o$ in Ausgangsstoffen der Formel V, eine veresterte Carboxylgruppe bedeutet, unmittelbar unter den Reaktionsbedingungen eines der Verfahren a)—d) erfolgen kann, und wobei in den obigen Ausgangsstoffen der Formeln II bis VI die, sofern sie salzbildende Eigenschaften aufweisen, auch in Form ihrer Salze verwendet werden können, die Reste Py, $R_1$, $R_2$, $R_3$, $Ac_1$ und $Ac_2$ die unter der Formel I gegebenen Bedeutungen haben, und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt, und/oder, wenn erwünscht, ein erhaltenes Gemisch von Isomeren in die einzelnen Isomeren auftrennt.

Ueblicherweise werden die in der Verfahrensvariante a) verwendeten Ausgangsstoffe der Formel II *in situ* gebildet und der verfahrensgemässe Ringschluss kann unter den Reaktionsbedingungen für die Herstellung des Ausgangsmaterials stattfinden. So kann man die Ausgangsstoffe der Formel II und unter den Reaktionsbedingungen üblicherweise auch die entsprechenden Endprodukte der Formel I erhalten, indem man aa) eine Verbindung der Formel III oder ein reaktionsfähiges funktionelles Derivat davon mit einer Verbindung der Formel

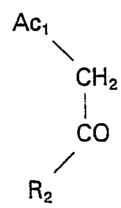

VII)

einer Verbindung der Formel

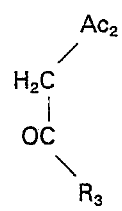

(VIII)..

und einer Verbindung der Formel $R_1$—$NH_2$ (IX) umsetzt, oder ab) eine Verbindung der Formel III oder ein reaktionsfähiges funktionelles Derivat davon mit einer Verbindung der Formel

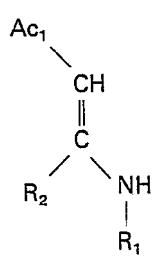

(X)

und einer Verbindung der Formel VIII umsetzt, oder ac) eine Verbindung der Formel III oder ein reaktionsfähiges funktionelles Derivat davon mit einer Verbindung der Formel

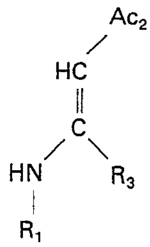

(XI)

und einer Verbindung der Formel VII oder X umsetzt, oder ad) eine Verbindung der Formel IX mit einer Verbindung der Formel

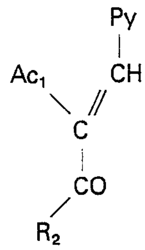

(XII)

und einer Verbindung der Formel VIII umsetzt, oder ae) eine Verbindung der Formel IX mit einer Verbindung der Formel

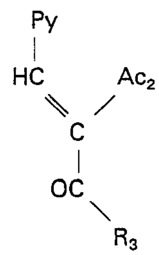

(XIII)

und einer Verbindung der Formel VII oder X umsetzt, oder af) eine Verbindung der Formel IX mit einer Verbindung der Formel

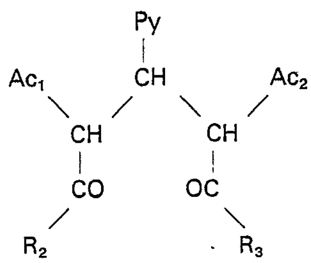

(XIV)

umsetzt, oder ag) eine Verbindung der Formel X mit einer Verbindung der Formel XIII oder der Formel

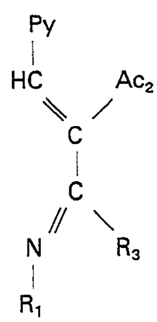

(XV)

umsetzt, oder ah) eine Verbindung der Formel XI mit einer Verbindung der Formel XII oder mit einer Verbindung der Formel

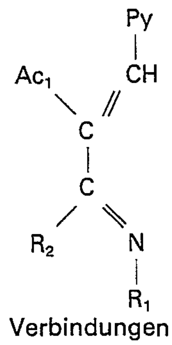

(XVI)

umsetzt. Dabei können mit Ausnahme der Verbindungen der Formelm III und IX die Verbindungen der Formelm VII, VIII und X bis XVI in Form von Tautomeren davon oder in Form von Tautomerengemischen verwendet werden; Ausgangsstoffe der obigen Formeln mit salzbildenden Eigenschaften können auch in Form von Salzen verwendet werden. Ferner haben in den obgenannten Verbindungen die Gruppen Py, $R_1$, $R_2$, $R_3$, $Ac_1$ und $Ac_2$ die im Zusammenhang mit der Formel I gegebenen Bedeutungen.

Reaktionsfähige funktionelle Derivate des Aldehyds der Formel III sind u.a. die entsprechenden Acetale, d.h. die entsprechenden Di-(veräthertes Hydroxy)-methyl-N-oxido-pyridin-Verbindungen, wie Di-($C_1$—$C_7$)-niederalkyl-, z.B. Dimethyl- oder Diäthylacetale, Acylale, z.B. die entsprechenden Di-Acyloxymethyl- oder Di-Halogen-methyl-N-oxido-pyridin-Verbindungen, wie Di-($C_1$—$C_7$)-niederalkanoylacylale, z.B. Diacetylacylale, oder die entsprechenden Dihalogen-, z.B. Dichlor- oder Dibrom-Verbindungen, ferner Additionsverbindungen, wie solche mit einem Alkalimetall-, z.B. Kaliumhydrogensulfit.

Eine Verbindung der Formel IX kann auch in Form eines, diese Verbindung in situ abgebenden Mittels, z.B. Ammoniak in Form eines Ammoniumsalzes, wie Ammoniumacetat oder Ammoniumhydrogencarbonat, oder einer Leichtmetall-, z.B. Alkalimetallverbindung, wie Natriumamid oder Lithium-N-methylamid, verwendet werden.

Bei der Ringschlussreakton a), sowie den Kondensationsreaktionen aa) bis ah) zur Herstellung des üblicherweise *in situ* gebildeten Ausgangsmaterials für die Ringschlussreaktion, handelt es sich um Varianten der Dihydropyridinsynthese von Hantzsch. Bei der Variante aa) werden insgesamt drei Moleküle Wasser abgespalten; bei weiteren Varianten tritt teilweise an die Stelle der Wasserabspaltung eine Additionsreaktion, d.h. die Wasserabspaltung erfolgt schon bei der Herstellung von einem oder von zwei Ausgangsstoffen. Bei der Umsetzung von Verbindungen der Formel III mit Verbindungen der Formelm XI und X gemäss ac), von Verbindungen der Formel X mit Verbindungen der Formel XV gemäss ag), oder von Verbindungen der Formel XI mit Verbindungen der Formel XVI gemäss ah) wird zusätzlich zu bzw. anstelle von Wasser eine Verbindung der Formel IX abgespalten. Falls nach der Variante aa) Verbindungen der Formel I hergestellt werden sollen, in denen sich $R_2$ und $R_3$ und/oder $Ac_1$ und $Ac_2$ jeweils voneinander unterscheiden, können Nebenprodukte entstehen, die in 3- und 5-Stellungen und/oder den 2- und 6-Stellungen die gleichen Substituenten enthalten. Durch nich-gleichzeitiges Zusammengeben der Reaktionsteilnehmer kann die Bildung solcher Nebenprodukte indessen herabgesetzt werden, indem man einen bestimmten Reaktionsverlauf fördert, der *in situ* gemäss einer andern Variante verläuft, da entsprechend der gestaffelten Zugabe der Reaktionskomponenten z.B. zunächst eine Verbindung der allgemeinen Formel X oder der Formel XI entstehen kann.

Die verfahrensgemässen Ringschluss- bzw. Kondensationsreaktionen werden in an sich bekannter Weise durchgeführt, falls nötwendig, in Gegenwart eines Kondensationsmittels, insbesondere eines basischen Kondensationsmittels, wie eines Ueberschusses einer basischen Reaktionskomponente oder einer zusätzlichen, z.B. organischen, Base, wie Piperidin oder Aethyl-diisopropyl-amin, oder eines Metallalkoholats, wie Alkalimetall-($C_1$—$C_7$)-niederalkanolats, oder, falls eine Verbindung der Formel IX als eine solche mit einem Leichtmetall, etwa als Natriumamid, vorliegt, in Gegenwart saurer Mittel, etwa einer organischen Carbonsäure, z.B. Essigsäure, und/oder eines geeigneten Dehydratisierungs- oder Wasseraufnehmenden Mittels, ferner üblicherweise in Gegenwart eines inerten organischen Lösungsmittels und bei Reaktionstemperaturen im Bereich von etwa Raumtemperatur bis etwa 200°C, insbesondere bei Siedetemperatur des Lösungsmittels. Gegebenenfalls erfolgt die Umsetzung in einer Inertgas-, z.B. Stickstoffatmosphäre, und/oder, z.B. bei Verwendung eines niedrigsiedenden Lösungsmittels und/oder eines Ausgangsstoffes der Formel IX, im geschlossenen Gefäss unter erhöhtem Druck.

Die in den Verfahrensvarianten verwendeten Ausgangsstoffe sind bekannt oder können nach an sich bekannten Verfahren hergestellt werden.

Die Verfahrensvariante b) wird in an sich bekannter Weise durchgeführt. Ein Derivat des Aldehyds der Formel III kann eines der oben genannten, z.B. die entsprechende Dihalogenmethyl-Verbindung sein. Die Umsetzung wird in Ab-, vorzugsweise jedoch in Anwesenheit eines Lösungs- oder Verdünnungsmittels oder eines entsprechenden Gemisches und/oder eines Kondensationsmittels durchgeführt, wobei man unter Kühlen, bei Raumtemperatur oder vorzugsweise unter Erwärmen, z.B. in einem Temperaturbereich von etwa 0° bis etwa 200°, vorzugsweise von etwa 40° bis etwa 150°, und, falls notwendig, in einem geschlossenen Gefäss, gegebenenfalls unter Druck und/oder einer Inertgasatmosphäre ableitet.

Ausgangsstoffe der Formel IV sind beispielsweise durch Umsetzung einer 3-($R_1$-Amino)-$R_2$-acrylsäure mit einer $R_3$-Carbonyl-essigsäure oder vorzugsweise einem Derivat, wie einem ($C_1$—$C_7$)-Niederalkylester

davon zugänglich; oder aber man setzt ein Gemisch einer $R_2$-Carbonyl-essigsäure und einer $R_3$-Carbonylessigsäure oder vorzugsweise einem Derivat, wie einem ($C_1$—$C_7$)-Niederalkylester davon, mit einem Amin der Formel $R_1$—$NH_2$ (IX) um, wobei die in diesen Fällen gebildeten Ausgangsstoffe *in situ*, d.h. im Reaktionsgemisch vorliegend zur weiteren Umsetzung mit dem Aldehyd der Formel III verwendet werden können.

Ausgangsstoffe der Formel V können entsprechend dem bzw. den in ihnen enthaltenen Rest(en) $Ac_2^o$ und/oder $Ac_2^o$ beispeilsweise Carbonsäuren ($Ac_1^o$ und/oder $Ac_2^o$ ist Carboxyl), Carbonsäurenanhydride, insbesondere gemischte Anhydride, wie Säurehalogenide, z.B. -chloride oder -bromide ($Ac_1^o$ und/oder $Ac_2^o$ ist Halogencarbonyl, z.B. Chlor- oder Bromcarbonyl), weiter aktivierte Ester, z.B. Cyanomethylester ($Ac_1^o$ und/oder $Ac_2^o$ ist Cyanmethoxycarbonyl) sein; diese können, gegebenenfalls in Gegenwart von Kondensationsmitteln, durch Behandeln mit einem Alkohol, wie einem unsubstituierten oder substituierten ($C_1$—$C_7$)-Niederalkanol, oder einem reaktionsfähigen Derivat davon, z.B. einem entsprechenden Alkoholat, freie Carbonsäuren auch durch Umsetzen mit geeigneten Diazo-Verbindungen, wie unsubstituierten oder substituierten Diazo-($C_1$—$C_7$)-niederalkanen, in Verbindungen der Formel I umgewandelt werden, in denen $Ac_1$ und/oder $Ac_2$ den Acylrest eines Monoesters der Kohlensäure darstellt. Solche Verbindungen können ebenfalls erhalten werden, wenn als Ausgangsstoffe Salze, insbesondere Alkalimetall- oder Erdalkalimetallsalze der freien Carbonsäuren verwendet und diese mit reaktionsfähigen Estern von Alkoholen, wie unsubstituierten oder substituierten ($C_1$—$C_7$)-Niederalkanolen, wie entsprechenden Halogeniden, z.B. Chloriden, Bromiden oder Iodiden, oder organischen Sulfonsäureestern, z.B. ($C_1$—$C_7$)-Niederalkansulfonsäure- oder Arensulfonsäureesterm, wie Methansulfonsäure- bzw. p-Toluolsulfonsäureestern, behandelt werden, oder wenn man entsprechende hydrolysierbare Iminoester, wie entsprechende Imino-($C_1$—$C_7$)-niederalkylester, zu den Estern hydrolysiert.

Die Umsetzung von freien Carbonsäuren mit Alkoholen, wie unsubstituierten oder substituierten ($C_1$—$C_7$)-Niederalkanolen erfolgt vorteilhft in Gegenwart eines sauren, wasserabspaltenden Katalysators, wie einer Protonensäure, z.B. Chlorwasserstoff-, Bromwasserstoff-, Schwefel-, Phosphor- oder Borsäure, Bensolsulfon- oder Toluolsulfonsäure, oder einer Lewissäure, z.B. von Bortrifluorid-Aetherat, in einem Ueberschuss des eingesetzten Alkohols und/oder in einem inerten Lösungsmittel, erforderlichenfalls unter destillativer, z.B. azeotroper, Entfernung des bei der Reaktion freigesetzten Wassers. Weiter kann man die Umsetzungen auch in Gegenwart von wasserbindenden Kondensationsmitteln, wie geeignet substituierten Carbodiimiden, z.B. N,N'-Diäthyl-, N,N'-Dicyclohexyl- oder N-Aethyl-N'-(3-dimethylaminopropyl)-carbodiimid, in inerten organischen Lösungsmitteln, durchführen. Gemischte Anhydride, insbesondere Säurehalogenide, werden beispeilsweise in Gegenwart säurebindender Mittel, z.B. organischer, insbesondere tertiärer Stickstoffbasen, wie Triäthylamin, Aethyldiisopropylamin oder Pyridin, oder auch anorganischer Basen, z.B. Alkalimetall- oder Erdalkalimetallhydroxiden oder -carbonaten, wie Natrium-, Kalium- oder Calciumhydroxid bzw. -carbomat, mit Alkoholen oder mit Alkoholaten, z.B. Alkalimetall-niederalkanolaten, umgesetzt.

Die Umsetzungen von reaktionsfähigen Estern, z.B. Cyanmethyl- oder Pentachlorphenylestern, mit Alkoholen werden beispeilsweise in einem gegenüber den Reaktionsteilnehmern inerten Lösungsmittel im Temperaturbereich von etwa 0°C bis etwa 120°C, vorzugsweise bei Raumtemperatur bis etwa 60°C, durchgeführt.

Die Hydrolyse von Imidoester-, insbesondere Imido-($C_1$—$C_7$)-niederalkylester-Ausgangsstoffen, erfolgt beispeilsweise mittels wasserhaltiger Mineralsäuren, wie Salzsäure oder Schwefelsäure, wobei man z.B. die bei der Addition von Chlorwasserstoff an Nitrile und Umsetzung mit wasserfreien Alkoholen, insbesondere unsubstituierten oder substituierten ($C_1$—$C_7$)-Niederalkanolen, erhaltenen Imidoester-salze, z.B. -hydrochloride, nach Zusatz von Wasser direkt zu den entsprechenden Estern hydrolysieren kann. Man kann Ester z.B. auch aus einem Gemisch von Nitril, Alkohol und Schwefelsäure mit geeignetem Wassergehalt ohne Isolierung des *in situ* entstandenen Imidoesters die gewünschte Esterverbindung der Formel I erhalten.

Verbindungen der Formel I, in denen mindestens einer der Reste $Ac_1$ und $Ac_2$ den Acylrest einen Kohlensäuremonoamids darstellt, kann man aus Verbindungen der Formel V erhalten, in denen $Ac_1^o$ und/oder $Ac_2^o$ für Carboxylgruppen, Säureanhydridgruppen, wie Halogencarbonyl, z.B. Chlorcarbonyl, oder aktivierte Estergruppen, wie Cyanmethoxycarbonyl, stehen indem man solche Ausgangsstoffe, gegebenenfalls in Gegenwart eines geeigneten Kondensationsmittels, mit Ammoniak oder einem Ammoniak abgebenden Mittel oder einem N-mono- oder N,N-disubstituierten Amin unsetzt.

Diese Umwandlungen von Carboxy- und geeignet funktionell abgewandelten reakionsfähigen Carboxygruppen in entsprechende Carbamoylgruppen werden in an sich bekannter Weise, z.B. nach den für die Bildung der Estergruppen beschriebenen Verfahren, durchgeführt.

Verbindungen der Formel I, in welchen mindestens eine der Gruppen $Ac_1$ und $Ac_2$ für Carbamoyl steht, kann man, ausgehend von Verbindungen der Formel V erhalten, in welchen einer der Reste $Ac_1$ und $Ac_2$ Cyan darstellt, ebenfalls erhalten, Solche Ausgangsstoffe können hydrolytisch, vorzugsweise unter sauren oder basischen Bedingungen, z.B. in Gegenwart eines Alkalimetall-, wie Natriumhydroxids, und, wenn erwünscht, von Wasserstoffperoxid in einem wässrigalkoholischen Lösungsmittel, wie wässrigem Aethanol, in die gewünschten Verbindungen der Formel I mit mindestens einer Carbamoylgruppe $Ac_1$ bzw. $Ac_2$ übergeführt werden.

Ausgangsstoffe der Formel V mit freier Carboxylgruppe $Ac_1^o$ und/oder $Ac_2^o$ können z.B. erhalten werden,

indem man den entsprechenden 2-Cyanoäthylester herstellt, wobei man z.B. in einem der vorstehend beschriebenen Verfahren ab) oder ag) eine Verbindung der Formel X einsetzt, in der $Ac_1$ eine 2-Cyanoäthoxycarbonylgruppe ist; z.B. kann man einen, gegebenenfalls in der Aminogruppe entsprechend der Definition für $R_1$ substituierten 3-Aminocrotonsäure-2-cyanoäthylester mit den übrigen Reaktionskomponenten unsetzen, und anschliessend die so erhaltene 2-Cyanoäthylester-Verbindung unter milden Bedingungen, z.B. mittels wässrigem oder wässerig-($C_1$—$C_7$)-niederalkanolischem 1-n. Natriumhydroxid bei Raumtemperatur, zur freien Carbonsäure spalten. Letztere kann, falls notwendig, in an sich bekannter Weise in die gewünschten reaktionsfähigen funktionellen Derivate übergeführt werden.

Die als Ausgangsstoffe für die Verfahrensvariante c) ebenfalls in Betracht kommenden Nitrilverbindungen der Formel V können z.B. analog zu einer der Verfahrensvarianten aa) bis ah) hergestellt werden, indem man Ausgangsstoffe verwendet, die anstelle des Restes $Ac_1$ und/oder $Ac_2$ eine Cyangruppe enthalten, wie beispielsweise anstelle einer Verbindung der Formel X 3-Aminocrotononitril oder dessen, den Definitionen von $R_1$ und/oder $R_2$ entsprechenden Derivate.

Die Oxidation von Verbindungen der Formel VI nach der Verfahrensvariante d) zu Verbindungen der Formel I mit einem N-Oxido-pyridylrest kann in an sich bekannter Weise durchgeführt werden, z.B. durch Behandeln mit organischen Persäuren, wie ($C_1$—$C_7$)-Niederalkanpersäuren oder Arenpersäuren, wie gegebenenfalls geeignet substituierten Perbenzoesäuren, z.B. Peressig- oder 3-Chlorperbenzoesäure, vorzugsweise bei Zimmertemperatur oder etwas darüber liegender Reaktionstemperatur, oder mit wässrigem Wasserstoffperoxid, z.B. bei Temperaturen von bis zu 100°C, in Gegenwart oder Abwesenheit von ($C_1$—$C_7$)-Niederalkansäuren, z.B. Essigsäure, durchgeführt. Dabei muss, insbesondere bei Verwendung von Persäuren, darauf geachtet werden, dass keine Ueberoxidation aufgrund einer zu langen Reaktionsdauer eintreten kann.

Die Ausgangsstoffe der Formel VI sind bekannt oder können in an sich bekannter Weise hergestellt werden, z.B. nach den obgenannten Verfahrensvarianten a) bis c), indem man diese mit Ausgangsstoffen durchführt, in denen der Rest Py durch $Py_o$ ersetzt ist.

Die obigen Reaktionen können unter an sich bekannten Reaktionsbedingungen in Ab- oder üblicherweise Anwesenheit von Lösungs- oder Verdünnungsmitteln, je nach Art der Reaktion und/oder Reaktionsteilnehmer bei erniedrigter oder erhöhter Temperatur, z.B. im Temperaturbereich von etwa −10°C bis etwa 150°C, unter atomosphärischem Druck oder in einem geschlossenen Gefäss, gegebenenfalls unter Druck, und/oder in einer inerten Atmosphäre, z.B. unter einer Stickstoffatmosphäre, durchgeführt werden.

Verfahrensgemäss erhältliche Verbindungen der Formel I können in an sich bekannter Weise in andere Verbindungen der Formel I umgewandelt werden.

So kann man z.B. in Verbindungen der Formel I, worin $R_1$ für Wasserstoff steht, durch Behandeln mit einem reaktionsfähigen Ester eines Alkohols der Formel $R_1$—OH (XVII) einen organischen Rest $R_1$ einführen und so zu Verbindungen der Formel I gelangen, worin $R_1$ von Wasserstoff verschieden ist.

Reaktionsfähige Ester von Verbindungen der Formel XVII, z.B. von unsubstituierten oder substituierten ($C_1$—$C_7$)-Niederalkanolen, sind solche mit starken, anorganischen oder organischen Säuren; dabei kommen beispielsweise die entsprechenden Halogenide, insbesondere Chloride, Bromide oder Jodide, ferner Sulfate, weiter ($C_1$—$C_7$)-Niederalkansulfonsäure- oder Arensulfonsäureester, z.B. Methansulfonsäure-, Benzolsulfonsäure- oder p-Toluolsulfonsäureester, in Betracht. Die Umsetzung wird, falls notwendig, unter Kühlen oder Erwärmen, z.B. in einem Temperaturbereich von etwa 0°C bis etwa 100°C, in Gegenwart eines geeigneten basischen Kondensationsmittels, z.B. eines Alkalimetalls, Alkalimetallamids oder -hydrids, oder eines Alkalimetallniederalkoxids, wie Natrium- oder Kalium-methoxid, -äthoxid oder tert.-butoxid, in An- oder Abwesenheit eines Lösungs- oder Verdünnungsmittels, bei erniedrigter oder erhöhter Temperatur, z.B. in einem Temperaturbereich von etwa 0°C bis etwa 100°C, und/oder unter atmosphärischem Druck oder in einem geschlossenen Gefäss durchgeführt.

Vorzugsweise verwendet man in solchen N-Substituierungsreaktionen in erster Linie Verbindungen der Formel I, die keine Hydroxy- und/oder andere Aminogruppen als Substituenten aufweisen, da diese unter Umständen ebenfalls mit dem reaktionsfähigen Ester eines Alkohols der Formel XVII in Reaktion treten können.

Ferner können in verfahrensgemäss erhältlichen Verbindungen der Formel I vorhandene Substituenten in andere Substituenten umgewandelt werden.

So kann man z.B. veresterte Carboxygruppen, wie entsprechende Gruppen $Ac_1$, $Ac_2$ und/oder $R_3$ oder entsprechende Substituenten von Resten $R_2$ und/oder $R_3$, durch Umesterung in andere Ester überführen. Dabei verwendet man vorzugsweise entsprechende Alkoholverbindungen, die einen Siedepunkt aufweisen, der deutlich über demjenigen des Alkohols der veresterten Gruppe in der umzuwandelnden Verbindung der Formel I liegt, und führt die Reaktion z.B. in einem Ueberschuss der Hydroxyverbindung und/oder einem inerten organischen, vorzugsweise einem ebenfalls deutlich über dem Alkohol der veresterten Gruppe siedenden Lösungsmittel, vorzugsweise in Gegenwart eines Katalysators, z.B. eines Alkalimetall-($C_1$—$C_7$)-niederalkoxids, wie Natrium- oder Kalium-methoxid oder -äthoxid, in der Wärme und üblicherweise unter Abdestillieren des freigesetzten Alkohols durch.

Verbindungen der Formel I mit veresterten Carboxygruppen, wie ($C_1$—$C_7$)-Niederalkoxycarbonylgruppen, insbesondere entsprechenden Gruppen $Ac_1$ und/oder $Ac_2$, können in solche mit entsprechenden Carboxamidgruppen umgewandelt werden, z.B. durch Behandeln mit Ammoniak, ferner mono- oder

disubstituierten Aminen, wenn notwendig, unter erhöhter Temperatur und/oder in einem geschlossenen Gefäss.

Verbindungen der Formel I, worin $R_2$ oder $R_3$ zusammen mit dem benachbarten Rest $Ac_1$ bzw. $Ac_2$ einen 2-Oxa-1-oxo-($C_1$—$C_7$)-niederalkylenrest, besonders einen 2-Oxa-1-oxo-1,3-propylenrest bilden, dessen Carbonylgruppe mit dem 3- bzw. 5-Ringkohlenstoffatom des 1,4-Dihydro-pyridinringes verbunden ist, können durch Ammonolyse oder Aminolyse, z.B. durch Behandeln mit Ammoniak oder einem mono- oder disubstituierten Amin, in Verbindungen der Formel I umgewandelt werden, worin $R_2$ oder $R_3$ für ($C_1$—$C_7$)-Hydroxyniederalkyl, insbesondere Hydroxymethyl steht, und die entsprechende benachbarte Gruppe $Ac_1$ bzw. $Ac_2$ eine amidierte Carboxylgruppe bedeutet.

Verbindungen der Formel I, worin einer der Reste $R_2$ und $R_3$ zusammen mit dem benachbarten Rest $Ac_1$ bzw. $Ac_2$ einen 2-Oxa-1-oxo-($C_2$—$C_3$)-niederalkylen-, besonders einen 2-Oxa-1-oxo-1,3-propylenrest bildet, dessen Carbonylgruppe mit dem 3- bzw. 5-Ringkohlenstoffatom des 1,4-Dihydro-pyridinrings verbunden ist, können z.B. durch ringschliessende Kondensation einer gegebenenfalls reaktionsfähigen veresterten ($C_1$—$C_7$)-Hydroxyniederalkylgruppe, etwa einer p-Tosyloxy- oder Acetyloxy- oder ($C_1$—$C_7$)-Halogenniederalkylgruppe $R_2$ und/oder $R_3$ mit einer benachbarten veresterten Carboxygruppe, z.B. einer ($C_1$—$C_7$)-Niederalkoxycarbonylgruppe $Ac_1$ bzw. $Ac_2$ in einer entsprechenden Verbindung der Formel I, z.B. bei erhöhter Temperatur, etwa in einem Bereich von ca. 50 bis ca. 200°C, insbesondere ca. 80 bis ca. 180°C erhalten werden.

Diese Verbindungen können jedoch auch direkt unter den Reaktionsbedingungen eines der beschriebenen Verfahren a) bis d), insbesondere bei erhöhter Temperatur, etwa wie angegeben erhalten werden. Verwendet man z.B. in der Reaktionsvariante ab) ein Ausgangsmaterial der Formel VIII, worin $R_3$ für Hydroxyniederalkyl, z.B. Hydroxymethyl, oder ($C_1$—$C_7$)-Halogenniederalkyl, z.B. Chloromethyl, steht, und $Ac_2$ eine veresterte Carboxygruppe, wie ($C_1$—$C_7$)-Niederalkoxycarbonyl, z.B. Methoxycarbonyl, bedeutet und führt die Reaktion unter erhöhter Temperatur durch etwa in einem Bereich von ca. 50 bis ca. 200°C, insbesondere ca. 80 bis ca. 180°C, so kann man direkt Verbindungen der Formel I erhalten, in denen $R_3$ und $Ac_2$ zusammen einen 2-Oxa-1-oxo-($C_2$—$C_3$)-niederalkylen-, z.B. den 2-Oxa-1-oxo-1,3-propylenrest bilden, dessen Carbonylgruppe mit dem 3-Ringkohlenstoffatom des 1,4-Dihydro-pyridinrings verbunden ist.

In einer Verbindung der Formel I, worin $R_2$ oder $R_3$ ($C_1$—$C_7$)-Hydroxyniederalkyl, z.B. Hydroxymethyl, darstellt, kann Hydroxy durch Behandeln mit einem Halogenierungsmittel, wie einem geeigneten anorganischen oder organischen Säurehalogenid z.B. Thionylchlorid, in Halogen, wie Chlor, umgewandelt werden.

Eine Verbindung der Formel I, worin $R_2$ bzw. $R_3$ ($C_1$—$C_7$)-Halogenniederalkyl, z.B. Chlormethyl, bedeutet, kann man mit einer geeigneten Cyanverbindung, wie einem Metall-, z.B. Alkalimetallcyanid, oder einem Ammoniumcyanid behandeln und so Verbindungen der Formel I erhalten, worin $R_2$ bzw. $R_3$ ($C_1$—$C_7$)-Cyanniederalkyl, z.B. Cyanmethyl, darstellt.

In Verbindung der Formel I, worin $R_2$ bzw. $R_3$ Cyan oder ($C_1$—$C_7$)-Cyanniederalkyl darstellt, kann z.B. durch Behandeln mit einem Alkohol, wie ($C_1$—$C_7$)-Niederalkanol, in Gegenwart einer Säure, wie einer Mineralsäure, z.B. Schwefelsäure, und von Wasser die Cyangruppe in eine veresterte Carboxylgruppe, wie ($C_1$—$C_7$)-Niederalkoxycarbonyl, umgewandelt werden.

Ferner kann man Verbindungen der Formel I, worin einer der Reste $R_2$ und $R_3$ für eine 2-($C_1$—$C_7$)-Aminoniederalkylgruppe, insbesondre eine 2-N,N-disubstituierte Amino-äthylgruppe steht, auch dadurch erhalten, dass man eine Verbindung der Formel I, worin einer der Reste $R_2$ und $R_3$ für ($C_1$—$C_7$)-Niederalkyl, insbesondere Methyl steht, mit Formaldehyd oder einem solchen abgebenden Mittel, wie Paraformaldehyd, und einem Amin, insbesondere einem N,N-disubstituierten Amin, nach dem Mannich-Verfahren umsetzt.

Ferner kann man Verbindungen der Formel I, worin $R_2$ bzw. $R_3$ ein am gleichen Kohlenstoffatom zwei ($C_1$—$C_7$)-Niederalkoxy, z.B. Methoxy oder Aethoxy, enthaltendes ($C_1$—$C_7$)-Niederalkyl, insbesondere Di-($C_1$—$C_7$)-niederalkoxymethyl, z.B. Dimethoxymethyl oder Diäthoxymethyl, darstellt, in Verbindungen der Formel I umwandeln, worin $R_2$ bzw. $R_3$ ein Oxo enthaltendes ($C_1$—$C_7$)-Niederalkyl, insbesondere Formyl, bedeutet. Die Umwandlung der Ketal- bzw. Acetalgruppierung in die freie Carbonylgruppe kann in an sich bekannter Weise, z.B. durch Behandeln mit einem sauren Reagens, wie einer Säure, insbesondere einer Mineralsäure, z.B. Chlorwasserstoffsäure, oder einer organischen Sulfonsäure, z.B. p-Toluolsulfonsäure, erreicht werden.

Eine Verbindung der Formel I, worin eine der Gruppen $R_2$ bzw. $R_3$ ein Oxo enthaltendes ($C_1$—$C_7$)-Niederalkyl, insbesondere Formyl, bedeutet, kann durch Reduktion, z.B. Behandeln mit einem geeigneten Hydridreduktionsmittel, wie einem Alkalimetallborhydrid, z.B. Natriumborhydrid, in eine Verbindung der Formel I umgewandelt werden, worin $R_2$ bzw. $R_3$ für ($C_1$—$C_7$)-Hydroxyniederalkyl, insbesondere Hydroxymethyl, steht. Ferner kann man sie durch Behandeln mit einem gegebenenfalls O-substituierten Hydroxylamin, wie ($C_1$—$C_7$)-Niederalkoxyamin, oder einem Säureadditionssalz davon, in eine Verbindung der Formel I umwandeln, worin $R_2$ bzw. $R_3$ ein gegebenenfalls O-substituiertes Hydroxyimino enthaltendes ($C_1$—$C_7$)-Niederalkyl, insbesondere gegebenenfalls O-substituiertes Hydroxyiminomethyl, darstellt.

In einer Verbindung der Formel I, worin $R_2$ bzw. $R_3$ ein gegebenenfalls O-substituiertes Hydroxyimino enthaltendes ($C_1$—$C_7$)-Niederalkyl darstellt, kann dieses durch Reduktion, z.B. durch Behandeln mit einem geeigneten Hydridreduktionsmittel, wie einem Alkalimetallborhydrid, z.B. Natriumcyanborhydrid, in ein

gegebenenfalls N-monosubstituiertes Amino enthaltendes ($C_1$—$C_7$)-Niederalkyl umgewandelt werden. Eine Verbindung der Formel I mit einer ($C_1$—$C_7$)-Hydroxyiminoniederalkyl-, insbesondere Hydroxyiminomethylgruppe als $R_2$ bzw. $R_3$ kann durch Dehydratisieren, z.B. durch Behandeln mit einem anorganischen Säurehalogenid, wie Phosphoroxychlorid, oder einer Carbodiimidverbindung, wie N,N'-Dicyclohexylcarbodiimid, in eine Verbindung der Formel I umgewandelt werden, worin $R_2$ bzw. $R_3$ Cyan oder ($C_1$—$C_7$)-Cyan-niederalkyl darstellt.

Je nach Reaktionsbedingungen können die Verbindungen der Formel I in freier Form oder in Form von Salzen erhalten werden.

Erhaltene Säureadditionssalze können in an sich bekannter Weise in die freien Verbindungen, z.B. durch Behandeln mit einer Base, wie einem Alkalimetallhydroxid, oder in andere Salze, z.B. durch Behandeln mit geeigneten Säuren oder Derivaten davon, umgewandelt werden. Erhaltene freie Verbindungen mit salzbildenden Eigenschaften, z.B. mit entsprechenden basischen Gruppen, können z.B. durch Behandeln mit Säuren oder entsprechenden Anionenaustauschern in ihre Salze umgewandelt werden.

Infolge der engen Beziehung zwischen den Verbindungen der Formel I in freier Form und in Form von Salzen, sind im Vorausgegangenen und nachfolgend unter den freien Verbindungen oder ihren Salzen sinn- und zweckmässig gegebenenfalls auch die entsprechenden Salze bzw. freien Verbindungen zu verstehen.

Die Verbindungen, einschliesslich ihre Salze, können auch in Form ihrer Hydrate erhalten werden, oder ihre Kristalle können z.B. das zur Kristallisation verwendete Lösungsmittel einschliessen.

Die Verbindungen der Formel I können, je nach Verfahrensreaktion und/oder Art der Ausgangsstoffe in Form von Racematen, Racematgemischen oder optischen Antipoden erhalten werden.

Erhaltene Racematgemische können auf Grund der physikalisch-chemischen Unterschiede der Bestandteile in bekannter Weise in die reinen Racemate bzw. Diastereomeren aufgetrennt werden, beispielsweise durch Chromatographie und/oder fraktionierte Kristallisation.

Racemate lassen sich nach an sich bekannten Methoden in die optischen Antipoden zerlegen, beispielsweise durch Umkristallisation aus einem optisch aktiven Lösungsmittel, mit Hilfe von geeigneten Mikroorganismen oder durch Umsetzen einer Verbindung der Formel I mit salzbildenden, z.B. basischen, Eigenschaften mit einem optisch aktiven, salzbildenden Mittel, wie einer optisch aktiven Säure, und Trennen der auf diese Weise erhaltenen Gemische von Salzen, z.B. auf Grund ihrer verschiedenen Löslichkeiten, in die diasteromeren Salze; aus diesen können die Antipoden, z.B. durch Behandeln mit einer Base, freigesetzt werden.

Optische Antipoden von neutralen Verbindungen der Formel I kann man z.B. auch gemäss Verfahren c) unter Verwendung einer optisch aktiven Säure der Formel V erhalten, wobei man diese z.B. aus der entsprechenden racemischen Säure in üblicher Weise, z.B. durch Salzbildung mit einer optisch aktiven Base, Trennung der diastereomeren Salze und Freisetzung der optisch aktiven Säure, oder unter Verwenden eines reaktionsfähigen funktionellen Derivats einer optisch aktiven Säure bilden kann.

Ferner kann man z.B. Verbindungen der Formel I, die eine veresterte Carboxygruppe, z.B. eine entsprechende Gruppe $Ac_1$ oder $Ac_2$, aufweisen, unter Verwendung eines optisch aktiven Alkohols nach dem oben beschriebenen Verfahren umestern und das so erhältliche Diastereomerengemisch, z.B. mittels fraktionierter Kristallisation, in die Antipoden auftrennen.

Vorteilhafterweise isoliert man aus einem Diastereomerengemisch bzw. Racemat das pharmakologisch aktivere Isomere bzw. den aktiveren Antipoden.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates, z.B. Salzes, und/oder Racemates bzw. Antipoden verwendet oder unter den Reaktionsbedingungen bildet.

Bei den Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Verfahren zu deren Herstellung bilden ebenfalls einen Gegenstand der vorliegenden Erfindung. Die Erfindung betrifft auch verfahrensgemäss erhältliche neue Zwischenprodukte, sowie Verfahren zu ihrer Herstellung.

Die Erfindung betrifft ebenfalls die Verwendung der Verbindungen der Formel I oder von pharmazeutisch verwendbaren Salzen von solchen Verbindungen mit salzbildenden Eigenschaften, insbesondere als pharmakologisch, insbesondere im cardiovasculären Bereich, aktive Verbindungen. Dabei kann man sie, vorzugsweise in Form von pharmazeutischen Präparaten, in einem Verfahren zur prophylaktischen und/oder therapeutischen Behandlung von Hypertonie einerseits und von anderen cardiovasculären Krankeiten, z.B. Herzinsuffizienz andererseits, verwenden. Die Dosierung des Wirkstoffs, der allein oder zusammen mit dem üblichen Träger- und Hilfsmaterial verabreicht, wird, hängt von der zu behandelnden Spezies, deren Alter und individuellen Zustand, sowie der Verabreichungsweise ab. Die täglichen Dosen der antihypertensiv wirkenden Verbindungen liegen für Mammalien mit einem Körpergewicht von etwa 70 kg, je nach Art der Erkrankung, individuellem Zustand und Alter, vorzugsweise zwischen 5 und 200 mg, während die täglichen Einzeldosen für die die myocardiale Kontraktilität erhöhenden Verbindungen in einem Bereich von etwa 100—600 mg liegen.

Die Erfindung betrifft im weiteren pharmazeutische Präparate, die Verbindungen der Formel I oder

pharmazeutisch verwendbare Salze von solchen Verbindungen mit salzbildenden Eigenschaften als Wirkstoffe enthalten, sowie Verfahren zu ihrer Herstellung.

Bei den erfindungsgemässen pharmazeutischen Präparaten handelt es sich um solche zur enteralen, wie peroralen oder rektalen, weiter zur sublingualen, sowie zur parenteralen Verabreichung an Warmblüter. Entsprechende Dosiseinheitsformen, insbesondere zur peroralen und/oder sublingualen Verabreichung, z.B. Dragées, Tabletten oder Kapseln, enthalten vorzugsweise von etwa 2,5 bis etwa 100 mg, insbesondere von etwa 5 bis etwa 25 mg einer Verbindung der Formel I oder eines pharmazeutisch annehmbaren Salzes einer zur Salzbildung befähigten entsprechenden Verbindung zusammen mit pharmazeutisch verwendbaren Trägerstoffen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyäthylenglykol und/oder Titandioxid enthalten, oder Lacklösungen in geeigneten organischen Lösungsmitteln oder Lösungsmittelgemischen, oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Lactose, Bindemitteln, wie Stärken und/oder Gleitmitteln, wie Talk oder Magnesiumstearat, und gegebenenfalls von Stabilisatoren, enthalten. In weichen Kapseln ist der Wirksstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können. Bevorzugt sind u.a. Kapseln, die sowohl leicht zerbissen werden können, um z.B. bei Anzeichen eines Anfalls von Angina pectoris durch sublinguale Aufnahme des Wirkstoffes eine möglichst rasche Wirkung zu erzielen, als auch unzerkaut geschluckt werden können.

Als rektal anwendbare pharmazeutische Präparate kommen z.B. Suppositorien in Betracht, welche aus einer Kombination des Wirkstoffs mit einer Suppositoriengrundmasse bestehen. Als Suppositorengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffes mit einer Grundmasse enthalten; als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässerige Lösungen eines Wirkstoffes in wasserlöslicher Form, z.B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat, oder Triglyceride verwendet, oder wässrige Injektionssuspensionen, welche viskositätserhöhende Stoffe, z.B. Natriumcarboxymethylcellulose, Sorbit und/oder Dextran und gegebenenfalls Stabilisatoren enthalten.

Die pharmazeutischen Präparate der vorliegenden Erfindung können in an sich bekannter Weise, z.B. mittels konventioneller Misch-, Granulier-, Dragier-, Lösungs- oder Lyophilisierungsverfahren hergestellt werden. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Die nachfolgenden Beispiele illustrieren die oben beschriebene Erfindung, schränken jedoch deren Umfang in keiner Weise ein. Temperaturen werden in Celsiusgraden angegeben.

### Beispiel 1

Ein Gemisch von 12.4 g Pyridin-3-carboxaldehyd-1-oxid, 17.3 ml Acetessigsäuremethylester, 16 ml absolutem Aethanol und 8 ml 30%-igen wässrigem Ammoniak wird bei einer Badtemperatur von 100° während 2 Stunden gerührt. Nach dem Abkühlen werden langsam 100 ml Wasser zugetropft und das Reaktionsgemisch unter vermindertem Druck (Wasserstrahlvakuum) eingeengt. Dabei fällt ein Niederschlag aus, der, aus Isopropanol umkristallisiert, den 2,6 - Dimethyl - 4 - (1 - oxido - 3 - pyridyl) - 1,4 - dihydro - pyridin - 3,5 - dicarbonsäure - dimethylester ergibt, und bei 222—224° schmilzt. Das Pyridin - 3 - carboxyldehyd - 1 - oxid wird nach dem in Z. Chem., Bd. 10, S. 184 (1970) beschriebenen Verfahren hergestellt.

Beispiel 2

Ein Gemisch von 18.4 g Pyridin-3-carboxaldehyd-1-oxid, 24 ml absolutem Aethanol, 38.1 ml Acetessigsäureäthylester und 15 ml 30%-igem wässrigen Ammoniak wird während 1-3/4 Stunden bei einer Badtemperatur von 100° erhitzt, dann abgekühlt und tropfenweise mit 144 ml Wasser versetzt. Die erhaltene Suspension wird während etwa einer Stunde bei Raumtemperatur gerührt und der Niederschlag abfiltriert. Der so erhaltene 2,6 - Dimethyl - 4 - (1 - oxido - 3 - pyridyl) - 1,4 - dihydro - pyridin - 3,5 - dicarbonsäure - diäthylester schmilzt nach Umkristallisieren aus 240 ml Acetonitril bei 191—192°.

Beispiel 3

Ein Gemisch von 12.3 g Pyridin-3-carboxaldehyd-1-oxid in 120 ml absoluten Aethanol wird mit 11 ml Acetessigsäuremethylester und 13.7 g 3-Amino-crotonsäureäthylester versetzt, und das Reaktionsgemisch bei einer Badtemperatur von 90° erwärmt. Man erhält nach kurzer Zeit eine klare Lösung; nach 3 1/2-stündigem Erwärmen bei dieser Temperatur, wird das Reaktionsgemisch unter Wasserstrahlvakuum eingedampft. Der ölige Rückstand wird mit 200 ml Wasser verrührt, worauf ein kristallines Produkt ausfällt. Dieser wird abfiltriert, mit Wasser gewaschen, über Phosphorpentoxid getrocknet und aus Acetonitril umkristallisiert. Der so erhältliche 2,6 - Dimethyl - 4 - (1 - oxido - 3 - pyridyl) - 1,4 - dihydro - pyridin - 3 - carbonsäuremethylester - 5 - carbonsäureäthylester schmilzt bei 183—185°.

Beispiel 4

Ein Gemisch von 6.15 g Pyridin-3-carboxaldehyd-1-oxid, 8,7 g Acetessigsäureisobutylester, 6.5 g 3-Amino-crotonsäuremethylester und 5 g Molekularsieb (Union Carbide, 3Å) in 50 ml absolutem Aethanol wird während 4 Stunden unter Erwärmen (Badtemperatur: 90°) erhitzt. Nach dem Abkühlen wird das Molekularsieb abfiltriert und das Filtrat unter vermindertem Druck zur Trockne eingedampft. Der Rückstand wird zwischen 50 ml. Diäthyläther und 50 ml 2-n. Salzsäure verteilt; die Schichten werden nach dem Durchschütteln getrennt, und die organische Phase zweimal mit je 50 ml 2-n. Salzsäure gewaschen. Die vereinigten Salzsäurelösungen werden mit Aktivkohle behandelt und filtriert, und das Filtrat mit 20 g Kaliumhydrogencarbonat versetzt, dann mit Essigsäureäthylester extrahiert. Der organische Extrakt wird über Natriumsulfat getrocknet und eingedampft, und der Rückstand mit 40 ml siedendem Acetonitril verrührt, dann filtriert. Das Filtrat wird unter vermindertem Druck zur Trockne eingedampft und der Rückstand durch Lösen in 60 ml Essigsäureäthylester und Verdünnen mit 30 ml Diäthyläther umkristallisiert. Der so erhältliche 2,6 - Dimethyl - 4 - (1 - oxido - 3 - pyridyl) - 1,4 - dihydro - pyridin - 3 - carbonsäureisobutylester - 5 - carbonsäuremethylester schmilzt bei 139—144°. Die Verbindung wird an Silicagel unter Verwendung von Essigsäureäthylester-Hexan-Methanol-Gemisch (10:20:5) als Laufmittel chromatographiert, wonach man beim Aufarbeiten der die gewünschte Verbindung enthaltenden Fraktionen die Verbindung in reiner Form erhält, die bei 155—156,5° schmilzt.

Beispiel 5

Analog dem im Beispiel 1 beschriebenen Verfahren erhält man aus 24,1 g 2-Methyl-pyridin-6-carboxaldehyd-1-oxid, 40 ml Aethanol, 50,4 ml Acetessigsäureäthylester und 20 ml 25%igem wässrigem Ammoniak den 2,6 - Dimethyl - 4 - (2 - methyl - 1 - oxido - 6 - pyridyl) - 1,4 - dihydropyridin - 3,5 -dicarbonsäure-diäthylester, der nach Umkristallisieren aus Aethanol bei 224—225° (unter Zersetzung) schmilzt.

Das als Ausgangsmaterial verwendete 2-Methyl-pyridin-6-carboxaldehyd-1-oxid kann analog dem in J. Org. Chem., Bd 40, S. 1391 (1975) beschriebenen Verfahren hergestellt werden, indem man ein Gemisch von 24.6 g 2,6-Lutidin-1-oxid und 26.6 g Selendioxid in 160 ml Pyridin während 20 Stunden bei einer Badtemperatur von 125° erhitzt; das Rohprodukt wird ohne weitere Reinigung weiterverarbeitet.

Beispiel 6

Ein Gemisch von 12.3 g Pyridin-3-carboxaldehyd-1-oxid, 100 ml Aethanol, 10 g Molekularsieb (Union Carbide, 3 Å) und 12.6 ml Acetessigsäureäthylester wird bei Raumtemperatur während 2 Stunden gerührt und dann mit 24 g 3-[2-(4-Morpholino)-äthyl-amino]-crotonsäureäthylester in 20 ml Aethanol versetzt. Das Reaktionsgemisch wird bei einer Badtemperatur von 90° während 3 Stunden unter Rühren erhitzt, dann filtriert und das Filtrat mit 40 ml einer 3-n. Lösung von Chlorwasserstoff in Diäthylätgher versetzt. Man rührt während einer Stunde, filtriert den Niederschlag ab (dieser wird verworfen) und behandelt das Filtrat nochmals mit 20 ml der 3-n. Chlorwasserstofflösung in Diäthyläther. Der entstandene Niederschlag wird abfiltriert und in Wasser gelöst; die Lösung wird mit 10 g Kaliumhydrogencarbonat versetzt und mit Essigsäureäthylester extrahiert. Der organische Extrakt wird zur Trockne eingedampft und der kristalline Rückstand aus 1,2-Dimethoxy-äthan umkristallisiert. Der so erhältliche 2,6-Dimethyl-1-[2-(4-morpholino)-äthyl]-4-(1-oxido-3-pyridyl)-1,4-dihydro-pyridin-3,5-dicarbonsäure-diäthylester schmilzt bei 142—144°.

Beispiel 7

Ein Gemisch von 3.3 g 2,6-Dimethyl-4-(3-pyridyl)-1,4-dihydro-pyridin-3,5-dicarbonsäure-diäthylester und 1.4 g Natriumacetat in 50 ml Methylenchlorid wird tropfenweise mit einer Lösung von 2,1 g 3-Chlorperbenzoesäure in 40 ml Methylenchlorid versetzt. Man rührt während 20 Stunden bei Raumtemperatur und extrahiert das Reaktionsgemisch mit einer 2-n. wässrigen Natriumcarbonatlösung und dann zweimal

mit einer wässrigen Eisen-II-sulfat-Lösung. Die organische Lösung wird getrocknet und unter vermindertem Druck eingedampft. Der Rückstand wird an 400 g Silikagel chromatographiert, wobei man als flüssige Phase ein 95:5-Gemisch von Chloroform und Methanol verwendet und den Verlauf des Chromatogramms mittels Dünnschichtchromatographie (Silikagel; mobile Phase: Chloroform/Methanol 95:5) verfolgt. Das Eluat, das den Hauptanteil der gewünschten Verbindung enthält, wird eingedampft und der Rückstand aus Acetonitril kristallisiert. Man erhält so den 2,6-Dimethyl-4-(1-oxido-3-pyridyl)-1,4-dihydro-pyridin-3,5-dicarbonsäure-diäthylester, der bei 191—193° schmilzt und mit dem nach dem Verfahren des Beispiels 2 erhältlichen Produkt identisch ist.

Beispiel 8

Ein Gemisch von 22.5 g 1-Methylsulfonyl-1-(1-oxido-3-pyridyl-methylen)-aceton, 16.5 g 3-Amino-crotonsäuremethylester und 45 g Molekularsieb (Union Carbide, 3 Å) in 250 ml absolutem Aethanol wird während 18 Stunden unter Rückfluss gekocht. Die erhaltene Suspension wird filtriert, der Filterrückstand mit Chloroform gewaschen und das Filtrat unter vermindertem Druck eingedampft. Der ölige Rückstand wird durch Zugabe von Toluol und erneutem Eindampfen unter vermindertem Druck getrocknet. Der Rückstand wird an der etwa 50- bis 100-fachen Menge Silikagel chromatographiert, wobei man als flüssige Phase ein 9:1-Gemisch von Chloroform und Methanol verwendet. Nach Auswaschen der Ausgangsstoffe und von Zwischenprodukt wird das erwünschte Produkte eluiert; die entsprechenden Fraktionen werden vereinigt und eingedampft, und der Rückstand aus einem Gemisch von Isopropanol und Diäthyläther umkristallisiert. Man erhält so den 2,6 - Dimethyl - 5 - methylsulfonyl - 4 - (1 - oxido - 3 - pyridyl) - 1,4 - dihydro - pyridin - 3 - carbonsäuremethylester, der bei 236—238° schmilzt.

Das Ausgangsmaterial kann man wie folgt erhalten: Ein Gemisch von 6.15 g Pyridin-3-carboxaldehyd-1-oxid, 6.8 g Methylsulfonyl-aceton und 1 ml Piperidin in 20 ml Dimethylformamid und 400 ml absolutem Benzol wird unter Abscheidung von entstandenem Wasser unter Rückfluss erhitzt und dann unter vermindertem Druck eingedampft; die letzten Reste Dimethylformamid werden durch Zugabe von Toluol und erneutem Eindampfen unter vermindertem Druck entfernt. Der Rückstand, enthaltend das rohe 1-Methylsulfonyl-1-(1-oxido-3-pyridyl-methylen)-aceton, wird ohne weitere Reinigung verwendet.

Beispiel 9

Ein Gemisch von 10.9 g Pyridin-3-carboxaldehyd-1-oxid und 11.4 g 3-Amino-crotonsäureäthylester, 14.57 g (4-Chlor-acetessigsäureäthylester und 10.0 g Molekularsieb (Union Carbide, 3 Å) in 50 ml absolutem Aethanol wird während 15 Stunden unter Rückfluss erhitzt. Die erhaltene Suspension wird filtriert, der Filterrückstand mit Chloroform gewaschen und das Filtrat unter vermindertem Druck eingedampft. Durch Zugabe von Toluol und erneutem Eindampfen unter vermindertem Druck wird der ölige Rückstand getrocknet und an der etwa 50- bis 100-fachen Menge Silikagel chromatographiert, wobei man mit einem 9:1-Gemisch von Chloroform und Methanol eluiert. Die mittels Dünnschichtchromatographie festgestellten einheitlichen Fraktionen werden vereinigt und zur Trockne eingedampft und der Rückstand aus Methanol umkristallisiert. Der so erhältliche 2 - Methyl - 4 - (1 - oxido - 3 - pyridyl) - 5 - oxo - 1,4,5,7 - tetrahydro - furo[3,4 - b]pyridin - 3 - carbonsäureäthylester der Formel

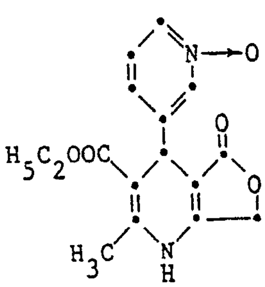

schmilzt bei 253—255°; er entsteht unter den Reaktionsbedingungen aus dem intermediär gebildeten 6 - Chlormethyl - 2 - methyl - 4 - (1 - oxido - 3 - pyridyl) - 1,4 - dihydro - pyridin - 3,5 - dicarbonsäure - diäthylester.

Beispiel 10

Ein Gemisch von 10.0 g 2 - Methyl - 4 - (1 - oxido - 3 - pyridyl) - 5 - oxo - 1,4,5,7 - tetrahydro - furo[3,4 - b]pyridin - 3 - carbonsäureäthylester und 100 ml einer 33%-igen Lösung von Methylamin in Aethanol wird in einem geschlossenen Rohe während 16 Stunden bei 90° erhitzt. Das Reaktionsgemisch wird unter vermindertem Druck zur Trockne eingedampft und der Rückstand zwischen Methylenchlorid und einer 2-n. wässrigen Natriumcarbonatlösung verteilt. Die organische Phase wird abgetrennt, mit Wasser gewaschen, getrocknet und unter vermindertem Druck eingedampft. Der Rückstand wird an Silikagel chromatographiert und mit Essigsäureäthylester eluiert. Die mittels Dünnschichtchromatogramm ermittelten einheitlichen Fraktionen werden unter vermindertem Druck zur Trockne eingedampft und der Rückstand aus einem Gemisch von Isopropanol und Petroläther umkristallisiert, ergibt den 6 -

Hydroxymethyl - 2 - methyl - 5 - methylaminocarbonyl - 4 - (1 - oxido - 3 - pyridyl) - 1,4 - dihydro - pyridin - 3 - carbonsäureäthylester, der bei 197° schmilzt.

Beispiel 11

Analog dem im Beispiel 1 beschriebenen Verfahren erhält man aus 22.5 g Pyridin-2-carboxaldehyd-1-oxid, 37 ml Acetessigsäureäthylester und 14.5 ml 30%-igem wässrigem Ammoniak in 29 ml absolutem Aethanol den 2,6 - Dimethyl - 4 - (1 - oxido - 2 - pyridyl) - 1,4 - dihydro - pyridin - 3,5 - dicarbonsäure - diäthylester, der nach Umkristallisieren aus 250 ml Acetonitril bei 198—200° schmilzt.

Das als Ausgangsmaterial verwendete Pyridin-2-carboxaldehyd-1-oxid kann z.B. nach dem in Z. Chem., Bd. 10, S. 184 (1970) beschriebenen Verfahren hergestellt werden.

Beispiel 12

Analog dem im Beispiel 1 beschriebenen Verfahren erhält man aus 12,3 g Pyridin-4-carboxaldehyde-1-oxid, 25,7 ml Acetessigsäureäthylester und 10 ml 30%igem wässrigem Ammoniak in 20 ml absolutem Aethanol den 2,6 - Dimethyl - 4 - (1 - oxido - 4 - pyridyl) - 1,4 - dihydro - pyridin - 3,5 - dicarbonsäure - diäthylester, der nach Umkristallisieren aus 100 ml Isopropanol bei 229—231° schmilzt.

Das als Ausgangsmaterial verwendete Pyridin-4-carboxaldehyd-1-oxid kann z.B. nach dem in Z.Chem., Bd. 10, S. 184 (1970) beschriebenen Verfahren hergestellt werden.

Beispiel 13

Analog dem im Beispiel 1 beschriebenen Verfahren erhält man aus 12,3 g Pyridin-3-carboxaldehyd-1-oxid, 28,8 g Acetessigsäure-n-propylester und 10 ml 30%igem wässrigem Ammoniak in 20 ml absolutem Aethanol den 2,6 - Dimethyl - 4 - (1 - oxido - 3 - pyridyl) - 1,4 - dihydro - pyridin - 3,5 - dicarbonsäure - di - n - propylester, der nach Umkristallisieren aus 125 ml Acetonitril bei 190—192° schmilzt.

Beispiel 14

Analog dem im Beispiel 1 beschriebenen Verfahren erhält man aus 8,4 g 2-Methyl-pyridin-3-carboxaldehyd-1-oxid, 16,5 ml Acetessigsäureäthylester und 6,1 ml 30%igem wässrigem Ammoniak in 34 ml absolutem Aethanol den 2,6 - Dimethyl - 4 - (2 - methyl - 1 - oxido - 3 - pyridyl) - 1,4 - dihydro - pyridin - 3,5 - dicarbonsäure - diäthylester, der nach Umkristallisieren aus 600 ml Acetonitril bei 228—230° schmilzt.

Das als Ausgangsmaterial verwendete 2-Methyl-pyridin-3-carboxaldehyd-1-oxid kann z.B. nach dem in Z.Chem., Bd. 10, S. 184 (1970) beschriebenen Verfahren hergestellt werden.

Beispiel 15

Analog dem im Beispiel 1 beschriebenen Verfahren erhält man aus 8,4 g 2-Chlor-pyridin-3-carboxaldehyd-1-oxid, 12,75 ml Acetissigsäureäthylester und 5 ml 30%igem wässrigem Ammoniak in 35 ml absolutem Aethanol den 2,6 - Dimethyl - 4 - (2 - chlor - 1 - oxido - 3 - pyridyl) - 1,4 - dihydro - pyridin - 3,5 - dicarbonsäure - diäthylester, der nach Umkristallisieren aus 500 ml Isopropanol bei 255—256° schmilzt.

Das als Ausgangsmaterial verwendete 2-Chlor-pyridin-3-carboxaldehyd-1-oxid kann z.B. nach dem in Z.Chem., Bd. 10, S. 184 (1970) beschriebenen Verfahren hergestellt werden.

Beispiel 16

Ein Gemisch von 14,7 g Pyridin-3-carboxaldehyd-1-oxid, 17,3 g Acetessigsäure-isopropylester und 12 g Molekularsieb (Union Carbide 3 Å) in 145 ml absolutem Aethanol, wird während 2 Stunden bei Raumtemperatur gerührt. Darauf fügt man 19 g 3-Amino-crotonsäure-(2-methoxyäthyl)-ester hinzu und erwämt während 3 Stunden bei einer Badtemperatur von 90°. Das erkaltete Reaktionsgemisch wird filtriert, das Molekularsieb mit Aethanol gewaschen und die vereinigten Filtrate unter vermindertem Druck eingedampft; der ölige Rückstand kristallisiert nach Zugabe von Acetonitril. Man filtriert das Rohprodukt ab und kristallisiert zuerst aus 620 ml Essigsäureäthylester unter Verwendung eines Aktivkohlepräparats und dann aus 110 ml Acetonitril um. Man erhält so den 2,6 - Dimethyl - 4 - (1 - oxido - 3 - pyridyl) - 1,4 - dihydro - pyridin - 3 - carbonsäureisopropylester - 5 - carbonsäure - (2 - methoxyäthyl) - ester, der bei 181—183° schmilzt.

Beispiel 17

Ein Gemisch von 12,3 g Pyridin-3-carboxaldehyd-1-oxid, 10 g Molekularsieb (Union Carbide, 3 Å) und 24,9 g Acetessigsäure-[2-(N-methyl-N-benzyl-amino)-äthyl]-ester in 120 ml absolutem Aethanol wird während 1 Stunde bei einer Badtemperatur von 90° erhitzt. Man lässt Erkalten, gibt darauf 11,9 g 3-Amino-crotonsäuremethylester hinzu und erwärmt nochmals während 3 Stunden bei einer Badtemperatur von 90°. Das Reaktionsgemisch wird bei Raumtemperatur abfiltriert, der Filterrückstand (Molekularsieb) mit Aethanol gewaschen und die vereinigten Filtrate unter vermindertem Druck eingedampft. Man löst den Rückstand in 310 ml Essigsäureäthylester, filtriert die kleine Menge unlöslichen Materials ab und extrahiert die Lösung zweimal mit je 100 ml 2-n-Salzsäure. Die wässrige Phase wird durch Zugabe von

Kaliumcarbonat basisch gestellt und dreimal mit je 100 ml Essigsäureäthylester extrahiert. Die vereinigten organischen Extrakte werden mit gesättigter wässriger Kaliumhydrogencarbonat-Lösung, dann mit Wasser und schliesslich mit einer gesättigten wässrigen Natriumchloridlösung gewaschen und über Natriumsulfat getrocknet. Nach Filtration und Eindampfen verbleibt ein klebriger Rückstand, der an 1200 g Kieselgel 60 (Korngrösse 0,063—0,2 mm) unter Verwendung von Methylenchlorid mit steigenden Anteilen von Methanol (1%, 7% und 10%) chromatographiert wird. Die das gewünschte Produkt enthaltenden Fraktionen werden mittels Dünnschichtchromatographie bestimmt, vereinigt und unter vermindertem Druck eingedampft. Der Rückstand wird mit 20 ml tert.-Butyl-methyläther bei Raumtemperatur verrührt, filtriert und aus 110 ml Dimethoxyäthan unter Verwendung eines Aktivkohlepräparates umkristallisiert. Man erhält so den 2,6-Dimethyl-4-(1-oxido-3-pyridyl)-1,4-dihydropyridin-3-carbonsäuremethylester-5-carbonsäure-[2-(N-methyl-N-benzylamino)-äthyl]-ester, der bei 147—149° schmilzt.

Beispiel 18

Ein Gemisch von 12,3 g Pyridin-3-carboxaldehyd-1-oxid und 12,9 g 3-Amino-crotonsäureäthylester, 14,6 g 4-Methoxy-acetessigsäuremethylester und 25,0 g Molekularsieb (Union Carbide, 3 Å) in 100 ml absolutem Aethanol wird während 19 Stunden unter Rückfluss erhitzt. Die erhaltene Suspension wird filtriert, der Filterrückstand mit Chloroform gewaschen und das Filtrat unter vermindertem Druck eingedampft. Durch Zugabe von Toluol und erneutem Eindampfen unter vermindertem Druck wird der ölige Rückstand getrocknet und an der etwa 50- bis 100-fachen Menge Silikagel chromatographiert, wobei man mit einem 9:1-Gemisch von Chloroform und Methanol eluiert. Die mittels Dünnschichtchromatographie festgestellten einheitlichen Fraktionen werden vereinigt und zur Trockne eingedampft, und der Rückstand aus einem Gemisch von Isopropanol und Petroläther umkristallisiert. Der so erhältliche 6 - Methoxymethyl - 2 - methyl - 4 - (1 - oxido - 3 - pyridyl) - 1,4 - dihydro - pyridin - 3 - carbonsäureäthylester - 5 - carbonsäuremethylester schmilzt bei 165—167°.

Beispiel 19

Ein Gemisch von 12,3 g Pyridin-3-carboxaldehyd-1-oxid, 12,8 g Amidino-essigsäureäthylester und 13,0 g Acetessigsäureäthylester in 500 ml absolutem Aethanol wird während 11 Stunden unter Rückfluss erhitzt und die erhaltene Lösung unter vermindertem Druck eingedampft. Durch Zugabe von Toluol und erneutem Eindampfen unter vermindertem Druck wird der ölige Rückstand getrocknet, dann in Essigsäureäthylester gelöst und die Lösung mit 2-n-Salzsäure mehrmals extrahiert. Die vereinigten sauren Extrakte werden mit einer konzentrierten wässrigen Ammoniaklösung basisch gestellt und mit Essigsäureäthylester extrahiert. Nach Trocknen und Eindampfen wird der Rückstand an der 50 bis 100 fachen Menge Silikagel chromatographiert, wobei man mit einem 9:1-Gemisch von Chloroform und Methanol eluiert. Die mittels Dünnschichtchromatographie festgestellten einheitlichen Fraktionen werden vereinigt und zur Trockne eingedampft, und der Rückstand aus einem Gemisch von Aethanol und Diäthyläther umkristallisiert. Der so erhältliche 2 - Amino - 6 - methyl - 4 - (1 - oxido - 3 - pyridyl) - 1,4 - dihydro - pyridin - 3,5 - dicarbonsäure - diäthylester schmilzt bei 232—234°.

Beispiel 20

Analog der im Beispiel 1 beschriebenen Verfahrensweise erhält man aus 4,1 g 2-Methylthio-pyridin-3-carboxaldehyd-1-oxid, 6,45 g Acetessigsäureäthylester und 2,45 ml 30%igem wässrigen Ammoniak in 4,8 ml 1,2-Dimethoxyäthan als Lösungsmittel 2,6 - Dimethyl - 4 - (2 - methylthio - 1 - oxido - 3 - pyridyl) - 1,4 - dihydro - pyridin - 3,5 - dicarbonsäurediäthylester, der nach dem Umkristallisieren aus Acetonitril bei 222—224° schmilzt.

Beispiel 21

Analog der im Beispiel 4 beschriebenen Verfahrenweise erhält man aus 13,7 g 2-Methyl-pyridin-3-carboxaldehyd-1-oxid, 17,4 g Acetessigsäureisobutylester, 13,0 g 3-Amino-crotonsäuremethylester in 100 ml 1,2-Dimethoxyäthan als Lösungsmittel ein Ester-Gemisch, welches man an Silicagel mit Essigsäureäthylester-Hexan-Methanol (10:20:5) als flüssige Phase chromatographiert, und die Eluate, welche den Hauptanteil der gewünschten Verbindung enthalten, vereinigt und eindampft. Nach Umkristallisieren des Rückstands aus Aethanol und Trocknen der Kristalle bei 110° im Hochvakuum erhält man 2,6 - Dimethyl - 4 - (2 - methyl - 1 - oxido - 3 - pyridyl) - 1,4 - dihydroxy - pyridin - 3 - carbonsäureisobutylester - 5 - carbonsäuremethylester · - 1/3 Aethanol, welcher bei 220—221° unter Zersetzung schmilzt.

Beispiel 22

Ein Gemisch von 4,0 g 2-Chlor-pyridin-3-carboxaldehyd-1-oxid, 3,3 g 3-Aminocrotonsäureäthylester und 4,12 g 4-Chlor-acetessigsäureäthylester in 80 ml absolutem Aethanol wird während 18 Stunden unter Rückfluss erhitzt, das Reaktionsgemisch abgekühlt und das ausgefallene Produkt abgesaugt. Der Filterrückstand wird mit viel Wasser gewaschen, getrocknet und dann aus einem Gemisch von Chloroform und Methanol umkristallisiert. Der so erhaltene 2 - Methyl - 4 - (1 - oxido - 2 - chlor - 3 - pyridyl) - 5 - oxo - 1,4,5,7 - tetrahydrofuro[3,4 - b]pyridin - 3 - carbonsäureäthylester schmilzt bei 235 bis 237°C.

Beispiel 23

Analog dem im Beispiel 22 beschriebenen Verfahren erhält man aus 6,5 g 2-Methylpyridin-3-carboxaldehyd-1-oxid, 6,1 g 3-Amino-crotonsäureäthylester und 7,75 g 4-Chlor-acetessigsäureäthylester in 100 ml absolutem Aethanol den 2 - Methyl - 4 - (1 - oxido - 2 - methyl - 3 - pyridyl) - 5 - oxo - 1,4,5,7 - tetrahydro - furo[3,4 - b]pyridin - 3 - carbonsäureäthylester, der nach Umkristallisieren aus einem Gemisch von Methanol und Methylenchlorid bei 278°c schmilzt.

Beispiel 24

Analog dem im Beispiel 22 beschriebenen Verfahren erhält man man aus 3,0 g 2-Methylmercapto-pyridin-3-carboxaldehyd-1-oxid, 2,3 g 3-Amino-crotonsäureäthylester und 2,9 g 4-Chlor-acetessigsäureäthylester in 60 ml absolutem Aethanol den 2 - Methyl - 4 - (1 - oxido - 2 - methylthio - 3 - pyridyl) - 5 - oxo - 1,4,5,7 - tetrahydro - furo[3,4 - b]pyridin - 3 - carbonsäureäthylester, der nach Umkristallisieren aus einem Gemisch von Methanol und Methylenchlorid bei 274 bis 278°C schmilzt.

Beispiel 25

Ein Gemisch von 8,9 g 2-Methoxy-pyridin-3-carboxaldehyd-1-oxid, 14 ml Acetessigsäureäthylester und 6 ml 30%-igem wässrigem Ammoniak in 14 ml 1,2-Dimethoxyäthan wird 15 Minuten bei 20°C, dann 3,5 Stunden bei einer Badtemperatur von 90°C gerührt. Das Reaktionsgemisch wird anschliessend im Vakuum eingeengt, der Rückstand mit 10 ml Acetonitril verrührt und der schwerlösliche Teil abgesaugt. Der Filterrückstand wird zuerst aus Acetonitril, dann aus 1,2-Dimethoxyäthan umkristallisiert. Der erhaltene 2,6 - Dimethyl - 4 - (1 - oxido - 2 - methoxy - 3 - pyridyl) - 1,4 - dihydro - pyridin - 3,5 - dicarbonsäure-diäthylester schmilzt bei 172 bis 174°C.

Das als Ausgangsmaterial verwendete 2-Methoxy-pyridin-3-carboxaldehyd-1-oxid kann wie folgt erhalten werden: 14 g 2-Chlor-pyridin-3-carboxaldehyd-1-oxid [hergestellt gemäss Z. Chem., Bd. 10, S. 184 (1970)] wird zu einer Lösung von 5,53 g Natriummethylat in 88 ml abs. Methanol gegeben und die Mischung während 3 Stunden bei einer Badtemperatur von 70°C erhitzt. Hierauf wird das Reaktionsgemisch im Vakuum eingeengt, der Rückstand mit Methylenchlorid ausgerührt und Unlösliches mittels eines Filterhilfsmittels abgesaugt. Das Filtrat wird eingedampft und der Rückstand mit Essigsäureäthylester zweimal ausgekocht. Die Extrakte werden filtriert, vereinigt und im Vakuum eingeengt. Das erhaltene rote 2-Methoxy-pyridin-3-carboxaldehyd-1-oxid wird als solches weiterverarbeitet.

Beispiel 26

Analog dem im Beispiel 1 beschriebenen Verfahren erhält man aus 1,9 g 2-Methylsulfinylpyridin-3-carboxaldehyd-1-oxid, 3,1 ml Acetessigsäureäthylester, 1,1 ml 30%-igem wässrigem Ammoniak und 0,12 g Ammoniumchlorid in 3,1 ml 1,2-Dimethoxyäthan den 2,6-Dimethyl-4-(2-methylsulfinyl-1-oxido-3-pyridyl)-1,4-dihydropyridin-3,5-dicarbonsäurediäthylester, der nach Kristallisation aus Acetonitril bei 220 bis 221°C unter Gasentwicklung schmilzt.

Das als Ausgangsmaterial verwendete 2-Methylsulfinyl-pyridin-3-carboxaldehyd-1-oxid und 10 mg 4,4'-Thiobis-(6-tert.butyl-3-methyl-phenol) in 10 ml Chloroform wird auf 0 abgekühlt und dazu eine Lösung von 2,1 g 3-Chlorperbenzoesäure in 20 ml Chloroform zugetropft. Man lässt das Gemisch über Nacht bei 0° stehen und gibt dann weitere 0,2 g 3-Chlorperbenzoesäure zu. Nach einer Stunde wird zum kalten Reaktionsgemisch 1,69 g Kaliumcarbonat zugegeben, eine Stunde ohne Kühlung gerührt und darauf abgesaugt. Der Filterrückstand wird mit Chloroform gewaschen und die vereinigten peroxidfreien Filtrate auf ein Volumen von ca. 10 ml eingeengt. Durch tropfenweise Zugabe von Aether fällt das Produkt aus, es wird abgesaugt, mit Aether gewaschen und stellt das 2-Methylsulfinylpyridin-3-carboxaldehyd-1-oxid dar, welches als solches weiterverarbeitet wird.

Beispiel 27

In analoger Weise, d.h. wie in der allgemeinen Beschreibung beschrieben und in den vorstehenden Beispielen illustriert, kann man bei geeigneter Wahl der Ausgangsstoffe und/oder Reaktionsbedingungen folgende Verbindungen erhalten:

6 - Hydroxymethyl - 2 - methyl - 4 - (1 - oxido - 3 - pyridyl) - 1,4 - dihydro - pyridin - 3,5 - dicarbonsäure - diäthylester, durch Umsetzen von Pyridin-3-carboxaldehyd-1-oxid, 3-Amino-crotonsäureäthylester und 4,4-Dimethoxyacetessigsäureäthylester, Spalten der Dimethoxymethylgruppe im so erhältlichen 6 - Dimethoxymethyl - 2 - methyl - 4 - (1 - oxido - 3 - pyridyl) - 1,4 - dihydro - pyridin - 3,5 - dicarbonsäure - diäthylester durch Behandeln mit Chlorwasserstoffsäure, und Reduktion der Formylgruppe im 6 - Formyl - 2 - methyl - 4 - (1 - oxido - 3 - pyridyl) - 1,4 - dihydro - pyridin - 3,5 - dicarbonsäurediäthylester durch Behandeln mit Natriumborhydrid;

6 - Cyanmethyl - methyl - 4 - (1 - oxido - 3 - pyridyl) - 1,4 - dihydro - pyridin - 3,5 - dicarbonsäure-diäthylester, durch Umsetzen von 6 - Hydroxymethyl - 2 - methyl - 4 - (1 - oxido - 3 - pyridyl) - 1,4 - dihydro - pyridin - 3,5 - dicarbonsäurediäthylester mit Thionylchlorid, und Behandeln des so erhältlichen 6 - Chlormethyl - 2 - methyl - 4 - (1 - oxido - 3 - pyridyl) - 1,4 - dihydro - pyridin - 3,5 - dicarbonsäure-diäthylester mit Natriumcyanid;

6 - Cyan - 2 - methyl - 4 - (1 - oxido - 3 - pyridyl) - 1,4 - dihydro - pyridin - 3,5 - dicarbonsäure-diäthylester, durch Umsetzen von 6 - Formyl - 2 - methyl - 4 - (1 - oxido - 3 - pyridyl) - 1,4 - dihydro -

pyridin - 3,5 - dicarbonsäure - diäthylester mit Hydroxylamin-hydrochlorid und Dehydratisieren des erhaltenen 6 - Hydroxyiminomethyl - 2 - methyl - 4 - (1 - oxido - 3 - pyridyl) - 1,4 - dihydro - pyridin - 3,5 - dicarbonsäurediäthylester durch Behandeln mit N,N' - Dicyclohexyl-carbodiimid;

6 - Aethoxycarbonyl - 2 - methyl - 4 - (1 - oxido - 3 - pyridyl) - 1,4 - dihydro - pyridin - 3,5 - dicarbonsäure - diäthylester, durch Behandeln von 6 - Cyanmethyl - 2 - methyl - 4 - (1 - oxido - 3 - pyridyl) - 1,4 - dihydro - pyridin - 3,5 - dicarbonsäurediäthylester mit Aethanol in Gegenwart von Schwefelsäure und Wasser;

6 - Aethoxycarbonylmethyl - 2 - methyl - 4 - (1 - oxido - 3 - pyridyl) - 1,4 - dihydro - pyridin - 3,5 - dicarbonsäure - diäthylester, durch Umsetzen von Pyridin - 3 - carboxaldehyd - 1 - oxid, 3-Amino-crotonsäureäthylester und Aceton-1,3-dicarbonsäure-diäthylester;

6 - (1 - Pyrrolidino) - 2 - methyl - 4 - (1 - oxido - 3 - pyridyl) - 1,4 - dihydropyridin - 3,5 - dicarbonsäure - diäthylester, durch Umsetzen von Pyridin-3-carboxaldehyd-1-oxid, Acetessigsäureäthylester und 2-(N,N-Tetramethylenamidino)-essigsäureäthylester;

6 - [4 - (2 - Oxo - 1 - imidazolidinyl) - 1 - piperidino] - 2 - methyl - 4 - (1 - oxido - 3 - pyridyl) - 1,4 - dihydro - pyridin - 3,5 - dicarbonsäure - diäthylester, durch Umsetzen von Pyridin-3-carboxaldehyd-1-oxid, Acetessigsäureäthylester und 3-Imino-3-[4-(2-oxo-1-imidazolidinyl)-1-piperidino] - propionsäureäthylester;

6 - [4 - (2 - Furoyl) - 1 - piperazino] - 2 - methyl - 4 - (1 - oxido - 3 - pyridyl) - 1,4 - dihydro - pyridin - 3,5 - dicarbonsäure - diäthylester, durch Umsetzen von Pyridin-3-carboxaldehyd-1-oxid, Acetessigsäureäthylester und 3-Imino-3-[4-(2-furoyl)-1-piperazino]-propionsäureäthylester;

6 - [2 - (4 - Morpholino) - äthyl] - 2 - methyl - 4 - (1 - oxido - 3 - pyridyl) - 1,4 - dihydro - pyridin - 3,5 - dicarbonsäure - diäthylester, durch Behandeln von 2,6 - Dimethyl - 4 - (1 - oxido - 3 - pyridyl) - 1,4 - dihydro - pyridin - 3,5 - dicarbonsäure - diäthylester mit Morpholin und Paraformaldehyd;

6 - (2 - Dimethylaminoäthyl) - 2 - methyl - 4 - (1 - oxido - 3 - pyridyl) - 1,4 - dihydropyridin - 3,5 - dicarbonsäure - diäthylester, durch Behandeln von 2,6 - Dimethyl - 4 - (1 - oxido - 3 - pyridyl) - 1,4 - dihydro-pyridin - 3,5 - dicarbonsäure - diäthylester, mit Dimethylamin und Paraformaldehyd;

2,6 - Dimethyl - 5 - äthylsulfonyl - 4 - (1 - oxido - 3 - pyridyl) - 1,4 - dihydropyridin - 3 - carbonsäureäthylester, durch Umsetzen von Pyridin-3-carboxyaldehyd-1-oxid, 3-Amino-crotonsäureäthylester und 1-Aethylsulfonyl-aceton;

5 - Acetyl - 2,6 - dimethyl - 4 - (1 - oxido - 3 - pyridyl) - 1,4 - dihydro - pyridin - 3 - carbonsäureäthylester, durch Umsetzen von Pyridin-3-carboxaldehyd-1-oxid, 3-Aminocrotonsäureäthylester und Acetylaceton;

6 - Hydroxymethyl - 2 - methyl - 4 - (1 - oxido - 2 - methyl - pyridyl) - 1,4 - dihydro - pyridin - 3,5 - dicarbonsäure - diäthylester, durch Umsetzen von Pyridin - 3 - carboxaldehyd - 2 - methyl - 1 - oxid, 3 - Amino - crotonsäureäthylester und 4,4'-Dimethoxyacetessigsäureäthylester, Spalten der Dimethoxymethylgruppe im so erhältlichen 6 - Dimethoxymethyl - 2 - methyl - 4 - (1 - oxido - 2 - methyl - 3 - pyridyl) - 1,4 - dihydro - pyridin - 3,5 - dicarbonsäure - diäthylester durch Behandeln mit Chlorwasserstoffsäure, und Reduktion der Formylgruppe im 6 - Formyl - 2 - methyl - 4 - (1 - oxido - 2 - methyl - 3 - pyridyl) - 1,4 - dihydro - pyridin - 3,5 - dicarbonsäure - diäthylester durch Behandeln mit Natriumborhydrid;

6 - Cyan - 2 - methyl - 4 - (1 - oxido - 3 - pyridyl) - 1,4 - dihydro - pyridin - 3,5 - dicarbonsäure - diäthylester, durch Umsetzen von 6 - Formyl - 2 - methyl - 4 - (1 - oxido - 2 - methyl - 3 - pyridyl) - 1,4 - dihydro - pyridin - 3,5 - dicarbonsäure - diäthylester mit Hydroxylamin-hydrochlorid und Dehydratisieren des erhaltenen 6 - Hydroxyiminomethyl - 2 - methyl - 4 - (1 - oxido - 2 - methyl - 3 - pyridyl) - 1,4 - dihydro - pyridin - 3,5 - dicarbonsäure - diäthylesters durch Behandeln mit N,N'-Dicyclohexyl-carbodiimid;

5 - Methyl - 7 - (1 - oxido - 3 - pyridyl) - 1,2,3,7 - tetrahydro - imidazo [1,2 - a]pyridin - 6,8 - dicarbonsäure - diäthylester der Formel

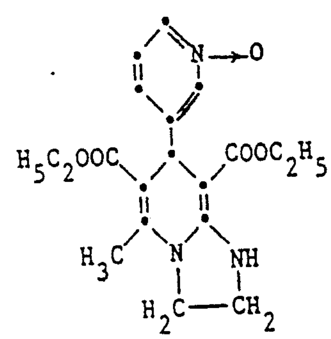

durch Umsetzen von Pyridin - 3 - carboxaldehyd - 1 - oxid, 2 - (2 - Imidazolin - 2 - yl) - essigsäureäthylester und Acetessigsäureäthylester; 2 - Methyl - 4 - (1 - oxido - 2 - methylsulfinyl - 3 - pyridyl) - 5 - oxo - 1,4,5,7 - tetrahydro - furo[3,4 - b]pyridin - 3 - carbonsäureäthylester durch Umsetzen von 2 -

Methylsulfinylpyridin - 3 - carboxaldehyd - 1 - oxid, 3 - Amino - crotonsäureäthylester und 4 - Chlor - acetessigsäureäthylester in einem geeigneten Lösungsmittel, z.B. absolutem Aethanol bei erhöhter Temperatur.

2 - Methyl - 4 - (1 - oxido - 2 - methylsulfonyl - 3 - pyridyl) - 5 - oxo - 1,4,5,7 - tetrahydrofuro[3,4 - b]pyridin - 3 - carbonsäureäthylester durch Umsetzen von 2 - Methylsulfonylpyridin - 3 - carboxaldehyd - 1 - oxid, 3 - Amino - crotonsäureäthylester und 4 - Chlor - acetessigsäureäthylester in einem geeigneten Lösungsmittel z.B. absolutem, Aethanol, bei erhöhter Temperatur.

2 - Methyl - 4 - (1 - oxido - 2 - methoxy - 3 - pyridyl) - 5 - oxo - 1,4,5,7 - tetrahydrofuro[3,4 - b]-pyridin - 3 - carbonsäureäthylester durch Umsetzen von 2 - Methoxy - pyridin - 3 - carboxaldehyd - 1 - oxid, 3 - Aminocrotonsäureäthylester und 4 - Chloracetessigsäureäthylester in einem geeigneten Lösungsmittel, z.B. absolutem Aethanol bei erhöhter Temperature.

2,6 - Dimethyl - 4 - (2 - methylsulfonyl - 1 - oxido - 3 - pyridyl) - 1,4 - dihydro - pyridin - 3,5 - dicarbonsäurediäthylester durch Umsetzen von 2 - Methylsulfonylpyridin - 3 - carboxaldehyd - 1 - oxid, Acetessigsäureäthylester und wässrigem Ammoniak in Gegenwart von Ammoniumchlorid.

Beispiel 28

Tabletten enthaltend 25 mg 2,6 - Dimethyl - 4 - (1 - oxido - 3 - pyridyl) - 1,4 - dihydro - pyridin - 3,5 - dicarbonsäure - diäthylester können wie folgt hergestellt werden:

Zusammensetzung (für 1000 Tabletten)

| | |
|---|---|
| 2,6-Dimethyl-4-(1-oxido-3-pyridyl)-1,4-dihydro-pyridin-3,5-dicarbonsäure-diäthylester | 25.0 g |
| Maisstärke | 70.0 g |
| Lactose (fein) | 78.5 g |
| Cellulose (mikrokristalline, granuliert) | 75.0 g |
| Magnesiumstearat | 1.5 g |
| Wasser | q.s. |

Herstellung:

Der 2,6 - Dimethyl - 4 - (1 - oxido - 3 - pyridyl) - 1,4 - dihydro - pyridin - 3,5 - dicarbonsäure - diäthylester wird mit 60 g Maisstärke und der Lactose vermischt und mit einem aus 10 g Maisstärke und Wasser hergestellten Kleister geknetet. Die feuchte Masse wird granuliert, getrocknet und mit der kristallinen Cellulose und dem Magnesiumstearat gemischt. Die homogene Mischung wird zu Tabletten von 250 mg (mit Bruchrille) verpresst; diese haben einen Durchmesser von 9 mm.

Beispiel 29

Kapseln enthaltend 10 mg an aktiver Substanz können wie folgt hergestellt werden:

Zusammensetzung:

| | |
|---|---|
| 2,6-Dimethyl-4-(1-oxido-3-pyridyl)-1,4-dihydro-pyridin-3,5-dicarbonsäure-diäthylester | 2500 mg |
| Talkum | 200 mg |
| Kolloidale Kieselsäure | 50 mg |

Herstellung:

Die aktive Substanz wird mit Talkum und kolloidaler Kieselsäure innig gemischt, das Gemisch durch ein Sieb mit 0,5 mm Maschenweite getrieben und dieses in Portionen von jeweils 11 mg in Hartgelatinekapseln geeigneter Grösse abgefüllt.

Beispiel 30

Eine sterile Lösung von 5,0 g 2,6 - Dimethyl - 4 - (1 - oxido - 3 - pyridyl) - 1,4 - dihydro - pyridin - 3,5 - dicarbonsäure - diäthylester in 5000 ml destillierten Wasser wird in Ampullen zu 5 ml abgefüllt, die in 5 ml Lösung 5 mg Wirkstoff enthalten.

Beispiel 31

Anstelle der in den Beispielen 28 bis 30 als Wirkstoff verwendeten Verbindungen können auch

folgende Verbindungen der Formel I oder deren pharmazeutisch verwendbaren Säureadditionssalze von solchen Verbindungen mit slazbildenden basischen Gruppen als Wirkstoffe in Tabletten, Dragées, Kepseln, Ampullenlösungen etc. verwendet werden: 2,6 - Dimethyl - 4 - (1 - oxido - 3 - pyridyl) - 1,4 - dihydro - pyridin - 3,5 - dicarbonsäure - dimethylester; 2,6 - Dimethyl - 4 - (1 - oxido - 3 - pyridyl) - 1,4 - dihydro - pyridin - 3 - carbonsäuremethylester - 5 - carbonsäureäthylester; 2,6 - Dimethyl - 4 - (1 - oxido - 3 - pyridyl) - 1,4 - dihydro - pyridin - 3 - carbonsäureisobutylester - 5 - carbonsäuremethylester; 2,6 - Dimethyl - 4 - (2 - methyl - 1 - oxido - 6 - pyridyl) - 1,4 - dihydro - pyridin - 3,5 - dicarbonsäure - diäthylester; 2,6 - Dimethyl - 1 - [2 - (4 - morpholino) - äthyl] - 4 - (1 - oxido - 3 - pyridyl) - 1,4 - dihydro - pyridin - 3,5 - dicarbonsäure - diäthylester; 2,6 - Dimethyl - 5 - methylsulfonyl - 4 - (1 - oxido - 3 - pyridyl) - 1,4 - dihydro - pyridin - 3 - carbonsäuremethylester; 2 - Methyl - 4 - (1 - oxido - 3 - pyridyl) - 5 - oxo - 1,4,5,7 - tetrahydro - furo[3,4 - b]pyridin - 3 - carbonsäureäthylester; 6 - Hydroxymethyl - 2 - methyl - 5 - methylaminocarbonyl - 4 - (1 - oxido - 3 - pyridyl) - 1,4 - dihydro - pyridin - 3 - carbonsäureäthylester; 2,6 - Dimethyl - 4 - (1 - oxido - 2 - pyridyl) - 1,4 - dihydro - pyridin - 3,5 - dicarbonsäure - diäthylester; 2,6 - Dimethyl - 4 - (1 - oxido - 4 - pyridyl) - 1,4 - dihydro - pyridin - 3,5 - dicarbonsäure - diäthylester; 2,6 - Dimethyl - 4 - (1 - oxido - 3 - pyridyl) - 1,4 - dihydro - pyridin - 3,5 - dicarbonsäure - di - n - propylester; 2,6 - Dimethyl - 4 - (2 - methyl - 1 - oxido - 3 - pyridyl) - 1,4 - dihydro - pyridin - 3,5 - dicarbonsäure - diäthylester; 2,6 - Dimethyl - 4 - (2 - chlor - 1 - oxido - 3 - pyridyl) - 1,4 - dihydro - pyridin - 3,5 - dicarbonsäure - diäthylester; 2,6 - Dimethyl - 4 - (1 - oxido - 3 - pyridyl) - 1,4 - dihydro - pyridin - 3 - carbonsäureisopropylester - 5 - carbonsäure - (2 - methoxyäthyl) - ester; 2,6 - Dimethyl - 4 - (1 - oxido - 3 - pyridyl) - 1,4 - dihydro - pyridin - 3 - carbonsäuremethylester - 5 - carbonsäure - [2 - (N - methyl - N - benzylamino) - äthyl] - ester; 6 - Methoxymethyl - 2 - methyl - 4 - (1 - oxido - 3 - pyridyl) - 1,4 - dihydro - pyridin - 3-carbonsäureäthylester - 5 - carbonsäuremethylester; 2 - Amino - 6 - methyl - 4 - (1 - oxido - 3 - pyridyl) - 1,4 - dihydro - pyridin - 3,5 - dicarbonsäure - diäthylester; 2,6 - Dimethyl - 4 - (2 - methylthio - 1 - oxido - 3 - pyridyl) - 1,4 - dihydro - pyridin - 3,5 - dicarbonsäurediäthylester; 2,6 - Dimethyl - 4 - (2 - methyl - 1 - oxido - 3 - pyridyl) - 1,4 - dihydro - pyridin - 3 - carbonsäureisobutylester - 5 - carbonsäuremethylester; 6 - Hydroxymethyl - 2 - methyl - 4 - (1 - oxido - 2 - methyl - 3 - pyridyl) - 1,4 - dihydropyridin - 3,5 - dicarbonsäure - diäthylester; 6 - Cyan - 2 - methyl - 4 - (1 - oxido - 2 - methyl - 3 - pyridyl) - 1,4 - dihydro - pyridin - 3,5 - dicarbonsäure - diäthylester, 2 - Methyl - 4 - (1 - oxido - 2 - chlor - 3 - pyridinyl) - 5 - oxo - 1,4,5,7 - tetrahydrofuro[3,4 - b]pyridin - 3 - carbonsäureäthylester, 2 - Methyl - 4 - (1 - oxido - 2 - methyl - 3 - pyridinyl) - 5 - oxo - 1,4,5,7 - tetrahydro - furo[3,4 - b]pyridin - 3 - carbonsäureäthylester, 2 - Methyl - 4 - (1 - oxido - 2 - methylthio - 3 - pyridinyl) - 5 - oxo - 1,4,5,7 - tetrahydro - furo[3,4 - b]pyridin - 3 - carbonsäureäthylester, 2 - Methyl - 4 - (1 - oxido - 2 - methylsulfinyl - 3 - pyridinyl) - 5 - oxo - 1,4,5,7 - tetrahydro - furo[3,4 - b]pyridin - 3 - carbonsäureäthylester, 2 - Methyl - 4 - (1 - oxido - 2 - methylsulfonyl - 3 - pyridinyl) - 5 - oxo - 1,4,5,7 - tetrahydro - furo[3,4 - b]pyridin - 3 - carbonsäureäthylester, 2,6 - Dimethyl - 4 - (1 - oxido - 2 - methoxy - 3 - pyridyl) - 1,4 - dihydro - pyridin - 3,5 - dicarbonsäure - diäthylester, 2,6 - Dimethyl - 4 - (2 - methylsulfinyl - 1 - oxido - 3 - pyridyl) - 1,4 - dihydro - pyridin - 3,5 - dicarbonsäure - diäthylester, sowie die im Beispiel 27 beschriebenen Verbindungen der Formel I.

**Patentansprüche für die Vertragsstaaten: BE CH DE FR IT LI LU NL SE**

1. Verbindungen der Formel

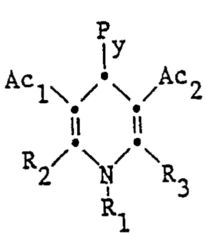

(I),

in welcher Py unsubstituiertes oder durch ($C_1$—$C_7$)-Niederalkyl, ($C_1$—$C_7$)-Niederalkoxy, ($C_1$—$C_7$)-Niederalkylthio, ($C_1$—$C_7$)-Niederalkylsulfinyl, ($C_1$—$C_7$)-Niederalkylsulfonyl und/oder Halogen substituiertes N-Oxidopyridyl bedeutet, $R_1$ Wasserstoff, ($C_1$—$C_7$)-Niederalkyl, Di-($C_1$—$C_7$)-niederalkylamino-($C_1$—$C_7$)-niederalkyl, ($C_4$—$C_6$)-Niederalkylenamino-($C_1$—$C_7$)-niederalkyl, Morpholino-($C_1$—$C_7$)-niederalkyl, Thiomorpholino-($C_1$—$C_7$)-niederalkyl, Piperazino-($C_1$—$C_7$)-niederalkyl oder 4-($C_1$—$C_7$)-Niederalkyl-piperazino-($C_1$—$C_7$)-niederalkyl darstellt, einer der Reste $R_2$ und $R_3$ ($C_1$—$C_7$)-Niederalkyl bedeutet, und der andere für Wasserstoff, ($C_1$—$C_7$)-Niederalkyl, Hydroxy-($C_1$—$C_7$)-niederalkyl, ($C_1$—$C_7$)-Niederalkoxy-($C_1$—$C_7$)-niederalkyl, gem-Di-($C_1$—$C_7$)-niederalkoxy-($C_1$—$C_7$)-niederalkyl, Halogen-($C_1$—$C_7$)-niederalkyl, Oxo-($C_1$—$C_7$)-niederalkyl, ($C_1$—$C_7$)-Niederalkoxycarbonyl-($C_1$—$C_7$)-niederalkyl, Carbamoyl-($C_1$—$C_7$)-niederalkyl, N-($C_1$—$C_7$)-Niederalkylcarbamoyl-($C_1$—$C_7$)-niederalkyl, N,N-Di-($C_1$—$C_7$)-niederalkylcarbamoyl-($C_1$—$C_7$)-

niederalkyl, Cyan-($C_1$—$C_7$)-niederalkyl, Amino-($C_1$—$C_7$)-niederalkyl, ($C_1$—$C_7$)-Niederalkylamino-($C_1$—$C_7$)-niederalkyl, Di-($C_1$—$C_7$)-niederalkylamino-($C_1$—$C_7$)-niederalkyl, ($C_4$—$C_6$)-Niederalkylamino-($C_1$—$C_7$)-niederalkyl, Morpholino-($C_1$—$C_7$)-niederalkyl, Thiomorpholino-($C_1$—$C_7$)-niederalkyl, Piperazino-($C_1$—$C_7$)-niederalkyl, 4-($C_1$—$C_7$)-Niederalkyl-piperazino-($C_1$—$C_7$)-niederalkyl, ($C_1$—$C_7$)-Niederalkoxyimino-($C_1$—$C_7$)-niederalkyl, worin ($C_1$—$C_7$)-Niederalkoxy und Imino das gleiche Kohlenstoffatom substituieren, Hydroxyimino-($C_1$—$C_7$)-niederalkyl, ($C_1$—$C_7$)-Niederalkoxyimino-($C_1$—$C_7$)-niederalkyl, ($C_1$—$C_7$)-Niederalkoxycarbonyl, Carbamoyl, N-($C_1$—$C_7$)-Niederalkylcarbamoyl, N,N-Di-($C_1$—$C_7$)-niederalkylcarbamoyl, Cyan, Amino, ($C_1$—$C_7$)-Niederalkylamino, Di-($C_1$—$C_7$)-niederalkylamino-($C_1$—$C_7$)-niederalkylamino, ($C_4$—$C_6$)-Niederalkylamino-($C_1$—$C_7$)-niederalkylamino, Morpholino-($C_1$—$C_7$)-niederalkylamino, Di-($C_1$—$C_7$)-niederalkylamino, ($C_4$—$C_6$)-Niederalkylenamino, (2-Oxo-1-imidazolidinyl)-($C_4$—$C_6$)-niederalkylenamino, Morpholino, Thiomorpholino, Piperazino, 4-($C_1$—$C_7$)-Niederalkyl-piperazino, 4-($C_1$—$C_7$)-Niederalkanoyl-piperazino, 4-Benzoyl-piperazino, 4-Furoyl-piperazino oder 4-Thienoyl-piperazino bedeutet, wobei eine Aminogruppe $R_2$ bzw. $R_3$ auch mit einem Niederalkylrest $R_1$ verbunden sein und mit diesem zusammen einen 1-Aza-($C_3$—$C_4$)-niederalkylenrest bilden kann, dessen Stickstoffatom mit dem 2- bzw. 6-Ringkohlenstoffatom des 1,4-Dihydro-pyridinrings verbunden ist, oder, falls $R_2$ bzw. $R_3$ für ($C_1$—$C_7$)-Niederalkyl steht, dieses zusammen mit dem benachbarten Acylrest $Ac_1$ bzw. $Ac_2$ einen 2-Oxa-1-oxo-($C_2$—$C_3$)-niederalkylenrest bilden kann, dessen Carbonylgruppe mir dem 3- bzw. 5-Ringkohlenstoffatom des 1,4-Dihydro-pyridinrings verbunden ist, und jeder der Reste $Ac_1$ und $Ac_2$ unabhängig voneinander für ($C_1$—$C_7$)-Niederalkanoyl, unsubstituiertes oder durch ($C_1$—$C_7$)-Niederalkyl, ($C_1$—$C_7$)-Niederalkoxy und/oder Halogen substituiertes Benzoyl, ($C_1$—$C_7$)-Niederalkylsulfonyl, unsubstituiertes oder durch ($C_1$—$C_7$)-Niederalkyl, ($C_1$—$C_7$)-Niederalkoxy und/oder Halogen substituiertes Phenylsulfonyl, ($C_1$—$C_7$)-Niederalkoxycarbonyl, Hydroxy-($C_1$—$C_7$)-niederalkoxycarbonyl, ($C_1$—$C_7$)-Niederalkoxy-($C_1$—$C_7$)-niederalkoxycarbonyl, Amino-($C_1$—$C_7$)-niederalkoxycarbonyl, ($C_1$—$C_7$)-Niederalkylamino-($C_1$—$C_7$)-niederalkoxycarbonyl, Di-($C_1$—$C_7$)-niederalkylamino-($C_1$—$C_7$)-niederalkoxycarbonyl, N-($C_1$—$C_7$)-Niederalkyl-N-phenyl-($C_1$—$C_7$)-niederalkyl-amino-($C_1$—$C_7$)-niederalkoxycarbonyl, ($C_4$—$C_6$)-Niederalkylenamino-($C_1$—$C_7$)-niederalkoxycarbonyl, Morpholino-($C_1$—$C_7$)-niederalkoxycarbonyl, Thiomorpholino-($C_1$—$C_7$)-niederalkoxycarbonyl, Piperazino-($C_1$—$C_7$)-niederalkoxycarbonyl, 4-($C_1$—$C_7$)-Niederalkyl-piperazino-($C_1$—$C_7$)-niederalkoxycarbonyl, unsubstituiertes oder durch ($C_1$—$C_7$)-Niederalkyl, ($C_1$—$C_7$)-Niederalkoxy und/oder Halogen substituiertes Phenyl-($C_1$—$C_7$)-niederalkoxycarbonyl, Carbamoyl, N-($C_1$—$C_7$)-Niederalkylcarbamoyl, N,N-Di-($C_1$—$C_7$)-niederalkyl-carbamoyl, N,N-($C_4$—$C_6$)-Niederalkylcarbamoyl, Morpholinocarbonyl, Thiomorpholinocarbonyl, Piperazinocarbonyl oder 4-($C_1$—$C_7$)-Niederalkylpiperazinocarbonyl steht, oder Salze von solchen Verbindungen mit salzbildenden Gruppen.

2. Verbindungen der Formel I gemäss Anspruch 1, worin Py, $R_1$, $Ac_1$ und $Ac_2$ die im Anspruch 1 gegebenen Bedeutungen haben, und einer der Reste $R_2$ und $R_3$ ($C_1$—$C_7$)-Niederalkyl bedeutet, und der andere für Wasserstoff, ($C_1$—$C_7$)-Niederalkyl, Hydroxy-($C_1$—$C_7$)-niederalkyl, ($C_1$—$C_7$)-Niederalkoxy-($C_1$—$C_7$)-niederalkyl, Halogen-($C_1$—$C_7$)-niederalkyl, ($C_1$—$C_7$)-Niederalkoxycarbonyl-($C_1$—$C_7$)-niederalkyl, Carbamoyl-($C_1$—$C_7$)-niederalkyl, N-($C_1$—$C_7$)-Niederalkylcarbamoyl-($C_1$—$C_7$)-niederalkyl, N,N-Di-($C_1$—$C_7$)-niederalkylcarbamoyl-($C_1$—$C_7$)-niederalkyl, Cyan-($C_1$—$C_7$)-niederalkyl, ($C_1$—$C_7$)-Aminoniederalkyl, ($C_1$—$C_7$)-Niederalkylamino-($C_1$—$C_7$)-niederalkyl, Di-($C_1$—$C_7$)-niederalkylamino-($C_1$—$C_7$)-niederalkyl, ($C_4$—$C_6$)-Niederalkylenamino-($C_1$—$C_7$)-niederalkyl, Morpholino-($C_1$—$C_7$)-niederalkyl, Thiomorpholino-($C_1$—$C_7$)-niederalkyl, Piperazino-($C_1$—$C_7$)-niederalkyl, 4-($C_1$—$C_7$)-Niederalkyl-piperazino-($C_1$—$C_7$)-niederalkyl, ($C_1$—$C_7$)-Niederalkoxycarbonyl, Carbamoyl, N-($C_1$—$C_7$)-Niederalkylcarbamoyl, N,N-Di-($C_1$—$C_7$)-niederalkylcarbamoyl, Cyan, Amino, ($C_1$—$C_7$)-Niederalkylamino, Di-($C_1$—$C_7$)-niederalkylamino-($C_1$—$C_7$)-niederalkylamino, ($C_4$—$C_6$)-Niederalkylenamino-($C_1$—$C_7$)-niederalkylamino, Morpholino-($C_1$—$C_7$)-niederalkylamino, Di-($C_1$—$C_7$)-niederalkylamino, ($C_4$—$C_6$)-Niederalkylenamino, (2-Oxo-1-imidazolidinyl)-piperidino, Morpholino, Thiomorpholino, Piperazino, 4-($C_1$—$C_7$)-Niederalkyl-piperazino, 4-($C_1$—$C_7$)-Niederalkanoyl-piperazino, 4-Benzoyl-piperazino, 4-Furoyl-piperazino oder 4-Thienoyl-piperazino bedeutet, wobei eine Aminogruppe $R_2$ bzw. $R_3$ auch mit einem ($C_1$—$C_7$)-Niederalkylrest $R_1$ verbunden sein und mit diesem zusammen einen 1-Aza-($C_3$—$C_4$)-niederalkylenrest bilden kann, dessen Stickstoffatom mit dem 2- bzw. 6-Ringkohlenstoffatom des 1,4-Dihydro-pyridinrings verbunden ist, oder, falls $R_2$ bzw. $R_3$ für ($C_1$—$C_7$)-Niederalkyl steht, dieses zusammen mit dem benachbarten Acylrest $Ac_1$ bzw. $Ac_2$ einen 2-Oxa-1-oxo-($C_2$—$C_3$)-niederalkylenrest bilden kann, dessen Carbonylgruppe mit dem 3- bzw. 5-Ringkohlenstoffatomen des 1,4-Dihydro-pyridinrings verbunden ist, oder Salze von solchen Verbindungen mit salzbildenden Gruppen.

3. Verbindungen der Formel I gemäss Anspruch 1, worin Py unsubstituiertes oder durch ($C_1$—$C_4$)-Niederalkyl oder ($C_1$—$C_7$)-Niederalkylsulfinyl substituiertes N-Oxidopyridyl bedeutet, $R_1$ Wasserstoff, ($C_1$—$C_4$)-Niederalkyl, 2-Di-($C_1$—$C_7$)-niederalkylamino-($C_1$—$C_7$)-niederalkyl, 2-($C_4$—$C_6$)-Niederalkylenamino-($C_1$—$C_7$)-niederalkyl oder 2-(4-Morpholino)-($C_1$—$C_7$)-niederalkyl darstellt, einer der Reste $R_2$ und $R_3$ für ($C_1$—$C_4$)-Niederalkyl steht, und der andere Wasserstoff, ($C_1$—$C_7$)-Niederalkyl, Hydroxy-($C_1$—$C_7$)-niederalkyl, Halogen-($C_1$—$C_7$)-niederalkyl, ($C_1$—$C_7$)-Niederalkoxycarbonyl-($C_1$—$C_7$)-niederalkyl, ($C_1$—$C_7$)-Cyanniederalkyl, Di-($C_1$—$C_7$)-niederalkylamino-($C_1$—$C_7$)-niederalkyl, ($C_1$—$C_7$)-Niederalkoxycarbonyl, Cyan, Amino, (4-Morpholino)-($C_1$—$C_7$)-niederalkylamino, ($C_4$—$C_6$)-Niederalkylenamino, (2-Oxo-1-imidazolidinyl)-piperazino, oder 4-(2-Furoyl)-piperazino bedeutet, wobei eine Aminogruppe $R_2$ bzw. $R_3$ mit einem ($C_1$—$C_7$)-Niederalkylrest $R_1$ verbunden sein und mit diesem zusammen einen 1-Aza-Niederalkylenrest bilden kann, dessen Azastickstoffatom mit dem 2- bzw. 6-Ringkohlenstoffatom des 1,4-Dihydro-pyridinrings verbunden

ist, oder, falls $R_2$ bzw. $R_3$ für ($C_1$—$C_7$)-Niederalkyl steht, dieses zusammen mit dem benachbarten Acylrest $Ac_1$ bzw. $Ac_2$ einen 2-Oxa-1-oxo-niederalkylenrest bilden kann, dessen Carbonylgruppe mit dem 3- bzw. 5-Ringkohlenstoffatom des 1,4-Dihydropyridinrings verbunden ist, und jeder der Reste $Ac_1$ und $Ac_2$ unabhängig voneinander für Niederalkanoyl, ($C_1$—$C_7$)-Niederalkylsulfonyl, ($C_1$—$C_7$)-Niederalkoxycarbonyl, Hydroxy-($C_1$—$C_7$)-niederalkoxycarbonyl, ($C_1$—$C_7$)-Niederalkoxy-($C_1$—$C_7$)-niederalkoxycarbonyl, Di-($C_1$—$C_7$)-niederalkylamino-($C_1$—$C_7$)-niederalkoxycarbonyl, N-($C_1$—$C_7$)-Niederalkyl-N-phenyl-($C_1$—$C_7$)-niederalkylamino-($C_1$—$C_7$)-niederalkoxycarbonyl, ($C_4$—$C_6$)-Niederalkylnamino-($C_1$—$C_7$)-niederalkoxycarbonyl, (4-Morpholino)-($C_1$—$C_7$)-niederalkoxycarbonyl, Carbamoyl, N-($C_1$—$C_7$)-Niederalkyl-carbamoyl, N,N-Di-($C_1$—$C_7$)-niederalkyl-carbamoyl, N,N-($C_4$—$C_5$)-Niederalkylen-carbamoyl oder 4- Morpholinocarbonyl steht,, wobei Niederalkyl, Niederalkoxy und Niederalkanoyl bis und mit 4 Kohlenstoffatome, Niederalkylen 4 oder 5 Kettenkohlenstoffatome, 1-Aza-niederalkylen 2 oder 3 Kettenkohlenstoffatome und 2-Oxa-1-oxo-niederalkylen 2 Kettenkohlenstoffatome enthalten, oder Salze von solchen Verbindungen mit salzbildenden Gruppen.

4. Verbindungen der Formel I gemäss Anspruch 1, worin Py für N-Oxidopyridyl steht, $R_1$ Wasserstoff, ($C_1$—$C_4$)-Niederalkyl, 2-(Di-($C_1$—$C_7$)-niederalkylamino)-($C_1$—$C_4$)-niederalkyl, 2-($C_4$—$C_6$)-(Niederalkylenamino)-($C_1$—$C_4$)-niederalkyl, oder 2-(4-Morpholino)-($C_1$—$C_4$)-niederalkyl bedeutet, einer der Reste $R_2$ und $R_3$ ($C_1$—$C_4$)-Niederalkyl darstellt, und der andere ($C_1$—$C_4$)-Niederalkyl, ($C_1$—$C_7$)-Hydroxyniederalkyl, ($C_1$—$C_7$)-Halogenniederalkyl, 2-(Di-($C_1$—$C_7$)-niederalkylamino)-($C_1$—$C_4$)-niederalkyl, ($C_1$—$C_7$)-Niederalkoxycarbonyl, Cyan, Amino, (4-Morpholino)-($C_1$—$C_7$)-niederalkylamino, ($C_4$—$C_6$)-Niederalkylenamino, (2-Oxo-1-imidazolidinyl)-($C_4$—$C_6$)-niederalkylenamino oder 4-(2-Furoyl)-piperazino bedeutet, wobei eine Aminogruppe $R_2$ bzw. $R_3$ mit einem ($C_1$—$C_4$)-Niederalkylrest $R_1$ verbunden sein und zusammen mit diesem einen 1-Aza-($C_2$—$C_3$)-niederalkylrest bilden kann, dessen Azastickstoffatom mit dem 2- bzw. 6-Ringkohlenstoffatomen des 1,4-Dihydro-pyridinrings verbunden ist, oder, falls $R_2$ bzw. $R_3$ für ($C_1$—$C_7$)-Niederalkyl steht, dieses zusammen mit dem benachbarten Acylrest $Ac_1$ bzw. $Ac_2$ einen 2-Oxa-1-oxo-($C_2$)-niederalkylenrest bilden kann, dessen Carbonylgruppe mit dem 3- bzw. 5-Ringkohlenstoffatom des 1,4-Dihydropyridinrings verbunden ist, und jeder der Reste $Ac_1$ und $Ac_2$ unabhängig voneinander ($C_1$—$C_4$)-Niederalkanoyl, ($C_1$—$C_7$)-Niederalkylsulfonyl, ($C_1$—$C_7$)-Niederalkoxycarbonyl, 2-($C_1$—$C_4$)-Niederalkoxy-($C_1$—$C_4$)-niederalkoxy-carbonyl, Di-($C_1$—$C_7$)-niederalkylamino-($C_1$—$C_7$)-niederalkoxycarbonyl oder N-($C_1$—$C_4$)-Niederalkyl-N-phenyl-($C_1$—$C_4$)-niederalkyl-amino-($C_1$—$C_7$)-niederalkoxycarbonyl, Carbamoyl, N-($C_1$—$C_4$)-Niederalkyl-carbamoyl, N,N-Di-($C_1$—$C_7$)-niederalkyl-carbamoyl oder 4-Morpholinocarbonyl bedeutet, wobei Niederalkyl, Niederalkoxy und Niederalkanoyl bis und mit 4 Kohlenstoffatome, Niederalkylen 4 oder 5 Kettenkohlenstoffatome, 1-Aza-niederalkylen 2 oder 3 Kettenkohlenstoffatome und 2-Oxa-1-oxo-niederalkylen 2 Kettenkohlenstoffatome enthalten, oder Salze von solchen Verbindungen mit salzbildenden basischen Gruppen.

5. Verbindung der Formel I gemäss Anspruch 1, worin Py für N-Oxidopyridyl steht, $R_1$ Wasserstoff oder 2-(4-Morpholino)-äthyl bedeutet, einer der Reste $R_2$ und $R_3$ Methyl darstellt, und der andere Methyl, Hydroxymethyl, ($C_1$—$C_4$)-Niederalkyoxycarbonyl, z.B. Methoxycarbonyl, Aethoxycarbonyl, n-Propyloxycarbonyl, Isopropyloxycarbonyl, n-Butyloxycarbonyl oder Isobutyloxycarbonyl, Cyan oder Amino bedeutet, und jeder der Reste $Ac_1$ und $Ac_2$ unabhängig voneinander Niederalkanoyl, z.B. Acetyl, ($C_1$—$C_7$)-Niederalkylsulfonyl, z.B. Aethylsulfonyl, Niederalkoxycarbonyl, z.B. Methoxycarbonyl, Aethoxycarbonyl, n-Propyloxycarbonyl, Isopropyloxycarbonyl, n-Butyloxycarbonyl oder Isobutyloxycarbonyl, Carbamoyl, N-Niederalkyl-carbamoyl, z.B. N-Methylcarbamoyl, oder N,N-Diniederalkyl-carbamoyl, z.B. N,N-Dimethylcarbamoyl, darstellt, wobei Niederalkyl, Niederalkoxy und Niederalkanoyl bis und mit 4 Kohlenstoffatome enthalten, oder Salze von solchen Verbindungen mit salzbildenden basischen Gruppen.

6. 2,6-Dimethyl-4-(1-oxido-3-pyridyl)-1,4-dihydro-pyridin-3,5-dicarbonsäure-dimethylester.

7. 2,6-Dimethyl-4-(1-oxido-3-pyridyl)-1,4-dihydro-pyridin-3,5-dicarbonsäure-diäthylester.

8. 2,6-Dimethyl-4-(1-oxido-3-pyridyl)-1,4-dihydro-pyridin-3-carbonsäureisobutylester-5-carbonsäuremethylester.

9. 2,6-Dimethyl-4-(1-oxido-2-pyridyl)-1,4-dihydro-pyridin-3,5-dicarbonsäure-diäthylester.

10. 2,6-Dimethyl-4-(1-oxido-4-pyridyl)-1,4-dihydro-pyridin-3,5-dicarbonsäure-diäthylester.

11. 2,6-Dimethyl-4-(1-oxido-3-pyridyl)-1,4-dihydro-pyridin-3,5-dicarbonsäure-di-n-propylester.

12. 2,6-Dimethyl-4-(2-methyl-1-oxido-3-pyridyl)-1,4-dihydro-pyridin-3,5-dicarbonsäure-diäthylester.

13. 2,6-Dimethyl-4-(2-chlor-1-oxido-3-pyridyl)-1,4-dihydro-pyridin-3,5-dicarbonsäure-diäthylester.

14. 2,6-Dimethyl-4-(1-oxido-3-pyridyl)-1,4-dihydro-pyridin-3-carbonsäure-isopropylester-5-carbonsäure-(2-methoxyäthyl)-ester.

15. 2,6-Dimethyl-4-(1-oxido-3-pyridyl)-1,4-dihydro-pyridin-3-carbonsäuremethylester-5-carbonsäure-[2-(N-methyl-N-benzyl-amino)-äthyl]-ester.

16. 6-Methoxymethyl-2-methyl-4-(1-oxido-3-pyridyl)-1,4-dihydro-pyridin-3-carbonsäureäthylester-5-carbonsäuremethylester.

17. 2-Amino-6-methyl-4-(1-oxido-3-pyridyl)-1,4-dihydro-pyridin-3,5-pyridin-dicarbonsäure-diäthylester.

18. 2,6-Dimethyl-4-(2-methylthio-1-oxido-3-pyridyl)-1,4-dihydro-pyridin-3,5-dicarbonsäure-diäthylester.

19. 2,6-Dimethyl-4-(2-methyl-1-oxido-3-pyridyl)-1,4-dihydro-pyridin-3-carbonsäureisobutylester-5-carbonsäuremethylester.

20. 6-Hydroxymethyl-2-methyl-4-(1-oxido-2-methyl-3-pyridyl)-1,4-dihydropyridin-3,5-dicarbonsäure-diäthylester.

21. 6-Cyan-2-methyl-4-(1-oxido-2-methyl-3-pyridyl)-1,4-dihydro-pyridin-3,5-dicarbonsäurediäthylester.

22. 2,6-Dimethyl-4-(1-oxido-2-methoxy-3-pyridyl)-1,4-dihydro-pyridin-3,5-dicarbonsäure-diäthylester.

23. 2,6-Dimethyl-4-(2-methylsulfinyl-1-oxido-3-pyridyl)-1,4-dihydro-pyridin-3,5-dicarbonsäure-diäthylester.

24. 2-Methyl-4-(1-oxido-3-pyridyl)-5-oxo-1,4,5,7-tetrahydro-furo[3,4,-b]pyridin-3-carbonsäureäthylester.

25. 2-Methyl-4-(1-oxido-2-chlor-3-pyridyl)-5-oxo-1,4,5,7-tetrahydro-furo[3,4-b]pyridin-3-carbonsäureäthylester.

26. 2-Methyl-4-(1-oxido-2-methyl-3-pyridyl)-5-oxo-1,4,5,7-tetrahydrofuro[3,4-b]pyridin-3-carbonsäureäthylester.

27. 2-Methyl-4-(1-oxido-2-methylthio-3-pyridyl)-5-oxo-1,4,5,7-tetrahydro-furo[3,4-b]pyridin-3-carbonsäureäthylester.

28. Die Verbindungen der Ansprüche 1 und 16 als pharmakologisch, insbesondere im cardiovasculären Bereich, aktive Verbindungen.

29. Die Verbindungen der Ansprüche 2, 6—15 und 17 als pharmakologisch, insbesondere im cardiovasculären Bereich, aktive Verbindungen.

30. Die Verbindungen der Ansprüche 1, 3—5 und 17—27 also pharmakologisch, insbesondere im cardiovasculären Bereich, aktive Verbindungen.

31. Die Verbindungen des Anspruchs 1, mit Ausnahme derer, worin $R_2$ bzw. $R_3$ für ($C_1$—$C_7$) Niederalkyl steht, und dieses zusammen mit dem benachbarten Acylrest $Ac_1$ bzw. $Ac_2$ einen 2-Oxa-1-oxo-($C_2$—$C_3$)-niederalkylenrest bildet, wobei dessen Carbonylgruppe mit dem 3- bzw. 5-Ringkohlenstoffatom des 1,4-Dihydropyridinrings verbunden ist, sowie des Anspruchs 16, als pharmakologisch aktive, insbesondere blutdrucksenkende Verbindungen.

32. Die Verbindungen des Anspruchs 2, mit Ausnahme derer, worin $R_2$ bzw. $R_3$ für ($C_1$—$C_7$)-Niederalkyl steht, und dieses zusammen mit dem benachbarten Acylrest $Ac_1$ bzw. $Ac_2$ einen 2-Oxa-1-oxo-($C_2$—$C_3$)-niederalkylenrest bildet, wobei dessen Carbonylgruppe mit dem 3- bzw. 5-Ringkohlenstoffatomn des 1,4-Dihydropyridinrings verbunden ist, sowie der Ansprüche 6—15 und 17 als pharmakologisch aktive, insbesondere blutdrucksenkende Verbindungen.

33. Die Verbindungen der Ansprüche 1 und 3—5, mit Ausnahme derer, worin $R_2$ bzw. $R_3$ für ($C_1$—$C_7$)-Niederalkyl bzw. Methyl steht, und dieses zusammen mit dem benachbarten Acylrest $Ac_1$ bzw. $Ac_2$ einen 2-Oxa-1-oxo-($C_2$—$C_3$)-niederalkylenrest bzw. einen 2-Oxa-1-oxo-1,3-propylenrest bildet, wobei deren Carbonylgruppe jeweils mit dem 3- bzw. 5-Ringkohlenstoffatom des 1,4-Dihydropyridinrings verbunden ist, sowie der Ansprüche 18—23 als pharmakologisch aktive, insbesondere blutdrucksenkende Verbindungen.

34. Die Verbindungen des Anspruchs 1, worin $R_2$ bzw. $R_3$ für ($C_1$—$C_7$)-Niederalkyl steht, und dieses zusammen mit dem benachbarten Acylrest $Ac_1$ bzw. $Ac_2$ einen 2-Oxa-1-oxo-($C_2$—$C_3$)-niederalkylenrest bildet, dessen Carbonylgruppe mit dem 3- bzw. 5-Ringkohlenstoffatom des 1,4-Dihydropyridinrings verbunden ist, sowie des Anspruchs 16 als pharmakologisch aktive insbesondere die myocardiale Kontraktilität steigernde Verbindungen.

35. Die Verbindungen des Anspruchs 2, worin $R_2$ bzw. $R_3$ für ($C_1$—$C_7$)-Niederalkyl steht, und dieses zusammen mit dem benachbarten Acylrest $Ac_1$ bzw. $Ac_2$ einen 2-Oxa-1-oxo-($C_2$—$C_3$)-niederalkylenrest bildet, dessen Carbonylgruppe mit dem 3- bzw. 5-Ringkohlenstoffatom des 1,4-Dihydropyridinrings verbunden ist, sowie der Ansprüche 6—15 und 17 als pharmakologisch aktive, insbesondere die myocardiale Kontraktilität steigernde Verbindungen.

36. Die Verbindungen der Ansprüche 1 und 3—5, worin $R_2$ bzw. $R_3$ für ($C_1$—$C_7$)-Niederalkyl steht, und dieses zusammen mit dem benachbarten Acylrest $Ac_1$ bzw. $Ac_2$ einen 2-Oxa-1-oxo-($C_2$—$C_3$)-niederalkylenrest bildet, dessen Carbonylgruppe mit dem 3- bzw. 5-Ringkohlenstoffatom des 1,4-Dihydropyridinrings verbunden ist, sowie der Ansprüche 26 bis 27 als pharmakologisch aktive, insbesondere die myocardiale Kontraktilität steigernde Verbindungen.

37. Die Verbindung des Anspruchs 16, zur Behandlung des menschlichen oder tierischen Körpers.

38. Die Verbindungen der Ansprüche 2, 6—15 und 17, zur Behandlung des menschlichen oder tierischen Körpers.

39. Die Verbindungen der Ansprüche 1, 3—7 und 18—27, zur Behandlung des menschlichen oder tierischen Körpers.

40. Verwendung der Verbindung des Anspruchs 16 oder von pharmazeutisch verwendbaren Salzen davon zur Herstellung von pharmazeutischen Präparaten zur prophylaktischen und/oder therapeutischen Behandlung von Hypertonie einerseits und von anderen cardiovasculären Krankkeiten andererseits.

41. Verwendung von Verbindungen der Ansprüche 2, 6—15 und 17 oder von pharmazeutisch verwendbaren Salzen davon zur Herstellung von pharmazeutischen Präparaten zur prophylaktischen und/oder therapeutischen Behandlung von Hypertonie einerseits und von anderen cardiovasculären Krankheiten andererseits.

42. Verwendung von Verbindungen der Ansprüche 1, 3—5 und 18—23 oder von pharmazeutisch verwendbaren Salzen davon zur Herstellung von pharmazeutischen Präparaten zur prophylaktischen und/oder therapeutischen Behandlung von Hypertonie einerseits und von anderen cardiovasculären Krankheiten andererseits.

43. Pharmazeutische Präparate enthaltend die Verbindung des Anspruchs 16.

44. Pharmazeutische Präparate enthaltend eine der Verbindungen der Ansprüche 2, 6—15 und 17.

45. Pharmazeutische Präparate enthaltend eine der Verbindungen der Ansprüche 1, 3—5 und 18—27.

46. Verwendung der Verbindung des Anspruchs 16, zur Herstellung von pharmazeutischen Präparaten.

47. Verwendung der Verbindungen der Ansprüche 2, 6—15 und 17, zur Herstellung von pharmazeutischen Präparaten.

48. Verwendung von Verbindungen der Ansprüche 1, 3—5 und 18—27, zur Herstellung von pharmazeutischen Präparaten.

49. Verfahren zur Herstellung von pharmazeutischen Präparaten, dadurch gekennzeichnet, dass man die Verbindung des Anspruchs 16 gegebenenfalls unter Beimischung von Hilfsstoffen, zu pharmazeutischen Präparaten verarbeitet.

50. Verfahren zur Herstellung von pharmazeutischen Präparaten, dadurch gekennzeichnet, dass man Verbindungen der Ansprüche 2, 6—15 und 17, gegebenenfalls unter Beimischung von Hilfsstoffen, zu pharmazeutischen Präparaten verarbeitet.

51. Verfahren zur Herstellung von pharmazeutischen Präparaten, dadurch gekennzeichnet, dass man Verbindungen der Ansprüche 1, 3—5 und 18—27, gegebenenfalls unter Beimischung von Hilfsstoffen zu pharmazeutischen Präparaten verarbeitet.

52. Verfahren zur Herstellung von in den Ansprüchen 1 und 16 definierten Verbindungen der Formel I, dadurch gekennzeichnet, dass man

a) eine Verbindung der Formel

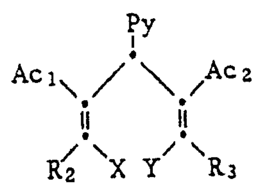

(II)

worin einer der Reste X und Y für die Gruppe der Formel —NH—$R_1$ steht und der andere Hydroxy oder die Gruppe der Formel —NH—$R_1$ bedeutet, oder ein Tautomeres davon oder ein ensprechendes Tautomerengemisch ringschliesst, oder

b) eine Verbindung der Formel Py-CHO (III) oder ein reaktionsfähiges funktionelles Derivat davon mit einer Verbindung der Formel

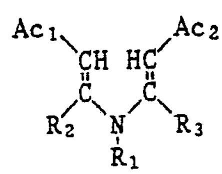

(IV)

oder einem Tautomeren davon oder einem entsprechenden Tautomerengemisch umsetzt, oder

c) in einer Verbindung der Formel

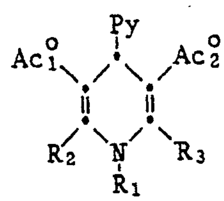

(V)

in welcher einer der Reste $Ac_1^o$ und $Ac_1^o$ einen in den Rest $Ac_1$ bzw. $Ac_2$ überführbaren Rest bedeutet, und der andere $Ac_1$ bzw. $Ac_2$ oder einen in einen $Ac_1$ bzw. $Ac_2$ überführbaren Rest bedeutet, den Rest $Ac_1^o$ und/oder $Ac_2^o$ in einen Rest $Ac_1$ und/oder $Ac_2$ überführt, oder

d) in einer Verbindung der Formel

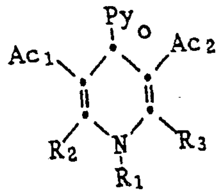

(VI)

worin $Py_o$ für einen unsubstituierten oder substituierten Pyridylrest steht, den Rest $Py_o$ zum entsprechenden N-Oxido-pyridylrest oxidiert, wobei die Bildung von Verbindungen der Formel I, worin $R_2$ bzw. $R_3$ als ($C_1$—$C_7$)-Niederalkyl zusammen mit dem benachbarten Acylrest $Ac_1$ bzw. $Ac_2$ einen 2-Oxa-1-oxo-($C_2$—$C_3$)-niederalkylenrest darstellen, dessen Carbonylgruppe mit dem 3- bzw. 5-Ringkohlenstoffatomen des 1,4-Dihydropyridinrings verbunden ist, aus Ausgangsstoffen der Formeln II, IV, V und VI, worin $R_2$ bzw. $R_3$ freies oder verestertes Hydroxy enthaltendes Niederalkyl bedeutet, und $Ac_1$ bzw. $Ac_2$, oder $Ac_1^o$ bzw. $Ac_1^o$ in Ausgangsstoffen der Formel V, eine veresterte Carboxylgruppe bedeutet, unmittelbar unter den Reaktionsbedingungen eines der Verfahren a)—d) erfolgen kann, und wobei in den obigen Ausgangsstoffen der Formeln II bis IV die, sofern sie salzbildende Eigenschaften aufweisen, auch in Form ihrer Salze verwendet werden können, die Reste Py, $R_1$, $R_2$, $R_3$, $Ac_1$ und $Ac_2$ die unter der Formel I gegebenen Bedeutungen haben, und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt, und/oder, wenn erwünscht, ein erhaltenes Gemisch von Isomeren in die einzelnen Isomeren auftrennt.

53. Verfahren zur Herstellung von in den Ansprüchen 2, 6—15 und 17 definierten Verbindungen der Formel I, dadurch gekennzeichnet, dass die im Anspruch 52 definierten Verfahren mit entsprechenden Ausgangsstoffen durchgeführt werden.

54. Verfahren zur Herstellung von in den Ansprüchen 1, 3—5 und 18—27 definierten Verbindungen der Formel I, dadurch gekennzeichnet, dass die im Anspruch 52 definierten Verfahren mit entsprechenden Ausgangsstoffen durchgeführt werden.

**Patentansprüche für den Vertragsstaat: AT**

1. Verfahren zur Herstellung von Verbindungen der Formel

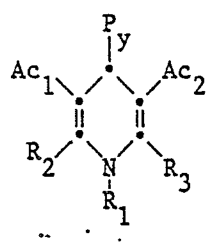

(I),

in welcher Py unsubstituiertes oder durch ($C_1$—$C_7$)-Niederalkyl, ($C_1$—$C_7$)-Niederalkoxy, ($C_1$—$C_7$)-Niederalkylthio, ($C_1$—$C_7$)-Niederalkylsulfinyl, ($C_1$—$C_7$)-Niederalkylsulfonyl und/oder Halogen substituiertes N-Oxidopyridyl bedeutet, $R_1$ Wasserstoff, ($C_1$—$C_7$)-Niederalkyl, Di-($C_1$—$C_7$)-niederalkylamino-($C_1$—$C_7$)-niederalkyl, ($C_4$—$C_6$)-Niederalkylenamino-($C_1$—$C_7$)-niederalkyl, Morpholino-($C_1$—$C_7$)-niederalkyl, Thiomorpholino-($C_1$—$C_7$)-niederalkyl, Piperazino-($C_1$—$C_7$)-niederalkyl oder 4-($C_1$—$C_7$)-Niederalkyl-piperazino-($C_1$—$C_7$)-niederalkyl darstellt, einer der Reste $R_2$ und $R_3$ ($C_1$—$C_7$)-Niederalkyl bedeutet, und der andere für Wasserstoff, ($C_1$—$C_7$)-Niederalkyl, Hydroxy-($C_1$—$C_7$)-niederalkyl, ($C_1$—$C_7$)-Niederalkoxy-($C_1$—$C_7$)-niederalkyl, gem-Di-($C_1$—$C_7$)-niederalkoxy-($C_1$—$C_7$)-niederalkyl, Halogen-($C_1$—$C_7$)-niederalkyl, Oxo-($C_1$—$C_7$)-niederalkyl, ($C_1$—$C_7$)-Niederalkoxycarbonyl-($C_1$—$C_7$)-niederalkyl, Carbamoyl-($C_1$—$C_7$)-niederalkyl, N-($C_1$—$C_7$)-Niederalkylcarbamoyl-($C_1$—$C_7$)-niederalkyl, N,N-Di-($C_1$—$C_7$)-niederalkylcarbamoyl-($C_1$—$C_7$)-niederalkyl, Cyan-($C_1$—$C_7$)-niederalkyl, Amino-($C_1$—$C_7$)-niederalkyl, ($C_1$—$C_7$)-Niederalkylamino-($C_1$—$C_7$)-niederalkyl, Di-($C_1$—$C_7$)-niederalkylamino-($C_1$—$C_7$)-niederalkyl, ($C_4$—$C_6$)-Niederalkylamino-($C_1$—$C_7$)-niederalkyl, Morpholino-($C_1$—$C_7$)-niederalkyl, Thiomorpholino-($C_1$—$C_7$)-niederalkyl, Piperazino-($C_1$—$C_7$)-niederalkyl, 4-($C_1$—$C_7$)-Niederalkyl-piperazino-($C_1$—$C_7$)-niederalkyl, ($C_1$—$C_7$)-Niederalkoxyimino-($C_1$—$C_7$)-niederalkyl, worin ($C_1$—$C_7$)-Niederalkoxy und Imino das gleiche Kohlenstoffatom substituieren, Hydroxyimino-($C_1$—$C_7$)-niederalkyl, ($C_1$—$C_7$)-Niederalkoxyimino-($C_1$—$C_7$)-niederalkyl, ($C_1$—$C_7$)-Niederalkoxycarbonyl, Carbamoyl, N-($C_1$—$C_7$)-Niederalkylcarbamoyl, N,N-Di-($C_1$—$C_7$)-niederalkylcarbamoyl, Cyan, Amino, ($C_1$—$C_7$)-Niederalkylamino, Di-($C_1$—$C_7$)-niederalkylamino-($C_1$—$C_7$)-niederalkylamino, ($C_4$—$C_6$)-Niederalkylamino-($C_1$—$C_7$)-niederalkylamino, Morpholino-($C_1$—$C_7$)-niederalkylamino, Di-($C_1$—$C_7$)-niederalkylamino, ($C_4$—$C_6$)-Niederalkylenamino, (2-Oxo-1-imidazolidinyl)-

$(C_4—C_6)$-niederalkylenamino, Morpholino, Thiomorpholino, Piperazino, 4-$(C_1—C_7)$-Niederalkyl-piperazino, 4-$(C_1—C_7)$-Niederalkanoyl-piperazino, 4-Benzoyl-piperazino, 4-Furoyl-piperazino oder 4-Thienoyl-piperazino bedeutet, wobei eine Aminogruppe $R_2$ bzw. $R_3$ auch mit einem Niederalkylrest $R_1$ verbunden sein und mit diesem zusammen einen 1-Aza-$(C_3—C_4)$-niederalkylenrest bilden kann, dessen Stickstoffatom mit dem 2- bzw. 6-Ringkohlenstoffatom des 1,4-Dihydro-pyridinrings verbunden ist, oder, falls $R_2$ bzw. $R_3$ für $(C_1—C_7)$-Niederalkyl steht, dieses zusammen mit dem benachbarten Acylrest $Ac_1$ bzw. $Ac_2$ einen 2-Oxa-1-oxo-$(C_2—C_3)$-niederalkylenrest bilden kann, dessen Carbonylgruppe mir dem 3- bzw. 5-Ringkohlenstoffatom des 1,4-Dihydro-pyridinrings verbunden ist, und jeder der Reste $Ac_1$ und $Ac_2$ unabhängig voneinander für $(C_1—C_7)$-Niederalkanoyl, unsubstituiertes oder durch $(C_1—C_7)$-Niederalkyl, $(C_1—C_7)$-Niederalkoxy und/oder Halogen substituiertes Benzoyl, $(C_1—C_7)$-Niederalkylsulfonyl, unsubstituiertes oder durch $(C_1—C_7)$-Niederalkyl, $(C_1—C_7)$-Niederalkoxy und/oder Halogen substituiertes Phenylsulfonyl, $(C_1—C_7)$-Niederalkoxycarbonyl, Hydroxy-$(C_1—C_7)$-niederalkoxycarbonyl, $(C_1—C_7)$-Niederalkoxy-$(C_1—C_7)$-niederalkoxycarbonyl, Amino-$(C_1—C_7)$-niederalkoxycarbonyl, $(C_1—C_7)$-Niederalkylamino-$(C_1—C_7)$-niederalkoxycarbonyl, Di-$(C_1—C_7)$-niederalkylamino-$(C_1—C_7)$-niederalkoxycarbonyl, N-$(C_1—C_7)$-Niederalkyl-N-phenyl-$(C_1—C_7)$-niederalkyl-amino-$(C_1—C_7)$-niederalkoxycarbonyl, $(C_4—C_6)$-Niederalkylenamino-$(C_1—C_7)$-niederalkoxycarbonyl, Morpholino-$(C_1—C_7)$-niederalkoxycarbonyl, Thiomorpholino-$(C_1—C_7)$-niederalkoxycarbonyl, Piperazino-$(C_1—C_7)$-niederalkoxycarbonyl, 4-$(C_1—C_7)$-Niederalkyl-piperazino-$(C_1—C_7)$-niederalkoxycarbonyl, unsubstituiertes oder durch $(C_1—C_7)$-Niederalkyl, $(C_1—C_7)$-Niederalkoxy und/oder Halogen substituiertes Phenyl-$(C_1—C_7)$-niederalkoxycarbonyl, Carbamoyl, N-$(C_1—C_7)$-Niederalkyl-carbamoyl, N,N-Di-$(C_1—C_7)$-niederalkyl-carbamoyl, N,N-$(C_4—C_6)$-Niederalkylcarbamoyl, Morpholinocarbonyl, Thiomorpholinocarbonyl, Piperazinocarbonyl oder 4-$(C_1—C_7)$-Niederalkyl-piperazinocarbonyl steht, oder Salze von solchen Verbindungen mit salzbildenden Gruppen, dadurch gekennzeichnet, dass man

a) eine Verbindung der Formel

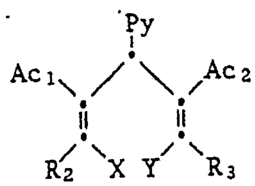

(II)

worin einer der Reste X und Y für die Gruppe der Formel —NH—$R_1$ steht und der andere Hydroxy oder die Gruppe der Formel —NH—$R_1$ bedeutet, oder ein Tautomeres davon oder ein ensprechendes Tautomerengemisch ringschliesst, oder

b) eine Verbindung der Formel Py-CHO (III) oder ein reaktionsfähiges funktionelles Derivat davon mit einer Verbindung der Formel

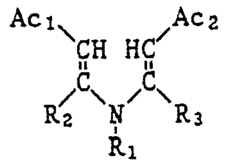

(IV)

oder einem Tautomeren davon oder einem entsprechenden Tautomerengemisch umsetzt, oder

c) in einer Verbindung der Formel

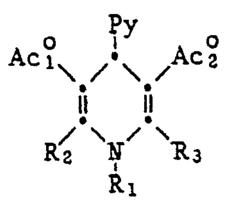

(V)

in welcher einer der Reste $Ac_1^o$ und $Ac_2^o$ einen in den Rest $Ac_1$ bzw. $Ac_2$ überführbaren Rest bedeutet, und der andere $Ac_1$ bzw. $Ac_2$ oder einen in einen $Ac_1$ bzw. $Ac_2$ überführbaren Rest bedeutet, den Rest $Ac_1^o$ und/oder $Ac_2^o$ in einen Rest $Ac_1$ und/oder $Ac_2$ überführt, oder

d) in einer Verbindung der Formel

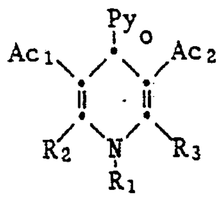

(VI)

worin $Py_o$ für einen unsubstituierten oder substituierten Pyridylrest steht, den Rest $Py_o$ zum entsprechenden N-Oxido-pyridylrest oxidiert, wobei die Bildung von Verbindungen der Formel I, worin $R_2$ bzw. $R_3$ als $(C_1—C_7)$-Niederalkyl zusammen mit dem benachbarten Acylrest $Ac_1$ bzw. $Ac_2$ einen 2-Oxa-1-oxo-$(C_2—C_3)$-niederalkylenrest darstellen, dessen Carbonylgruppe mit dem 3- bzw. 5-Ringkohlenstoffatomen des 1,4-Dihydropyridinrings verbunden ist, aus Ausgangsstoffen der Formeln II, IV, V und VI, worin $R_2$ bzw. $R_3$ freies oder verestertes Hydroxy enthaltendes Niederalkyl bedeutet, und $Ac_1$ bzw. $Ac_2$, oder $Ac_1^o$ bzw. $Ac_2^o$ in Ausgangsstoffen der Formel V, eine veresterte Carboxylgruppe bedeutet, unmittelbar unter den Reaktionsbedingungen eines der Verfahren a)—d) erfolgen kann, und wobei in den obigen Ausgangsstoffen der Formeln II bis IV die, sofern sie salzbildende Eigenschaften aufweisen, auch in Form ihrer Salze verwendet werden können, die Reste Py, $R_1$, $R_2$, $R_3$, $Ac_1$ und $Ac_2$ die unter der Formel I gegebenen Bedeutungen haben, und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt, und/oder, wenn erwünscht, ein erhaltenes Gemisch von Isomeren in die einzelnen Isomeren auftrennt.

2. Verfahren nach Anspruch 1 zur Herstellung von Verbindungen der Formel I worin Py, $R_1$, $Ac_1$ und $Ac_2$ die im Anspruch 1 gegebenen Bedeutungen haben, und einer der Reste $R_2$ und $R_3$ $(C_1—C_7)$-Niederalkyl bedeutet, und der andere für Wasserstoff, $(C_1—C_7)$-Niederalkyl, Hydroxy-$(C_1—C_7)$-niederalkyl, $(C_1—C_7)$-Niederalkoxy-$(C_1—C_7)$-niederalkyl, Halogen-$(C_1—C_7)$-niederalkyl, $(C_1—C_7)$-Niederalkoxycarbonyl-$(C_1—C_7)$-niederalkyl, Carbamoyl-$(C_1—C_7)$-niederalkyl, N-$(C_1—C_7)$-Niederalkylcarbamoyl-$(C_1—C_7)$-niederalkyl, N,N-Di-$(C_1—C_7)$-niederalkylcarbamoyl-$(C_1—C_7)$-niederalkyl, Cyan-$(C_1—C_7)$-niederalkyl, $(C_1—C_7)$-Aminoniederalkyl, $(C_1—C_7)$-Niederalkylamino-$(C_1—C_7)$-niederalkyl, Di-$(C_1—C_7)$-niederalkylamino-$(C_1—C_7)$-niederalkyl, $(C_4—C_6)$-Niederalkylenamino-$(C_1—C_7)$-niederalkyl, Morpholino-$(C_1—C_7)$-niederalkyl, Thiomorpholino-$(C_1—C_7)$-niederalkyl, Piperazino-$(C_1—C_7)$-niederalkyl, 4-$(C_1—C_7)$-Niederalkyl-piperazino-$(C_1—C_7)$-niederalkyl, $(C_1—C_7)$-Niederalkoxycarbonyl, Carbamoyl, N-$(C_1—C_7)$-Niederalkylcarbamoyl, N,N-Di-$(C_1—C_7)$-niederalkylcarbamoyl, Cyan, Amino, $(C_1—C_7)$-Niederalkylamino, Di-$(C_1—C_7)$-niederalkylamino-$(C_1—C_7)$-niederalkylamino, $(C_4—C_6)$-Niederalkylenamino-$(C_1—C_7)$-niederalkylamino, Morpholino-$(C_1—C_7)$-niederalkylamino, Di-$(C_1—C_7)$-niederalkylamino, $(C_4—C_6)$-Niederalkylenamino, (2-Oxo-1-imidazolidinyl)-piperidino, Morpholino, Thiomorpholino, Piperazino, 4-$(C_1—C_7)$-Niederalkyl-piperazino, 4-$(C_1—C_7)$-Niederalkanoyl-piperazino, 4-Benzoyl-piperazino, 4-Furoyl-piperazino oder 4-Thienoyl-piperazino bedeutet, wobei eine Aminogruppe $R_2$ bzw. $R_3$ auch mit einem $(C_1—C_7)$-Niederalkylrest $R_1$ verbunden sein und mit diesem zusammen einen 1-Aza-$(C_3—C_4)$-niederalkylenrest bilden kann, dessen Stickstoffatom mit dem 2- bzw. 6-Ringkohlenstoffatom des 1,4-Dihydro-pyridinrings verbunden ist, oder, falls $R_2$ bzw. $R_3$ für $(C_1—C_7)$-Niederalkyl steht, dieses zusammen mit dem benachbarten Acylrest $Ac_1$ bzw. $Ac_2$ einen 2-Oxa-1-oxo-$(C_2—C_3)$-niederalkylenrest bilden kann, dessen Carbonylgruppe mit dem 3- bzw. 5-Ringkohlenstoffatomen des 1,4-Dihydro-pyridinrings verbunden ist, oder Salze von solchen Verbindungen mit salzbildenden Gruppen.

3. Verfahren nach Anspruch 1 zur Herstellung von Verbindungen der Formel I, worin Py unsubstituiertes oder durch $(C_1—C_4)$-Niederalkyl oder $(C_1—C_7)$-Niederalkylsulfinyl substituiertes N-Oxidopyridyl bedeutet, $R_1$ Wasserstoff, $(C_1—C_4)$-Niederalkyl, 2-Di-$(C_1—C_7)$-niederalkylamino-$(C_1—C_7)$-niederalkyl, 2-$(C_4—C_6)$-Niederalkylenamino-$(C_1—C_7)$-niederalkyl oder 2-(4-Morpholino)-$(C_1—C_7)$-niederalkyl darstellt, einer der Reste $R_2$ und $R_3$ für $(C_1—C_4)$-Niederalkyl steht, und der andere Wasserstoff, $(C_1—C_7)$-Niederalkyl, Hydroxy-$(C_1—C_7)$-niederalkyl, Halogen-$(C_1—C_7)$-niederalkyl, $(C_1—C_7)$-Niederalkoxycarbonyl-$(C_1—C_7)$-niederalkyl, $(C_1—C_7)$-Cyanniederalkyl, Di-$(C_1—C_7)$-niederalkylamino-$(C_1—C_7)$-niederalkyl, $(C_1—C_7)$-Niederalkoxycarbonyl, Cyan, Amino, (4-Morpholino)-$(C_1—C_7)$-niederalkylamino, $(C_4—C_6)$-Niederalkylenamino, (2-Oxo-1-imidazolidinyl)-piperazino, oder 4-(2-Furoyl)-piperazino bedeutet, wobei eine Aminogruppe $R_2$ bzw. $R_3$ mit einem $(C_1—C_7)$-Niederalkylrest $R_1$ verbunden sein und mit diesem zusammen einen 1-Aza-Niederalkylenrest bilden kann, dessen Azastickstoffatom mit dem 2- bzw. 6-Ringkohlenstoffatom des 1,4-Dihydro-pyridinrings verbunden ist, oder, falls $R_2$ bzw. $R_3$ für $(C_1—C_7)$-Niederalkyl steht, dieses zusammen mit dem benachbarten Acylrest $Ac_1$ bzw. $Ac_2$ einen 2-Oxa-1-oxo-niederalkylenrest bilden kann, dessen Carbonylgruppe mit dem 3- bzw. 5-Ringkohlenstoffatom des 1,4-dihydropyridinrings verbunden ist, und jeder der Reste $Ac_1$ und $Ac_2$ unabhängig voneinander für Niederalkanoyl, $(C_1—C_7)$-Niederalkylsulfonyl, $(C_1—C_7)$-Niederalkoxycarbonyl, Hydroxy-$(C_1—C_7)$-niederalkoxycarbonyl, $(C_1—C_7)$-Niederalkoxy-$(C_1—C_7)$-niederalkoxycarbonyl, Di-$(C_1—C_7)$-niederalkylamino-$(C_1—C_7)$-niederalkoxycarbonyl, N-$(C_1—C_7)$-Niederalkyl-N-phenyl-$(C_1—C_7)$-niederalkyl-

amino-($C_1$—$C_7$)-niederalkoxycarbonyl, ($C_4$—$C_6$)-Niederalkylenamino-($C_1$—$C_7$)-niederalkoxycarbonyl, (4-Morpholino)-($C_1$—$C_7$)-niederalkoxycarbonyl, Carbamoyl, N-($C_1$—$C_7$)-Niederalkyl-carbamoyl, N,N-Di-($C_1$—$C_7$)-niederalkyl-carbamoyl, N,N-($C_4$—$C_5$)-Niederalkylen-carbamoyl oder 4- Morpholinocarbonyl steht, wobei Niederalkyl, Niederalkoxy und Niederalkanoyl bis und mit 4 Kohlenstoffatome, Niederalkylen 4 oder 5 Kettenkohlenstoffatome, 1-Aza-niederalkylen 2 oder 3 Kettenkohlenstoffatome und 2-Oxa-1-oxo-niederalkylen 2 Kettenkohlenstoffatome enthalten, oder Salze von solchen Verbindungen mit salzbildenden Gruppen.

4. Verfahren nach Anspruch 1, zur Herstellung von Verbindungen der Formel I, worin Py für N-Oxidopyridyl steht, $R_1$ Wasserstoff, ($C_1$—$C_4$)-Niederalkyl, 2-(Di-($C_1$—$C_7$)-niederalkylamino)-($C_1$—$C_4$)-niederalkyl, 2-($C_4$—$C_6$)-(Niederalkylenamino)-($C_1$—$C_4$)-niederalkyl, oder 2-(4-Morpholino)-($C_1$—$C_4$)-niederalkyl bedeutet, einer der Reste $R_2$ und $R_3$ ($C_1$—$C_4$)-Niederalkyl darstellt, und der andere ($C_1$—$C_4$)-Niederalkyl, ($C_1$—$C_7$)-Hydroxyniederalkyl, ($C_1$—$C_7$)-Halogenniederalkyl, 2-(Di-($C_1$—$C_7$)-niederalkylamino)-($C_1$—$C_4$)-niederalkyl, ($C_1$—$C_7$)-Niederalkoxycarbonyl, Cyan, Amino, (4-Morpholino)-($C_1$—$C_7$)-niederalkylamino, ($C_4$—$C_6$)-Niederalkylenamino, (2-Oxo-1-imidazolidinyl)-($C_4$—$C_6$)-niederalkylenamino oder 4-(2-Furoyl)piperazino bedeutet, wobei eine Aminogruppe $R_2$ bzw. $R_3$ mit einem ($C_1$—$C_4$)-Niederalkylrest $R_1$ verbunden sein und zusammen mit diesem einen 1-Aza-($C_2$—$C_3$)-niederalkylrest bilden kann, dessen Azastickstoffatom mit dem 2- bzw. 6-Ringkohlenstoffatomen des 1,4-Dihydro-pyridinrings verbunden ist, oder, falls $R_2$ bzw. $R_3$ für ($C_1$—$C_7$)-Niederalkyl steht, diese zusammen mit dem benachbarten Acylrest $Ac_1$ bzw. $Ac_2$ einen 2-Oxa-1-oxo-($C_2$)-niederalkylenrest bilden kann, dessen Carbonylgruppe mit dem 3- bzw. 5-Ringkohlenstoffatom des 1,4-Dihydropyridinrings verbunden ist, und jeder der Reste $Ac_1$ und $Ac_2$ unabhängig voneinander ($C_1$—$C_4$)-Niederalkanoyl, ($C_1$—$C_7$)-Niederalkylsulfonyl, ($C_1$—$C_7$)-Niederalkoxycarbonyl, 2-($C_1$—$C_4$)-Niederalkoxy-($C_1$—$C_4$)-niederalkoxy-carbonyl, Di-($C_1$—$C_7$)-niederalkylamino-($C_1$—$C_7$)-niederalkoxycarbonyl oder N-($C_1$—$C_4$)-Niederalkyl-N-phenyl-($C_1$—$C_4$)-niederalkyl-amino-($C_1$—$C_7$)-niederalkoxycarbonyl, Carbamoyl, N-($C_1$—$C_4$)-Niederalkyl-carbamoyl, N,N-Di-($C_1$—$C_7$)-niederalkyl-carbamoyl oder 4-Morpholino-carbonyl bedeutet, wobei Niederalkyl, Niederalkoxy und Niederalkanoyl bis und mit 4 Kohlenstoffatome, Niederalkylen 4 oder 5 Kettenkohlenstoffatome, 1-Aza-niederalkylen 2 oder 3 Kettenkohlenstoffatome und 2-Oxa-1-oxo-niederalkylen 2 Kettenkohlenstoffatome enthalten, oder Salze von solchen Verbindungen mit salzbildenden basischen Gruppen.

5. Verfahren nach Anspruch 1 zur Herstellung von Verbindungen der Formel I, worin Py für N-Oxidopyridyl steht, $R_1$ Wasserstoff oder 2-(4-Morpholino)-äthyl bedeutet, einer der Reste $R_2$ und $R_3$ Methyl darstellt, und der andere Methyl, Hydroxymethyl, ($C_1$—$C_4$)-Niederalkyoxycarbonyl, z.B. Methoxycarbonyl, Aethoxycarbonyl, n-Propyloxycarbonyl, Isopropyloxycarbonyl, n-Butyloxycarbonyl oder Isobutyloxycarbonyl, Cyan oder Amino bedeutet, und jeder der Reste $Ac_1$ und $Ac_2$ unabhängig voneinander Niederalkanoyl, z.B. Acetyl, ($C_1$—$C_7$)-Niederalkylsulfonyl, z.B. Aethylsulfonyl, Niederalkoxycarbonyl, z.B. Methoxycarbonyl, Aethoxycarbonyl, n-Propyloxycarbonyl, Isopropyloxycarbonyl, n-Butyloxycarbonyl oder Isobutyloxycarbonyl, Carbamoyl, N-Niederalkyl-carbamoyl, z.B. N-Methylcarbamoyl, oder N,N-Diniederalkyl-carbamoyl, z.B. N,N-Dimethylcarbamoyl, darstellt, wobei Niederalkyl, Niederalkoxy und Niederalkanoyl bis und mit 4 Kohlenstoffatome enthalten, oder Salze von solchen Verbindungen mit salzbildenden basischen Gruppen.

6. Verfahren nach Anspruch 1 zur Herstellung von 2,6-Dimethyl-4-(1-oxido-3-pyridyl)-1,4-dihydro-pyridin-3,5-dicarbonsäure-dimethylester.

7. Verfahren nach Anspruch 1 zur Herstellung von 2,6-Dimethyl-4-(1-oxido-3-pyridyl)-1,4-dihydro-pyridin-3,5-dicarbonsäure-diäthylester.

8. Verfahren nach Anspruch 1 zur Herstellung von 2,6-Dimethyl-4-(1-oxido-3-pyridyl)-1,4-dihydro-pyridin-3-carbonsäureisobutylester-5-carbonsäuremethylester.

9. Verfahren nach Anspruch 1 zur Herstellung von 2,6-Dimethyl-4-(1-oxido-2-pyridyl)-1,4-dihydro-pyridin-3,5-dicarbonsäure-diäthylester.

10. Verfahren nach Anspruch 1 zur Herstellung von 2,6-Dimethyl-4-(1-oxido-4-pyridyl)-1,4-dihydro-pyridin-3,5-dicarbonsäure-diäthylester.

11. Verfahren nach Anspruch 1 zur Herstellung von 2,6-Dimethyl-4-(1-oxido-3-pyridyl)-1,4-dihydro-pyridin-3,5-dicarbonsäure-diäthylester.

12. Verfahren nach Anspruch 1 zur Herstellung von 2,6-Dimethyl-4-(2-methyl-1-oxido-3-pyridyl)-1,4-dihydro-pyridin-3,5-dicarbonsäure-diäthylester.

13. Verfahren nach Anspruch 1 zur Herstellung von 2,6-Dimethyl-4-(2-chlor-1-oxido-3-pyridyl)-1,4-dihydro-pyridin-3,5-dicarbonsäure-diäthylester.

14. Verfahren nach Anspruch 1 zur Herstellung von 2,6-Dimethyl-4-(1-oxido-3-pyridyl)-1,4-dihydro-pyridin-3-carbonsäure-isopropylester-5-carbonsäure-(2-methoxyäthyl)-ester.

15. Verfahren nach Anspruch 1 zur Herstellung von 2,6-Dimethyl-4-(1-oxido-3-pyridyl)-1,4-dihydro-pyridin-3-carbonsäuremethylester-5-carbonsäure-[2-(N-methyl-N-benzyl-amino)-äthyl]-ester.

16. Verfahren nach Anspruch 1 zur Herstellung von 6-Methoxymethyl-2-methyl-4-(1-oxido-3-pyridyl)-1,4-dihydro-pyridin-3-carbonsäureäthylester-5-carbonsäuremethylester.

17. Verfahren nach Anspruch 1 zur Herstellung von 2-Amino-6-methyl-4-(1-oxido-3-pyridyl)-1,4-dihydro-pyridin-3,5-pyridin-dicarbonsäure-diäthylester.

18. Verfahren nach Anspruch 1 zur Herstellung von 2,6-Dimethyl-4-(2-methylthio-1-oxido-3-pyridyl)-1,4-dihydro-pyridin-3,5-dicarbonsäure-diäthylester.

19. Verfahren nach Anspruch 1 zur Herstellung von 2,6-Dimethyl-4-(2-methyl-1-oxido-3-pyridyl)-1,4-dihydro-pyridin-3-carbonsäureisobutylester-5-carbonsäuremethylester.

20. Verfahren nach Anspruch 1 zur Herstellung von 6-Hydroxymethyl-2-methyl-4-(1-oxido-2-methyl-3-pyridyl)-1,4-dihydro-pyridin-3,5-dicarbonsäurediäthylester.

21. Verfahren nach Anspruch 1 zur Herstellung von 6-Cyan-2-methyl-4-(1-oxido-2-methyl-3-pyridyl)-1,4-dihydro-pyridin-3,5-dicarbonsäurediäthylester.

22. Verfahren nach Anspruch 1 zur Herstellung von 2,6-Dimethyl-4-(1-oxido-2-methoxy-3-pyridyl)-1,4-dihydro-pyridin-3,5-dicarbonsäure-diäthylester.

23. Verfahren nach Anspruch 1 zur Herstellung von 2,6-Dimethyl-4-(2-methylsulfinyl-1-oxido-3-pyridyl)-1,4-dihydro-pyridin-3,5-dicarbonsäurediäthylester.

24. Verfahren nach Anspruch 1 zur Herstellung von 2-Methyl-4-(1-oxido-3-pyridyl)-5-oxo-1,4,5,7-tetrahydro-furo[3,4,-b]pyridin-3-carbonsäureäthylester.

25. Verfahren nach Anspruch 1 zur Herstellung von 2-Methyl-4-(1-oxido-2-chlor-3-pyridyl)-5-oxo-1,4,5,7-tetrahydro-furo[3,4-b]pyridin-3-carbonsäureäthylester.

26. Verfahren nach Anspruch 1 zur Herstellung von 2-Methyl-4-(1-oxido-2-methyl-3-pyridyl)-5-oxo-1,4,5,7-tetrahydro-furo[3,4-b]pyridin-3-carbonsäureäthylester.

27. Verfahren nach Anspruch 1 zur Herstellung von 2-Methyl-4-(1-oxido-2-methylthio-3-pyridyl)-5-oxo-1,4,5,7-tetrahydro-furo[3,4-b]pyridin-3-carbonsäureäthylester.

28. Verfahren zur Herstellung von pharmazeutischen Präparaten, dadurch gekennzeichnet, dass man nach oben Ansprüchen 1—27 hergestellte Verbindungen, gegebenenfalls unter Beimischung von Hilfsstoffen zu pharmazeutischen Präparaten verarbeitet.

**Claims for the Contracting States: BE CH DE FR IT LI LU NL SE**

1. A compound of formula

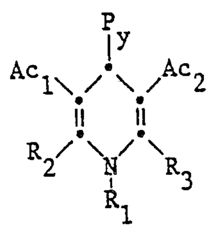

(I),

wherein Py is N-oxidopyridyl, unsubstituted or substituted by $C_1$—$C_7$alkyl, $C_1$—$C_7$alkoxy, $C_1$—$C_7$alkylthio, $C_1$—$C_7$alkylsulfinyl, $C_1$—$C_7$alkylsulfonyl and/or halogen, $R_1$ is hydrogen, $C_1$—$C_7$alkyl, $C_1$—$C_7$dialkylamino-$C_1$—$C_7$alkyl, $C_4$—$C_6$alkyleneamino-$C_1$—$C_7$alkyl, morpholino-$C_1$—$C_7$alkyl, thiomorpholino-$C_1$—$C_7$lower alkyl, piperazino-$C_1$—$C_7$alkyl or 4-$C_1$—$C_7$alkylpiperazino-$C_1$—$C_7$alkyl, one of the substituents $R_2$ and $R_3$ is $C_1$—$C_7$alkyl and the other is hydrogen, $C_1$—$C_7$alkyl, $C_1$—$C_7$hydroxyalkyl, $C_1$—$C_7$alkoxy-$C_1$—$C_7$alkyl, gem-$C_1$—$C_7$dialkoxy-$C_1$—$C_7$alkyl, $C_1$—$C_7$haloalkyl, $C_1$—$C_7$oxoalkyl, $C_1$—$C_7$alkoxycarbonyl-$C_1$—$C_7$alkyl, $C_1$—$C_7$carbamoylalkyl, N-$C_1$—$C_7$alkylcarbamoyl-$C_1$—$C_7$alkyl, N,N-$C_1$—$C_7$dialkylcarbamoyl-$C_1$—$C_7$alkyl, $C_1$—$C_7$cyanoalkyl, $C_1$—$C_7$aminoalkyl, $C_1$—$C_7$alkylamino-$C_1$—$C_7$alkyl, $C_1$—$C_7$dialkylamino-$C_1$—$C_4$alkyl, $C_4$—$C_6$alkyleneamino-$C_1$—$C_7$alkyl, morpholino-$C_1$—$C_7$alkyl, thiomorpholino-$C_1$—$C_7$alkyl, piperazino-$C_1$—$C_4$alkyl, 4-$C_1$—$C_4$alkylpiperazino-$C_1$—$C_7$alkyl, $C_1$—$C_7$alkoxyimino-$C_1$—$C_7$alkyl, wherein $C_1$—$C_7$alkoxy and imino substituents are attached to the same carbon atom, hydroxyimino-$C_1$—$C_7$alkyl, $C_1$—$C_7$alkoxy-imino-$C_1$—$C_7$alkyl, $C_1$—$C_7$alkoxycarbonyl, carbamoyl, N-$C_1$—$C_7$alkylcarbamoyl, N,N-$C_1$—$C_7$dialkyl-carbamoyl, cyano, amino, $C_1$—$C_7$alkylamino, $C_1$—$C_7$dialkylamino-$C_1$—$C_7$alkylamino, $C_4$—$C_6$alkyleneamino-$C_1$—$C_7$alkylamino, morpholino-$C_1$—$C_7$alkylamino, $C_1$—$C_7$dialkylamino, $C_4$—$C_6$alkyleneamino, (2-oxo-1-imidazolidinyl)-$C_4$—$C_6$alkyleneamino, morpholino, thiomorpholino, piperazino, 4-$C_1$—$C_7$alkylpiperazino, 4-$C_1$—$C_7$alkanoylpiperazino, 4-benzoylpiperazino, 4-furoylpiperazino, or 4-thienoylpiperazino, while an amino group $R_2$ or $R_3$ may also be attached to a lower alkyl radical $R_1$ and, together with said radical, can form a 1-aza-$C_3$—$C_4$alkylene radical whose nitrogen atom is attached to the 2- or 6-ring carbon atom of the 1,4-dihydropyridine ring, or, if $R_2$ or $R_3$ is $C_1$—$C_7$alkyl, said alkyl radical, together with the adjacent acyl radical $Ac_1$ or $Ac_2$, can form a 2-oxa-1-oxo-$C_2$—$C_3$alkylene radical whose carbonyl group is attached to the 4- or 5-ring carbon atom of the 1,4-dihydropyridine ring, and each of the radicals $Ac_1$ and $Ac_2$ independently of the other is $C_1$—$C_7$alkanoyl, benzoyl which is unsubstituted or substituted by $C_1$—$C_7$alkyl, $C_1$—$C_7$alkoxy and/or halogen, $C_1$—$C_7$alkylsulfonyl, phenylsulfonyl, unsubstituted or substituted by $C_1$—$C_7$alkyl, $C_1$—$C_7$-alkoxy and/or halogen, $C_1$—$C_7$alkoxycarbonyl, hydroxy-$C_1$—$C_7$alkoxycarbonyl, $C_1$—$C_7$alkoxy-$C_1$—$C_7$alkoxy-carbonyl, amino-$C_1$—$C_7$alkoxycarbonyl, $C_1$—$C_7$alkylamino-$C_1$—$C_7$alkoxycarbonyl, $C_1$—$C_7$dialkylamino-$C_1$—$C_7$alkoxycarbonyl, N-$C_1$—$C_7$alkyl-N-phenyl-$C_1$—$C_7$alkylamino-$C_1$—$C_7$alkoxycarbonyl, $C_4$—$C_6$alkylene-amino-$C_1$—$C_7$alkoxycarbonyl, morpholino-$C_1$—$C_7$alkoxycarbonyl, thiomorpholino-$C_1$—$C_7$alkoxycarbonyl, piperazino-$C_1$—$C_7$alkoxycarbonyl, 4-$C_1$—$C_7$alkylpiperazino-$C_1$—$C_7$alkoxycarbonyl, phenyl-$C_1$—$C_7$alkoxy-

carbonyl, unsubstituted or substituted by $C_1$—$C_7$alkyl, $C_1$—$C_7$alkoxy and/or halogen, or is carbamoyl, N-$C_1$—$C_7$alkylcarbamoyl, N,N-$C_1$—$C_7$dialkylcarbamoyl, N,N-$C_4$—$C_6$alkylenecarbamoyl, morpholinocarbonyl, thiomorpholinocarbonyl, piperazinocarbonyl or 4-$C_1$—$C_7$alkylpiperazinocarbonyl, or a salt thereof containing salt-forming groups.

2. A compound of formula I according to claim 1, wherein Py, $R_1$, $Ac_1$ and $Ac_2$ are as defined in claim 1, and one of the substituents $R_2$ and $R_3$ is $C_1$—$C_7$alkyl and the other is hydrogen, $C_1$—$C_7$alkyl, $C_1$—$C_7$hydroxyalkyl, $C_1$—$C_7$alkoxy-$C_1$—$C_7$alkyl, $C_1$—$C_7$haloalkyl, $C_1$—$C_7$alkoxycarbonyl-$C_1$—$C_7$alkyl, carbamoyl-$C_1$—$C_7$alkyl, N-$C_1$—$C_7$alkylcarbamoyl-$C_1$—$C_7$alkyl, N,N-$C_1$—$C_7$dialkylcarbamoyl-$C_1$—$C_7$alkyl, cyano-$C_1$—$C_7$alkyl, amino-$C_1$—$C_7$alkyl, $C_1$—$C_7$alkylamino-$C_1$—$C_7$alkyl, $C_1$—$C_7$dialkylamino-$C_1$—$C_7$alkyl, $C_1$—$C_7$alkyleneamino-$C_1$—$C_7$alkyl, morpholino-$C_1$—$C_7$alkyl, thiomorpholino-$C_1$—$C_7$alkyl, piperazino-$C_1$—$C_7$alkyl, 4-$C_1$—$C_7$alkylpiperazino-$C_1$—$C_7$alkyl, $C_1$—$C_7$alkoxycarbonyl, carbamoyl, N-$C_1$—$C_7$alkylcarbamoyl, N,N-$C_1$—$C_7$alkylcarbamoyl, cyano, amino, lower alkylamino, dialkylamino-$C_1$—$C_7$alkylamino, $C_4$—$C_6$alkyleneamino-$C_1$—$C_7$alkylamino, morpholino-$C_1$—$C_7$alkylamino, $C_1$—$C_7$dialkylamino, $C_4$—$C_6$alkyleneamino, (2-oxo-1-imidazolidinyl)-$C_4$—$C_6$alkyleneamino, morpholino, thiomorpholino, piperazino, 4-$C_1$—$C_7$alkylpiperazino, 4-$C_1$—$C_7$alkanoylpiperazino, 4-benzoylpiperazino, 4-furoylpiperazino or 4-thienoylpiperazino, while an amino group $R_2$ or $R_3$ may also be attached to a $C_1$—$C_7$alkyl radical $R_1$ and, together with said radical, can form a 1-aza-$C_3$—$C_4$alkylene radical whose nitrogen atom is attached to the 2- or 6-ring carbon atom of the 1,4-dihydropyridine ring, or, if $R_2$ or $R_3$ is $C_1$—$C_7$alkyl, said radical, together with the adjacent acyl radical $Ac_1$ or $Ac_2$, can form a 2-oxa-1-oxo-$C_2$—$C_3$alkylene radical whose carbonyl group is attached to the 3- or 5-ring carbon atom of the 1,4-dihydropyridine ring; or a salt thereof containing salt-forming groups.

3. A compound of formula I according to claim 1, wherein Py is N-oxidopyridyl, unsubstituted or substituted by $C_1$—$C_4$alkyl or $C_1$—$C_7$alkylsulfinyl, $R_1$ is hydrogen, $C_1$—$C_4$alkyl, 2-$C_1$—$C_7$dialkylamino-$C_1$—$C_7$alkyl, 2-$C_4$—$C_6$alkyleneamino-$C_1$—$C_7$alkyl or 2-(4-morpholino)-$C_1$—$C_7$alkyl, one of the substituents $R_2$ and $R_3$ is $C_1$—$C_4$alkyl and the other is hydrogen, $C_1$—$C_7$alkyl, $C_1$—$C_7$hydroxyalkyl, $C_1$—$C_7$haloalkyl, $C_1$—$C_7$alkoxycarbonyl-$C_1$—$C_7$alkyl, $C_1$—$C_7$cyanoalkyl, $C_1$—$C_7$dialkylamino-$C_1$—$C_7$alkyl, $C_1$—$C_7$alkoxycarbonyl, cyano, amino, (4-morpholino)-$C_1$—$C_7$alkylamino, $C_4$—$C_6$alkyleneamino, (2-oxo-1-imidazolidinyl)-piperazino or 4-(2-furoyl)-piperazino, while an amino group $R_2$ or $R_3$ may be attached to a $C_1$—$C_7$alkyl radical $R_1$ and, together with said radical, can form a 1-aza-lower alkylene radical whose aza nitrogen atom is attached to the 2- or 6-ring carbon atom of the 1,4-dihydropyridine ring, or, if $R_2$ or $R_3$ is $C_1$—$C_7$lower alkyl, said alkyl radical, together with the adjacent acyl radical $Ac_1$ or $Ac_2$, can form a 2-oxa-1-oxo-lower alkylene radical whose carbonyl group is attached to the 3- or 5-ring carbon atom of the 1,4-dihydropyridine ring, and each of the radicals $Ac_1$ and $Ac_2$, independently of the other, is lower alkanoyl, $C_1$—$C_7$alkylsulfonyl, $C_1$—$C_7$alkoxycarbonyl, hydroxy-$C_1$—$C_7$alkoxycarbonyl, $C_1$—$C_7$alkoxy-$C_1$—$C_7$alkoxycarbonyl, $C_1$—$C_7$dialkylamino-$C_1$—$C_7$alkoxycarbonyl, N-$C_1$—$C_7$alkyl-N-phenyl-$C_1$—$C_7$alkylamino-$C_1$—$C_7$alkoxycarbonyl, $C_4$—$C_6$-alkyleneamino-$C_1$—$C_7$alkoxycarbonyl, (4-morpholino)-$C_1$—$C_7$alkoxycarbonyl, carbamoyl, N-$C_1$—$C_7$alkylcarbamoyl, N,N-$C_1$—$C_7$dialkylcarbamoyl, N,N-$C_4$—$C_5$alkylenecarbamoyl or 4-morpholinocarbonyl, with lower alkyl, lower alkoxy and lower alkanoyl containing up to and including 4 carbon atoms, lower alkylene containing 4 or 5 chain carbon atoms, 1-aza-lower alkylene containing 2 or 3 chain carbon atoms, and 2-oxa-1-oxo-lower alkylene containing 2 chain carbon atoms, or a salt thereof containing salt-forming groups.

4. A compound of formula I according to claim 1, wherein Py is N-oxidopyridyl, $R_1$ is hydrogen, $C_1$—$C_4$alkyl, 2-($C_1$—$C_7$dialkylamino)-$C_1$—$C_4$alkyl, 2-$C_4$—$C_6$alkyleneamino)-$C_1$—$C_4$alkyl, or 2-(4-morpholino)-$C_1$—$C_4$alkyl, one of the substituents $R_2$ and $R_3$ is $C_1$—$C_4$alkyl, and the other is $C_1$—$C_4$alkyl, $C_1$—$C_7$hydroxyalkyl, $C_1$—$C_7$haloalkyl, 2-$C_1$—$C_7$dialkylamino)-$C_1$—$C_4$alkyl, $C_1$—$C_7$alkoxycarbonyl, cyano, amino, (4-morpholino)-$C_1$—$C_7$alkylamino, $C_4$—$C_6$alkyleneamino, (2-oxo-1-imidazolidinyl)-$C_4$—$C_6$alkyleneamino, or 4-(2-furoyl)piperazino, while an amino group $R_2$ or $R_3$ may be attached to a $C_1$—$C_4$alkyl radical $R_1$ and, together with said radical, can form a 1-aza-$C_2$—$C_3$alkylene radical whose aza nitrogen atom is attached to the 2- or 6-ring carbon atom of the 1,4-dihydropyridine ring, or, if $R_2$ or $R_3$ is $C_1$—$C_7$alkyl, said alkyl radical, together with the adjacent acyl radical $Ac_1$ or $Ac_2$, can from a 2-oxa-1-oxo-$C_2$alkylene radical whose carbonyl group is attached to the 3- or 5-ring carbon atom of the 1,4-dihydropyridine ring, and each of the radicals $Ac_1$ and $Ac_2$, independently of the other, is $C_1$—$C_4$alkanoyl, $C_1$—$C_7$alkylsulfonyl, $C_1$—$C_7$alkoxycarbonyl, 2-$C_1$—$C_4$alkoxy-$C_1$—$C_4$alkoxycarbonyl, $C_1$—$C_7$dialkylamino-$C_1$—$C_7$alkoxycarbonyl, or N-$C_1$—$C_4$alkyl-N-phenyl-$C_1$—$C_4$alkylamino-$C_1$—$C_7$alkoxycarbonyl, carbamoyl, N-$C_1$—$C_4$alkylcarbamoyl, N,N-$C_1$—$C_7$dialkylcarbamoyl, or 4-morpholinocarbonyl, with lower alkyl, lower alkoxy and lower alkanoyl containing up to and including 4 carbon atoms, lower alkylene containing 4 or 5 chain carbon atoms, 1-aza-lower alkylene containing 2 or 3 chain carbon atoms and 2-oxa-1-oxo-lower alkylene containing 2 chain carbon atoms, or a salt thereof containing salt-forming basic groups.

5. A compound of formula I according to claim 1, wherein Py is N-oxidopyridyl, $R_1$ is hydrogen or 2-(4-morpholino)ethyl, one of the substituents $R_2$ and $R_3$ is methyl and the other is methyl, hydroxymethyl, $C_1$—$C_4$alkoxycarbonyl, for example methoxycarbonyl, ethoxycarbonyl, n-propoxycarbonyl, isopropoxycarbonyl, n-butoxycarbonyl or isobutoxycarbonyl, cyano or amino, and each of the radicals $Ac_1$ and $Ac_2$, independently of the other, is lower alkanoyl, for example acetyl, $C_1$—$C_7$alkylsulfonyl, for example ethylsulfonyl, lower alkoxycarbonyl, for example methoxycarbonyl, ethoxycarbonyl, n-propoxycarbonyl, isopropoxycarbonyl, n-butoxycarbonyl or isobutoxycarbonyl, carbamoyl, N-lower alkylcarbamoyl, for example N-methylcarbamoyl, or N,N-di-lower alkylcarbamoyl, for example N,N-dimethylcarbamoyl, with

lower alkyl, lower alkoxy and lower alkanoyl containing up to and including 4 carbon atoms, or a salt thereof containing salt-forming basic groups.

6. 2,6 - Dimethyl - 4 - (1 - oxido - 3 - pyridyl) - 1,4 - dihydropyridine - 3,5 - dicarboxylic acid dimethyl ester.

7. 2,6 - Dimethyl - 4 - (1 - oxido - 3 - pyridyl) - 1,4 - dihydropyridine - 3,5 - dicarboxylic acid diethyl ester.

8. 2,6 - Dimethyl - 4 - (1 - oxido - 3 - pyridyl) - 1,4 - dihydropyridine - 3 - carboxylic acid isobutyl ester 5 - carboxylic acid methyl ester.

9. 2,6 - Dimethyl - 4 - (1 - oxido - 2 - pyridyl) - 1,4 - dihydropyridine - 3,5 - dicarboxylic acid diethyl ester.

10. 2,6 - Dimethyl - 4 - (1 - oxido - 4 - pyridyl) - 1,4 - dihydropyridine - 3,5 - dicarboxylic acid diethyl ester.

11. 2,6 - Dimethyl - 4 - (1 - oxido - 3 - pyridyl) - 1,4 - dihydropyridine - 3,5 - dicarboxylic acid di - n - propyl ester.

12. 2,6 - Dimethyl - 4 - (2 - methyl - 1 - oxido - 3 - pyridyl) - 1,4 - dihydropyridine - 3,5 - dicarboxylic acid diethyl ester.

13. 2,6 - Dimethyl - 4 - (2 - chloro - 1 - oxido - 3 - pyridyl) - 1,4 - dihydropyridine - 3,5 - dicarboxylic acid diethyl ester.

14. 2,6 - Dimethyl - 4 - (1 - oxido - 3 - pyridyl) - 1,4 - dihydropyridine - 3 - carboxylic acid isopropyl ester 5 - carboxylic acid (2 - methoxyethyl)ester.

15. 2,6 - Dimethyl - 4 - (1 - oxido - 3 - pyridyl) - 1,4 - dihydropyridine - 3 - carboxylic acid methyl ester 5 - carboxylic acid [2 - (N - methyl - N - benzylamino)ethyl]ester.

16. 6 - Methoxymethyl - 2 - methyl - 4 - (1 - oxido - 3 - pyridyl) - 1,4 - dihydropyridine - 3 - carboxylic acid ethyl ester 5 - carboxylic acid methyl ester.

17. 2 - Amino - 6 - methyl - 4 - (1 - oxido - 3 - pyridyl) - 1,4 - dihydropyridine - 3,5 - dicarboxylic acid diethyl ester.

18. 2,6 - Dimethyl - 4 - (2 - methylthio - 1 - oxido - 3 - pyridyl) - 1,4 - dihydropyridine - 3,5 - dicarboxylic acid diethyl ester.

19. 2,6 - Dimethyl - 4 - (2 - methyl - 1 - oxido - 3 - pyridyl) - 1,4 - dihydropyridine - 3 - carboxylic acid isobutyl ester 5 - carboxylic acid methyl ester.

20. 6 - Hydroxymethyl - 2 - methyl - 4 - (1 - oxido - 2 - methyl - 3 - pyridyl) - 1,4 - dihydropyridine - 3,5 - dicarboxylic acid diethyl ester.

21. 6 - Cyano - 2 - methyl - 4 - (1 - oxido - 2 - methyl - 3 - pyridyl) - 1,4 - dihydropyridine - 3,5 - dicarboxylic acid diethyl ester.

22. 2,6 - Dimethyl - 4 - (1 - oxido - 2 - methoxy - 3 - pyridyl) - 1,4 - dihydropyridine - 3,5 - dicarboxylic acid diethyl ester.

23. 2,6 - Dimethyl - 4 - (2 - methylsulfinyl - 1 - oxido - 3 - pyridyl) - 1,4 - dihydropyridine - 3,5 - dicarboxylic acid diethyl ester.

24. 2 - Methyl - 4 - (1 - oxido - 3 - pyridyl) - 5 - oxo - 1,4,5,7 - tetrahydrofuro[3,4 - b]pyridine - 3 - carboxylic acid ethyl ester.

25. 2 - Methyl - 4 - (1 - oxido - 2 - chloro - 3 - pyridyl) - 5 - oxo - 1,4,5,7 - tetrahydrofuro[3,4 - b]pyridine - 3 - carboxylic acid ethyl ester.

26. 2 - Methyl - 4 - (1 - oxido - 2 - methyl - 3 - pyridyl) - 5 - oxo - 1,4,5,7 - tetrahydrofuro[3,4 - b]pyridine - 3 - carboxylic acid ethyl ester.

27. 2 - Methyl - 4 - (1 - oxido - 2 - methylthio - 3 - pyridyl) - 5 - oxo - 1,4,5,7 - tetrahydrofuro[3,4 - b]pyridine - 3 - carboxylic acid ethyl ester.

28. A compound according to claim 1 or 16 as pharmacologically active compound, especially in the cardiovascular field.

29. A compound according to any one of claims 2, 6 to 15 and 17 as pharmacologically active compound, especially in the cardiovascular field.

30. A compound according to any one of claims 1, 3 to 5 and 17 to 27 as pharmacologically active compound, especially in the cardiovascular field.

31. A compound according to claim 1, with the exception of one in which $R_2$ or $R_3$ is a $C_1$—$C_7$alkyl radical which, together with the adjacent acyl radical $Ac_1$ or $Ac_2$, forms a 2-oxa-1-oxo-$C_2$—$C_3$alkylene radical whose carbonyl group is attached to the 3- or 5-ring carbon atom of the 1,4-dihydropyridine ring, and of claim 18, as pharmacologically active, especially antihypertensive, compound.

32. A compound according to claim 2, with the exception of one in which $R_2$ or $R_3$ is a $C_1$—$C_7$alkyl radical which, together with the adjacent acyl radical $Ac_1$ or $Ac_2$, forms a 2-oxa-1-oxo-$C_2$—$C_3$alkylene radical whose carbonyl group is attached to the 3- or 5-ring carbon atom of the 1,4-dihydropyridine ring, and of any one of claims 6 to 15 and 17 as pharmacologically active, especially antihypertensive, compound.

33. A compound according to any one of claims 1 and 3 to 5, with the exception of one in which $R_2$ and $R_3$ is a $C_1$—$C_7$alkyl or methyl radical which, together with the adjacent acyl radical $Ac_1$ or $Ac_2$, forms a 2-oxa-1-oxo-1,3-propylene radical whose carbonyl group is in each case attached to the 3- or 5-ring carbon atom of the 1,4-dihydropyridine ring, and of any one of claims 18 to 23 as pharmacologically active, especially antihypertensive, compound.

34. A compound according to claim 1, wherein $R_2$ or $R_3$ is a $C_1$—$C_7$alkyl radical which, together with the adjacent acyl radical $Ac_1$ or $Ac_2$, forms a 2-oxa-1-oxo-$C_2$—$C_3$alkylene radical whose carbonyl group is attached to the 3- or 5-ring carbon atom of the 1,4-dihydropyridine ring, or of claim 16, as pharmacologically active compound, especially a pharmacologically active compound that increases myocardial contractility.

35. A compound according to claim 2, wherein $R_2$ or $R_3$ is a $C_1$—$C_7$alkyl radical which, together with the adjacent acyl radical $Ac_1$ or $Ac_2$, forms a 2-oxa-1-oxo-$C_2$—$C_3$alkylene radical whose carbonyl group is attached to the 3- or 5-ring carbon atom of the 1,4-dihydropyridine ring, or of any one of claims 6 to 15 and 17 as pharmacologically active compound, especially a pharmacologically active compound that increases myocardial contractility.

36. A compound according to any one of claims 1 and 3 to 5, wherein $R_2$ or $R_3$ is a $C_1$—$C_7$alkyl radical which, together with the adjacent acyl radical $Ac_1$ or $Ac_2$, forms a 2-oxa-1-oxo-$C_2$—$C_3$alkylene radical whose carbonyl group is attached to the 3- or 5-ring carbon atom of the 1,4-dihydropyridine ring, or of any one of claims 26 to 27 as pharmacologically active compound, especially a pharmacologically active compound that increases myocardial contractility.

37. A compound according to claim 16 for the treatment of the human or animal body.

38. A compound according to any one of claims 2, 6 to 15 and 17 for the treatment of the human or animal body.

39. A compound according to any one of claims 1, 3 to 7 and 18 to 27, for the treatment of the human or animal body.

40. Use of a compound as claimed in claim 16 or of a pharmaceutically acceptable salt thereof for the preparation of a pharmaceutical composition for the preventive and/or therapeutic treatment of hypertension on the one hand and of cardiovascular diseases on the other.

41. Use of a compound as claimed in any one of claims 2, 6 to 15 and 17, or of a pharmaceutically acceptable salt thereof, for the preparation of a pharmaceutical composition for the preventive and/or therapeutic treatment of hypertension on the one hand and of cardiovascular diseases on the other.

42. Use of a compound as claimed in any one of claims 1, 3 to 5 and 18 to 23, or of a pharmaceutically acceptable salt thereof, for the preparation of a pharmaceutical composition for the preventive and/or therapeutic treatment of hypertension on the one hand and of cardiovascular diseases on the other.

43. A pharmaceutical composition containing the compound of claim 16.

44. A pharmaceutical composition containing a compound as claimed in any one of claims 1, 3 to 5 and 17.

45. A pharmaceutical composition containing a compound as claimed in any one of claims 1, 3 to 5 and 18 to 27.

46. Use of the compound of claim 16 for the preparation of a pharmaceutical composition.

47. Use of a compound as claimed in any one of claims 2, 6 to 15 and 17 for the preparation of a pharmaceutical composition.

48. Use of a compound as claimed in any one of claims 1, 3 to 5 and 18 to 27 for the preparation of a pharmaceutical composition.

49. A process for the preparation of a pharmaceutical composition, which comprises processing the compound as claimed in claim 16, with the optional addition of excipients, to such a composition.

50. A process for the preparation of a pharmaceutical composition, which comprises processing a compound as claimed in any one of claims 2, 6 to 15 and 17, with the optional addition of excipients, to such a composition.

51. A process for the preparation of a pharmaceutical composition, which comprises processing a compound as claimed in any one of claims 1, 3 to 5 and 18 to 27, with the optional addition of excipients, to such a composition.

52. A process for the preparation of a compound of formula I as defined in claims 1 and 16, which comprises

a) cyclising a compound of formula

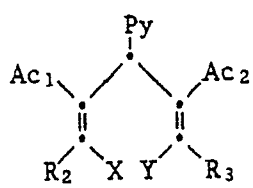

(II)

wherein one of the substituents X and Y is a group of formula —NH—$R_1$ and the other is hydroxy or a group of formula —NH—$R_1$, or a tautomer thereof or a corresponding tautomeric mixture, or

b) reacting a compound of formula Py—CHO (III) or a reactive functional derivative thereof with a compound of formula

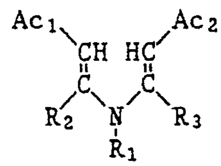

(IV)

or a tautomer thereof or a corresponding tautomeric mixture, or

c) in a compound of formula

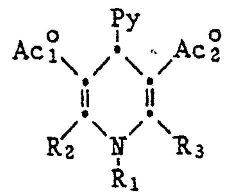

(V)

wherein one of the substituents $Ac_1$ and $Ac_2$ is a radical that can be converted into the radical $Ac_1$ or $Ac_2$ and the other is $Ac_1$ or $Ac_2$ or a radical that can be converted into an $Ac_1$ or $Ac_2$, so converting the radical $Ac_1$ and/or $Ac_2$, or

d) in a compound of formula

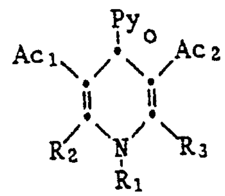

(VI)

wherein $Py_o$ is an unsubstituted or substituted pyridyl radical, oxidising the radical $Py_o$ to the corresponding N-oxidopyridyl radical, while compounds of formula I, wherein $R_2$ or $R_3$ as $C_1$—$C_7$alkyl, together with the adjacent acyl radical $Ac_1$ or $Ac_2$, are a 2-oxa-1-oxo-$C_2$—$C_3$ alkylene radical whose carbonyl group is attached to the 3- or 5-ring carbon atom of the 1,4-dihydropyridine ring, can be formed direct under the reaction conditions of one of processes a) to d) from starting materials of formulae II, IV, V, and VI, in which $R_2$ or $R_3$ is lower alkyl containing free or esterified hydroxy, and $Ac_1$ or $Ac_2$ or $Ac_1^o$ or $Ac_2^o$ in starting materials of the formula V is an esterified carboxyl group, and, in the above starting materials of formulae II to VI, which if they have salt-forming properties, can also be used in the form of their salts, the substituents Py, $R_1$, $R_2$, $R_3$, $Ac_1$ and $Ac_2$ are as defined for formula I, and, if desired, a resultant compound of formula I is converted into a different compound of formula I, and/or, if desired, a resultant is converted into the free compound or into a different salt, and/or, if desired, a resultant free compound of formula I containing salt-forming groups is converted into a salt, and/or, if desired, a resultant mixture of isomers is separated into the individual isomers.

53. A process for the preparation of a compound of formula I as defined in any one of claims 2, 6 to 15 and 17, wherein the processes defined in claim 52 are carried out with corresponding starting materials.

54. A process for the preparation of a compound of formula I as defined in claims 3 to 5 and 18 to 27, wherein the processes defined in claim 52 are carried out with corresponding starting materials.

**Claims for the Contracting State: AT**

1. A process for the preparation of a compound of formula

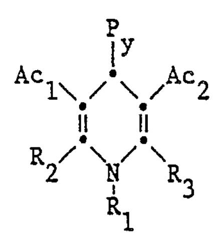

(I),

wherein Py is N-oxidopyridyl, unsubstituted or substituted by $C_1$—$C_7$alkyl, $C_1$—$C_7$alkoxy, $C_1$—$C_7$alkylthio,

$C_1$—$C_7$alkylsulfinyl, $C_1$—$C_7$alkylsulfonyl and/or halogen, $R_1$ is hydrogen, $C_1$—$C_7$alkyl, $C_1$—$C_7$dialkylamino-$C_1$—$C_7$alkyl, $C_4$—$C_6$alkyleneamino-$C_1$—$C_7$alkyl, morpholino-$C_1$—$C_7$alkyl, thiomorpholino-$C_1$—$C_7$lower alkyl, piperazino-$C_1$—$C_7$alkyl or 4-$C_1$—$C_7$alkylpiperazino-$C_1$—$C_7$alkyl, one of the substituents $R_2$ and $R_3$ is $C_1$—$C_7$alkyl and the other is hydrogen, $C_1$—$C_7$alkyl, $C_1$—$C_7$hydroxyalkyl, $C_1$—$C_7$alkoxy-$C_1$—$C_7$alkyl, gem-$C_1$—$C_7$dialkoxy-$C_1$—$C_7$alkyl, $C_1$—$C_7$haloalkyl, $C_1$—$C_7$oxoalkyl, $C_1$—$C_7$alkoxycarbonyl-$C_1$—$C_7$alkyl, $C_1$—$C_7$carbamoylalkyl, N-$C_1$—$C_7$alkylcarbamoyl-$C_1$—$C_7$alkyl, N,N-$C_1$—$C_7$dialkylcarbamoyl-$C_1$—$C_7$alkyl, $C_1$—$C_7$cyanoalkyl, $C_1$—$C_7$aminoalkyl, $C_1$—$C_7$alkylamino-$C_1$—$C_7$alkyl, $C_1$—$C_7$dialkylamino-$C_1$—$C_4$alkyl, $C_4$—$C_6$alkyleneamino-$C_1$—$C_7$alkyl, morpholino-$C_1$—$C_7$alkyl, thiomorpholino-$C_1$—$C_7$alkyl, piperazino-$C_1$—$C_4$alkyl, 4-$C_1$—$C_4$alkylpiperazino-$C_1$—$C_7$alkyl, $C_1$—$C_7$alkoxyimino-$C_1$—$C_7$alkyl, wherein $C_1$—$C_7$alkoxy and imino substituents are attached to the same carbon atom, hydroxyimino-$C_1$—$C_7$alkyl, $C_1$—$C_7$alkoxy-imino-$C_1$—$C_7$alkyl, $C_1$—$C_7$alkoxycarbonyl, carbamoyl, N-$C_1$—$C_7$alkylcarbamoyl, N,N-$C_1$—$C_7$dialkyl-carbamoyl, cyano, amino, $C_1$—$C_7$alkylamino, $C_1$—$C_7$dialkylamino-$C_1$—$C_7$alkylamino, $C_4$—$C_6$alkyleneamino-$C_1$—$C_7$alkylamino, morpholino-$C_1$—$C_7$alkylamino, $C_1$—$C_7$dialkylamino, $C_4$—$C_6$alkyleneamino, (2-oxo-1-imidazolidinyl)-$C_4$—$C_6$alkyleneamino, morpholino, thiomorpholino, piperazino, 4-$C_1$—$C_7$alkylpiperazino, 4-$C_1$—$C_7$alkanoylpiperazino, 4-benzoylpiperazino, 4-furoylpiperazino, or 4-thienoylpiperazino, while an amino group $R_2$ or $R_3$ may also be attached to a lower alkyl radical $R_1$ and, together with said radical, can form a 1-aza-$C_3$—$C_4$alkylene radical whose nitrogen atom is attached to the 2- or 6-ring carbon atom of the 1,4-dihydropyridine ring, or, if $R_2$ or $R_3$ is $C_1$—$C_7$alkyl, said alkyl radical, together with the adjacent acyl radical $Ac_1$ or $Ac_2$, can form a 2-oxa-1-oxo-$C_2$—$C_3$alkylene radical whose carbonyl group is attached to the 4- or 5-ring carbon atom of the 1,4-dihydropyridine ring, and each of the radicals $Ac_1$ and $Ac_2$ independently of the other is $C_1$—$C_7$alkanoyl, benzoyl which is unsubstituted or substituted by $C_1$—$C_7$alkyl, $C_1$—$C_7$alkoxy and/or halogen, $C_1$—$C_7$alkylsulfonyl, phenylsulfonyl, unsubstituted or substituted by $C_1$—$C_7$alkyl, $C_1$—$C_7$alkoxy and/or halogen, $C_1$—$C_7$alkoxycarbonyl, hydroxy-$C_1$—$C_7$alkoxycarbonyl, $C_1$—$C_7$alkoxy-$C_1$—$C_7$alkoxycarbonyl, amino-$C_1$—$C_7$alkoxycarbonyl, $C_1$—$C_7$alkylamino-$C_1$—$C_7$alkoxycarbonyl, $C_1$—$C_7$dialkylamino-$C_1$—$C_7$alkoxycarbonyl, N-$C_1$—$C_7$alkyl-N-phenyl-$C_1$—$C_7$alkylamino-$C_1$—$C_7$alkoxy-carbonyl, $C_4$—$C_6$alkyleneamino-$C_1$—$C_7$alkoxycarbonyl, morpholino-$C_1$—$C_7$alkoxycarbonyl, thio-morpholino-$C_1$—$C_7$alkoxycarbonyl, piperazino-$C_1$—$C_7$alkoxycarbonyl, 4-$C_1$—$C_7$alkylpiperazino-$C_1$—$C_7$alkoxycarbonyl, phenyl-$C_1$—$C_7$alkoxycarbonyl, unsubstituted or substituted by $C_1$—$C_7$alkyl, $C_1$—$C_7$alkoxy and/or halogen, or is carbamoyl, N-$C_1$—$C_7$alkylcarbamoyl, N,N-$C_1$—$C_7$dialkylcarbamoyl, N,N-$C_4$—$C_6$alkylenecarbamoyl, morpholinocarbonyl, thiomorpholinocarbonyl, piperazinocarbonyl or 4-$C_1$—$C_7$alkylpiperazinocarbonyl, or a salt thereof containing salt-forming groups, which comprises

a) cyclising a compound of formula

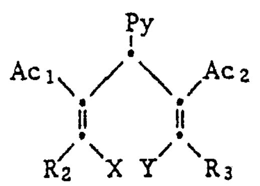

(II)

wherein one of the substituents X and Y is a group of formula —NH—$R_1$ and the other is hydroxy or a group of formula —NH—$R_1$, or a tautomer thereof or a corresponding tautomeric mixture, or

b) reacting a compound of formula Py—CHO (III) or a reactive functional derivative thereof with a compound of formula

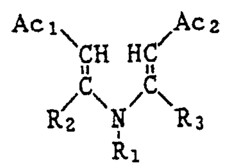

(IV)

or a tautomer thereof or a corresponding tautomeric mixture, or

c) in a compound of formula

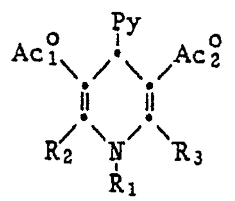

(V)

wherein one of the substituents $Ac_1$ and $Ac_2$ is a radical that can be converted into the radical $Ac_1$ or $Ac_2$ and the other is $Ac_1$ or $Ac_2$ or a radical that can be converted into an $Ac_1$ or $Ac_2$, so converting the radical $Ac_1$ and/or $Ac_2$, or

d) in a compound of formula

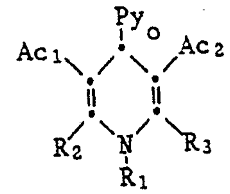

(VI)

wherein $Py_o$ is an unsubstituted or substituted pyridyl radical, oxidising the radical $Py_o$ to the corresponding N-oxidopyridyl radical, while compounds of formula I, wherein $R_2$ or $R_3$ as $C_1$—$C_7$alkyl, together with the adjacent acyl radical $Ac_1$ or $Ac_2$, are a 2-oxa-1-oxo-$C_2$—$C_3$ alkylene radical whose carbonyl group is attached to the 3- or 5-ring carbon atom of the 1,4-dihydropyridine ring, can be formed direct under the reaction conditions of one of processes a) to d) from starting materials of formulae II, IV, V, and VI, in which $R_2$ or $R_3$ is lower alkyl containing free or esterified hydroxy, and $Ac_1$ or $Ac_2$ or $Ac_1^o$ or $Ac_2^o$ in starting materials of the formula V is an esterified carboxyl group, and, in the above starting materials of formulae II to VI, which if they have salt-forming properties, can also be used in the form of their salts, the substituents Py, $R_1$, $R_2$, $R_3$, $Ac_1$ and $Ac_2$ are as defined for formula I, and, if desired, a resultant compound of formula I is converted into a different compound of formula I, and/or, if desired, a resultant salt is converted into the free compound or into a different salt, and/or, if desired, a resultant free compound of formula I containing salt-forming groups is converted into a salt, and/or, if desired, a resultant mixture of isomers is separated into the individual isomers.

2. A process according to claim 1 for the preparation of a compound of formula I, wherein Py, $R_1$, $Ac_1$ and $Ac_2$ are as defined in claim 1, and one of the substituents $R_2$ and $R_3$ is $C_1$—$C_7$alkyl and the other is hydrogen, $C_1$—$C_7$alkyl, $C_1$—$C_7$hydroxyalkyl, $C_1$—$C_7$alkoxy-$C_1$—$C_7$alkyl, $C_1$—$C_7$haloalkyl, $C_1$—$C_7$alkoxycarbonyl-$C_1$—$C_7$alkyl, carbamoyl-$C_1$—$C_7$alkyl, N-$C_1$—$C_7$alkylcarbamoyl-$C_1$—$C_7$alkyl, N,N-$C_1$—$C_7$dialkylcarbamoyl-$C_1$—$C_7$alkyl, cyano-$C_1$—$C_7$alkyl, amino-$C_1$—$C_7$alkyl, $C_1$—$C_7$alkylamino-$C_1$—$C_7$alkyl, $C_1$—$C_7$dialkylamino-$C_1$—$C_7$alkyl, $C_1$—$C_7$alkyleneamino-$C_1$—$C_7$alkyl, morpholino-$C_1$—$C_7$alkyl, thiomorpholino-$C_1$—$C_7$alkyl, piperazino-$C_1$—$C_7$alkyl, 4-$C_1$—$C_7$alkylpiperazino-$C_1$—$C_7$alkyl, $C_1$—$C_7$alkoxycarbonyl, carbamoyl, N-$C_1$—$C_7$alkylcarbamoyl, N,N-$C_1$—$C_7$alkylcarbamoyl, cyano, amino, lower alkylamino, dialkylamino-$C_1$—$C_7$alkylamino, $C_4$—$C_6$alkyleneamino-$C_1$—$C_7$alkylamino, morpholino-$C_1$—$C_7$alkylamino, $C_1$—$C_7$dialkylamino, $C_4$—$C_6$alkyleneamino, (2-oxo-1-imidazolidinyl)-$C_4$—$C_6$alkyleneamino, morpholino, thiomorpholino, piperazino, 4-$C_1$—$C_7$alkylpiperazino, 4-$C_1$—$C_7$alkanoylpiperazino, 4-benzoylpiperazino, 4-furoylpiperazino or 4-thienoylpiperazino, while an amino group $R_2$ or $R_3$ may also be attached to a $C_1$—$C_7$alkyl radical $R_1$ and, together with said radical, can form a 1-aza-$C_3$—$C_4$alkylene radical whose nitrogen atom is attached to the 2- or 6-ring carbon atom of the 1,4-dihydropyridine ring, or, if $R_2$ or $R_3$ is $C_1$—$C_7$alkyl, said radical, together with the adjacent acyl radical $Ac_1$ or $Ac_2$, can form a 2-oxa-1-oxo-$C_2$—$C_3$alkylene radical whose carbonyl group is attached to the 3- or 5-ring carbon atom of the 1,4-dihydropyridine ring, or a salt thereof containing salt-forming groups.

3. A process according to claim 1 for the preparation of a compound of formula I, wherein Py is N-oxidopyridyl, unsubstituted or substituted by $C_1$—$C_4$alkyl or $C_1$—$C_7$alkylsulfinyl, $R_1$ is hydrogen, $C_1$—$C_4$alkyl, 2-$C_1$—$C_7$dialkylamino-$C_1$—$C_7$alkyl, 2-$C_4$—$C_6$alkyleneamino-$C_1$—$C_7$alkyl or 2-(4-morpholino)-$C_1$—$C_7$alkyl, one of the substituents $R_2$ and $R_3$ is $C_1$—$C_4$alkyl and the other is hydrogen, $C_1$—$C_7$alkyl, $C_1$—$C_7$hydroxyalkyl, $C_1$—$C_7$haloalkyl, $C_1$—$C_7$alkoxycarbonyl-$C_1$—$C_7$alkyl, $C_1$—$C_7$cyanoalkyl, $C_1$—$C_7$dialkylamino-$C_1$—$C_7$alkyl, $C_1$—$C_7$alkoxycarbonyl, cyano, amino, (4-morpholino)-$C_1$—$C_7$alkylamino, $C_4$—$C_6$alkyleneamino, (2-oxo-1-imidazolidinyl)piperazino or 4-(2-furoyl)-piperazino, while an amino group $R_2$ or $R_3$ may be attached to a $C_1$—$C_7$alkyl radical $R_1$ and, together with said radical, can form a 1-aza-lower alkylene radical whose aza nitrogen atom is attached to the 2- or 6-ring carbon atom of the 1,4-dihydropyridine ring, or, if $R_2$ or $R_3$ is $C_1$—$C_7$lower alkyl, said alkyl radical, together with the adjacent acyl radical $Ac_1$ or $Ac_2$, can form a 2-oxa-1-oxo-lower alkylene radical whose carbonyl group is attached to the 3- or 5-ring carbon atom of the 1,4-dihydropyridine ring, and each of the radicals $Ac_1$ and $Ac_2$, independently of the other, is lower alkanoyl, $C_1$—$C_7$alkylsulfonyl, $C_1$—$C_7$alkoxycarbonyl, hydroxy-$C_1$—$C_7$alkoxycarbonyl, $C_1$—$C_7$alkoxy-$C_1$—$C_7$alkoxycarbonyl, $C_1$—$C_7$dialkylamino-$C_1$—$C_7$alkoxycarbonyl, N-$C_1$—$C_7$alkyl-N-phenyl-$C_1$—$C_7$alkylamino-$C_1$—$C_7$alkoxycarbonyl, $C_4$—$C_6$alkyleneamino-$C_1$—$C_7$alkoxycarbonyl, (4-morpholino)-$C_1$—$C_7$alkoxycarbonyl, carbamoyl, N-$C_1$—$C_7$alkylcarbamoyl, N,N-$C_1$—$C_7$dialkylcarbamoyl, N,N-$C_4$—$C_5$alkylenecarbamoyl or 4-morpholinocarbonyl, with lower alkyl, lower alkoxy and lower alkanoyl containing up to and including 4 carbon atoms, lower alkylene containing 4 or 5 chain carbon atoms, 1-aza-lower alkylene containing 2 or 3 chain carbon atoms, and 2-oxa-1-oxo-lower alkylene containing 2 chain carbon atoms, or a salt containing salt-forming groups.

4. A process according to claim 1 for the preparation of a compound of formula I, wherein Py is N-oxidopyridyl, $R_1$ is hydrogen, $C_1$—$C_4$alkyl, 2-($C_1$—$C_7$dialkylamino)-$C_1$—$C_4$alkyl, 2-$C_4$—$C_6$alkyleneamino)-$C_1$—$C_4$alkyl, or 2-(4-morpholino)-$C_1$—$C_4$alkyl, one of the substituents $R_2$ and $R_3$ is $C_1$—$C_4$alkyl, and the other is $C_1$—$C_4$alkyl, $C_1$—$C_7$hydroxyalkyl, $C_1$—$C_7$haloalkyl, 2-$C_1$—$C_7$dialkylamino)-$C_1$—$C_7$alkyl, $C_1$—$C_7$alkoxycarbonyl, cyano, amino, (4-morpholino)-$C_1$—$C_7$alkylamino, $C_4$—$C_6$alkyleneamino, (2-oxo-1-imidazolidinyl)-

$C_4$—$C_6$alkyleneamino, or 4-(2-furoyl)piperazino, while an amino group $R_2$ or $R_3$ may be attached to a $C_1$—$C_4$alkyl radical $R_1$ and, together with said radical, can form a 1-aza-$C_2$—$C_3$alkylene radical whose aza nitrogen atom is attached to the 2- or 6-ring carbon atom of the 1,4-dihydropyridine ring, or, if $R_2$ or $R_3$ is $C_1$—$C_7$alkyl, said alkyl radical, together with the adjacent acyl radical $Ac_1$ or $Ac_2$, can from a 2-oxa-1-oxo-$C_2$alkylene radical whose carbonyl group is attached to the 3- or 5-ring carbon atom of the 1,4-dihydropyridine ring, and each of the radicals $Ac_1$ and $Ac_2$, independently of the other, is $C_1$—$C_4$alkanoyl, $C_1$—$C_7$alkylsulfonyl, $C_1$—$C_7$alkoxycarbonyl, 2-$C_1$—$C_4$alkoxy-$C_1$—$C_4$alkoxycarbonyl, $C_1$—$C_7$dialkylamino-$C_1$—$C_7$alkoxycarbonyl, or N-$C_1$—$C_4$alkyl-N-phenyl-$C_1$—$C_4$alkylamino-$C_1$—$C_7$alkoxycarbonyl, carbamoyl, N-$C_1$—$C_4$alkylcarbamoyl, N,N-$C_1$—$C_7$dialkylcarbamoyl, or 4-morpholinocarbonyl, with lower alkyl, lower alkoxy and lower alkanoyl containing up to and including 4 carbon atoms, lower alkylene containing 4 or 5 chain carbon atoms, 1-aza-lower alkylene containing 2 or 3 chain carbon atoms and 2-oxa-1-oxo-lower alkylene containing 2 chain carbon atoms, or a salt thereof containing salt-forming basic groups.

5. A process according to claim 1 for the preparation of a compound of formula I, wherein Py is N-oxidopyridyl, $R_1$ is hydrogen or 2-(4-morpholino)ethyl, one of the substituents $R_2$ and $R_3$ is methyl and the other is methyl, hydroxymethyl, $C_1$—$C_4$alkoxycarbonyl, for example methoxycarbonyl, ethoxycarbonyl, n-propoxycarbonyl, isopropoxycarbonyl, n-butoxycarbonyl or isobutoxycarbonyl, cyano or amino, and each of the radicals $Ac_1$ and $Ac_2$, independently of the other, is lower alkanoyl, for example acetyl, $C_1$—$C_7$alkylsulfonyl, for example ethylsulfonyl, lower alkoxycarbonyl, for example methoxycarbonyl, ethoxycarbonyl, n-propoxycarbonyl, isopropoxycarbonyl, n-butoxycarbonyl or isobutoxycarbonyl, carbamoyl, N-lower alkylcarbamoyl, for example N-methylcarbamoyl, or N,N-di-lower alkylcarbamoyl, for example N,N-dimethylcarbamoyl, with lower alkyl, lower alkoxy and lower alkanoyl containing up to and including 4 carbon atoms, or a salt thereof containing salt-forming basic groups.

6. A process according to claim 1 for the preparation of 2,6 - dimethyl - 4 - (1 - oxido - 3 - pyridyl) - 1,4 - dihydropyridine - 3,5 - dicarboxylic acid dimethyl ester.

7. A process according to claim 1 for the preparation of 2,6 - dimethyl - 4 - (1 - oxido - 3 - pyridyl) - 1,4 - dihydropyridine - 3,5 - dicarboxylic acid diethyl ester.

8. A process according to claim 1 for the preparation of 2,6 - dimethyl - 4 - (1 - oxido - 3 - pyridyl) - 1,4 - dihydropyridine - 3 - carboxylic acid isobutyl ester 5-carboxylic acid methyl ester.

9. A process according to claim 1 for the preparation of 2,6 - dimethyl - 4 - (1 - oxido - 2 - pyridyl) - 1,4 - dihydropyridine - 3,5 - dicarboxylic acid diethyl ester.

10. A process according to claim 1 for the preparation of 2,6 - dimethyl - 4 - (1 - oxido - 4 - pyridyl) - 1,4 - dihydropyridine - 3,5 - dicarboxylic acid diethyl ester.

11. A process according to claim 1 for the preparation of 2,6 - dimethyl - 4 - (1 - oxido - 3 - pyridyl) - 1,4 - dihydropyridine - 3,5 - dicarboxylic acid di - n - propyl ester.

12. A process according to claim 1 for the preparation of 2,6 - dimethyl - 4 - (2 - methyl - 1 - oxido - 3 - pyridyl) - 1,4 - dihydropyridine - 3,5 - dicarboxylic acid diethyl ester.

13. A process according to claim 1 for the preparation of 2,6 - dimethyl - 4 - (2 - chloro - 1 - oxido - 3 - pyridyl) - 1,4 - dihydropyridine - 3,5 - dicarboxylic acid diethyl ester.

14. A process according to claim 1 for the preparation of 2,6 - dimethyl - 4 - (1 - oxido - 3 - pyridyl) - 1,4 - dihydopyridine - 3 - carboxylic acid isopropyl ester 5-carboxylic acid (2-methoxyethyl)ester.

15. A process according to claim 1 for the preparation of 2,6 - dimethyl - 4 - (1 - oxido - 3 - pyridyl) - 1,4 - dihydropyridine - 3 - carboxylic acid methyl ester 5 - carboxylic acid [2 - (N - methyl - N - benzylamino)ethyl]ester.

16. A process according to claim 1 for the preparation of 6 - methoxymethyl - 2 - methyl - 4 - (1 - oxido - 3 - pyridyl) - 1,4 - dihydropyridine - 3 - carboxylic acid ethyl ester 5 - carboxylic acid methyl ester.

17. A process according to claim 1 for the preparation of 2 - amino - 6 - methyl - 4 - (1 - oxido - 3 - pyridyl) - 1,4 - dihydropyridine - 3,5 - dicarboxylic acid diethyl ester.

18. A process according to claim 1 for the preparation of 2,6 - dimethyl - 4 - (2 - methylthio - 1 - oxido - 3 - pyridyl) - 1,4 - dihydropyridine - 3,5 - dicarboxylic acid diethyl ester.

19. A process according to claim 1 for the preparation of 2,6 - dimethyl - 4 - (2 - methyl - 1 - oxido - 3 - pyridyl) - 1,4 - dihydropyridine - 3 - carboxylic acid isobutyl ester 5 - carboxylic acid methyl ester.

20. A process according to claim 1 for the preparation of 6 - hydroxymethyl - 2 - methyl - 4 - (1 - oxido - 2 - methyl - 3 - pyridyl) - 1,4 - dihydropyridine - 3,5 - dicarboxylic acid diethyl ester.

21. A process according to claim 1 for the preparation of 6 - cyano - 2 - methyl - 4 - (1 - oxido - 2 - methyl - 3 - pyridyl) - 1,4 - dihydropyridine - 3,5 - dicarboxylic acid diethyl ester.

22. A process according to claim 1 for the preparation of 2,6 - dimethyl - 4 - (1 - oxido - 2 - methoxy - 3 - pyridyl) - 1,4 - dihydropyridine - 3,5 - dicarboxylic acid diethyl ester.

23. A process according to claim 1 for the preparation of 2,6 - dimethyl - 4 - (2 - methylsulfinyl - 1 - oxido - 3 - pyridyl) - 1,4 - dihydropyridine - 3,5 - dicarboxylic acid diethyl ester.

24. A process according to claim 1 for the preparation of 2 - methyl - 4 - (1 - oxido - 3 - pyridyl) - 5 - oxo - 1,4,5,7 - tetrahydrofuro[3,4 - b]pyridine - 3 - carboxylic acid ethyl ester.

25. A process according to claim 1 for the preparation of 2 - methyl - 4 - (1 - oxido - 2 - chloro - 3 - pyridyl) - 5 - oxo - 1,4,5,7 - tetrahydrofuro[3,4 - b]pyridine - 3 - carboxylic acid ethyl ester.

26. A process according to claim 1 for the preparation of 2 - methyl - 4 - (1 - oxido - 2 - methyl - 3 -

pyridyl) - 5 - oxo - 1,4,5,7 - tetrahydrofuro[3,4 - b]pyridine - 3 - carboxylic acid ethyl ester.

27. A process according to claim 1 for the preparation of 2 - methyl - 4 - (1 - oxido - 2 - methylthio - 3 - pyridyl) - 5 - oxo - 1,4,5,7 - tetrahydrofuro[3,4 - b]pyridine - 3 - carboxylic acid ethyl ester.

28. A process for the preparation of a pharmaceutical composition, which comprises processing a compound as claimed in any one of claims 1 to 27, with the optional addition of excipients, to such a composition.

**Revendications pour les Etats Contractants: BE CH DE FR IT LI LU NL SE**

1. Composés de formule

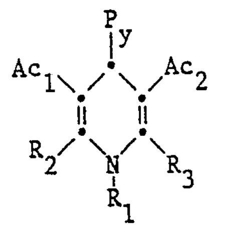

(I),

dans laquelle Py représente un groupe N-oxydopyridyle non substitué ou substitué par des groupes alkyle inférieurs en $C_1$—$C_7$, alcoxy inférieurs en $C_1$—$C_7$, alkylthio inférieurs en $C_1$—$C_7$, alkylsulfinyle inférieurs en $C_1$—$C_7$, alkylsulfonyle inférieurs en $C_1$—$C_7$ et/ou des halogènes, $R_1$ représente l'hydrogène, un groupe alkyle inférieur en $C_1$—$C_7$, di-(alkyle inférieur en $C_1$—$C_7$)-amino-alkyle inférieur en $C_1$—$C_7$, (alkylène inférieure en $C_4$—$C_6$)-amino-alkyle inférieur en $C_1$—$C_7$, morpholino-alkyle inférieur en $C_1$—$C_7$, thiomorpholino-alkyle inférieur en $C_1$—$C_7$, pipérazino-alkyle inférieur en $C_1$—$C_7$ ou 4-(alkyle inférieur en $C_1$—$C_7$)-pipérazino-alkyle inférieur en $C_1$—$C_7$, l'un des symboles $R_2$ et $R_3$ représente un groupe alkyle inférieur en $C_1$—$C_7$ et l'autre l'hydrogène, un groupe alkyle inférieur en $C_1$—$C_7$, hydroxyalkyle inférieur en $C_1$—$C_7$, (alcoxy inférieur en $C_1$—$C_7$)-alkyle inférieur en $C_1$—$C_7$, gem-di-(alcoxy inférieur en $C_1$—$C_7$)-alkyle inférieur en $C_1$—$C_7$, halogéno-alkyle inférieur en $C_1$—$C_7$, oxo-alkyle inférieur en $C_1$—$C_7$, (alcoxy inférieur en $C_1$—$C_7$)-carbonylalkyle inférieur en $C_1$—$C_7$, carbamoyl-(alkyle inférieur en $C_1$—$C_7$), N-(alkyle inférieur en $C_1$—$C_7$)-carbamoylalkyle inférieur en $C_1$—$C_7$, N,N-di-(alkyle inférieur en $C_1$—$C_7$)-carbamoyl-alkyle inférieur en $C_1$—$C_7$, cyano-(alkyle inférieur en $C_1$—$C_7$), aminoalkyle inférieur en $C_1$—$C_7$, (alkyle inférieur en $C_1$—$C_7$)-amino-alkyle inférieur en $C_1$—$C_7$, di-(alkyle inférieur en $C_1$—$C_7$)-amino-alkyle inférieur en $C_1$—$C_7$, (alkylène inférieur en $C_4$—$C_6$)-amino-alkyle inférieur en $C_1$—$C_7$, morpholino-alkyle inférieur en $C_1$—$C_7$, thiomorpholino-alkyle inférieur en $C_1$—$C_7$, pipérazino-alkyle inférieur en $C_1$—$C_7$, 4-(alkyle inférieur en $C_1$—$C_7$)-pipérazino-alkyle inférieur en $C_1$—$C_7$, (alcoxy inférieur en $C_1$—$C_7$)-imino-alkyle inférieur en $C_1$—$C_7$ dans lequel le groupe alcoxy inférieur en $C_1$—$C_7$ et le groupe imino sont substituants du même atome de carbone, hydroxyimino-alkyle inférieur en $C_1$—$C_7$, (alcoxy inférieur en $C_1$—$C_7$)-imino-alkyle inférieur en $C_1$—$C_7$, (alcoxy inférieur en $C_1$—$C_7$)-carbonyle, carbamoyle, N-(alkyle inférieur en $C_1$—$C_7$)-carbamoyle, N,N-di-(alkyle inférieur en $C_1$—$C_7$)-carbamoyle, cyano, amino, (alkyle inférieur en $C_1$—$C_7$)-amino, di-(alkyle inférieur en $C_1$—$C_7$)-amino-(alkyle inférieur en $C_1$—$C_7$)-amino, (alkylène inférieur en $C_4$—$C_6$)-amino-(alkyle inférieur en $C_1$—$C_7$)-amino, morpholino-(alkyle inférieur en $C_1$—$C_7$)-amino, di-(alkyle inférieur en $C_1$—$C_7$)-amino, (alkylène inférieur en $C_4$—$C_6$)-amino, (2-oxo-1-imidazolidinyl)-(alkylène inférieur en $C_4$—$C_6$)-amino, morpholino, thiomorpholino, pipérazino, 4-(alkyle inférieur en $C_1$—$C_7$)-pipérazino, 4-(alcanoyle inférieur en $C_1$—$C_7$)-pipérazino, 4-benzoyl-pipérazino, 4-furoyl-pipérazino ou 4-thiénoyl-pipérazino, un groupe amino $R_2$ ou $R_3$ pouvant également être relié à un groupe alkyle inférieur $R_1$ et former avec ce dernier un groupe 1-aza-(alkylène inférieur en $C_3$—$C_4$) dont l'atome d'azote est relié à l'atome de carbone cyclique en position 2 ou 6 du cycle 1,4-dihydropyridine, ou bien, lorsque $R_2$ ou $R_3$ représente un groupe alkyle inférieur en $C_1$—$C_7$, celui-ci peut former avec le radical acyle voisin $Ac_1$ ou $Ac_2$ respectivement, un groupe 2-oxa-1-oxo-(alkylène inférieur en $C_2$—$C_3$) dont le groupe carbonyle est relié à l'atome de carbone cyclique en position 3 ou 5 du cycle 1,4-dihydropyridine, et chacun des symboles $Ac_1$ et $Ac_2$ représente, indépendamment de l'autre, un groupe alcanoyle inférieur en $C_1$—$C_7$, benzoyle non substitué ou substitué par un groupe alkyle inférieur en $C_1$—$C_7$, alcoxy inférieur en $C_1$—$C_7$ et/ou un halogène, un groupe alkylsulfonyle inférieur en $C_1$—$C_7$, un groupe phénylsulfonyle non substitué ou substitué par un groupe alkyle inférieur en $C_1$—$C_7$, alcoxy inférieur en $C_1$—$C_7$ et/ou un halogène, un groupe (alkoxy inférieur en $C_1$—$C_7$)-carbonyle, hydroxy-(alcoxy inférieur en $C_1$—$C_7$)-carbonyle, (alcoxy inférieur en $C_1$—$C_7$)-(alcoxy inférieur en $C_1$—$C_7$)-carbonyle, amino-(alcoxy inférieur en $C_1$—$C_7$)-carbonyle, (alkyle inférieur en $C_1$—$C_7$)-amino-(alcoxy inférieur en $C_1$—$C_7$)-carbonyle, di-(alkyle inférieur en $C_1$—$C_7$)-amino-(alcoxy inférieur en $C_1$—$C_7$)-carbonyle, N-(alkyle inférieur en $C_1$—$C_7$)-N-phényl-(alkyle inférieur en $C_1$—$C_7$)-amino-(alcoxy inférieur en $C_1$—$C_7$)-carbonyle, (alkylène inférieur en $C_4$—$C_6$)-amino-(alcoxy inférieur en $C_1$—$C_7$)-carbonyle, morpholino-(alcoxy inférieur en $C_1$—$C_7$)-carbonyle, thiomorpholino-(alcoxy inférieur en $C_1$—$C_7$)-carbonyle, pipérazino-(alcoxy inférieur en $C_1$—$C_7$)-carbonyle, 4-(alkyle inférieur en $C_1$—$C_7$)-pipérazino-(alcoxy inférieur en $C_1$—$C_7$)-carbonyle, phényl-(alcoxy inférieur en $C_1$—$C_7$)-carbonyle non substitué ou substitué par des groupes alkyle inférieur

en $C_1$—$C_7$, alcoxy inférieurs en $C_1$—$C_7$ et/ou des halogènes, carbamoyle, N-(alkyle inférieur en $C_1$—$C_7$)-carbamoyle, N,N-di-(alkyle inférieur en $C_1$—$C_7$)-carbamoyle, N,N-(alkylène inférieur en $C_4$—$C_6$)-carbamoyle, morpholinocarbonyle, thiomorpholinocarbonyle, pipérazinocarbonyle ou 4-(alkyle inférieur en $C_1$—$C_7$)-pipérazinocarbonyle, ou bien les sels de ces composés portant des groupes aptes à former des sels.

2. Composés de formule I selon la revendication 1, dans laquelle Py, $R_1$, $Ac_1$ et $Ac_2$ ont les significations indiquées dans la revendication 1, et l'un des symboles $R_2$ et $R_3$ représente un groupe alkyle inférieur en $C_1$—$C_7$ et l'autre l'hydrogène, un groupe alkyle inférieur en $C_1$—$C_7$, hydroxyalkyle inférieur en $C_1$—$C_7$, (alcoxy inférieur en $C_1$—$C_7$)-alkyle inférieur en $C_1$—$C_7$, halogénoalkyle inférieur en $C_1$—$C_7$, (alcoxy inférieur en $C_1$—$C_7$)-carbonyl-alkyle inférieur en $C_1$—$C_7$, carbamoyl-(alkyle inférieur en $C_1$—$C_7$), N-(alkyle inférieur en $C_1$—$C_7$)-carbamoyl-(alkyle inférieur en $C_1$—$C_7$)-, N,N-di-(alkyle inférieur en $C_1$—$C_7$)-carbamoyl-(alkyle inférieur en $C_1$—$C_7$), cyano-(alkyle inférieur en $C_1$—$C_7$), aminoalkyle inférieur en $C_1$—$C_7$, (alkyle inférieur en $C_1$—$C_7$)--amino-alkyle inférieur en $C_1$—$C_7$, di-(alkyle inférieur en $C_1$—$C_7$)-amino-alkyle inférieur en $C_1$—$C_7$, (alkylène inférieur en $C_4$—$C_6$)-amino-alkyle inférieur en $C_1$—$C_7$, morpholino-alkyle inférieur en $C_1$—$C_7$, thiomorpholino-alkyle inférieur en $C_1$—$C_7$, pipérazino-alkyle inférieur en $C_1$—$C_7$, 4-(alkyle inférieur en $C_1$—$C_7$)-pipérazino-alkyle inférieur en $C_1$—$C_7$, (alcoxy inférieur en $C_1$—$C_7$)-carbonyle, carbamoyle, N-(alkyle inférieur en $C_1$—$C_7$)-carbamoyle, N,N-di-(alkyle inférieur en $C_1$—$C_7$)-carbamoyle, cyano, amino, alkylamino inférieur en $C_1$—$C_7$, di-(alkyle inférieur en $C_1$—$C_7$)-amino-(alkyle inférieur en $C_1$—$C_7$)-amino, (alkylène inférieur en $C_4$—$C_6$)-amino-(alkyle inférieur en $C_1$—$C_7$)-amino, morpholino-(alkyle inférieur en $C_1$—$C_7$)-amino, di-(alkyle inférieur en $C_1$—$C_7$)-amino, (alkylène inférieur en $C_4$—$C_6$)-amino, (2-oxo-1-imidazolidinyl)-pipéridino, morpholino, thiomorpholino, pipérazino, 4-(alkyle inférieur en $C_1$—$C_7$)-pipérazino, 4-(alcanoyle inférieur en $C_1$—$C_7$)-pipérazino, 4-benzoyl-pipérazino, 4-furoyl-pipérazino ou 4-thiénoyl-pipérazino, un groupe amino $R_2$ ou $R_3$ pouvant également être relié avec un groupe alkyle inférieur en inférieur en $C_1$—$C_7$ $R_1$ et former avec ce dernier un groupe 1-aza-(alkylène inférieur en $C_3$—$C_4$) dont l'atome d'azote est relié à l'atome de carbone cyclique en position 2 ou 6 respectivement du cycle 1,4-dihydropyridine, ou bien, lorsque $R_2$ ou $R_3$ représente un groupe alkyle inférieur en $C_1$—$C_7$, celui-ci peut former avec le radical acyle voisin $Ac_1$ ou $Ac_2$ respectivement un groupe 2-oxa-1-oxo-(alkylène inférieur en $C_2$—$C_3$) dont le groupe carbonyle est relié à l'atome de carbone cyclique en position 3 ou 5 respectivement du cycle 1,4-dihydropyridine, ou les sels de ces composés portant des groupes aptes à une salification.

3. Composés de formule I selon la revendication 1, dans laquelle Py représente un groupe N-oxydopyridyle non substitué ou substitué par un groupe alkyle inférieur en $C_1$—$C_4$ ou alkylsulfinyle inférieur en $C_1$—$C_7$, $R_1$ représente l'hydrogène, un groupe alkyle inférieur en $C_1$—$C_4$, 2-di-(alkyle inférieur en $C_1$—$C_7$)-amino-alkyle inférieur en $C_1$—$C_7$, 2-(alkylène inférieur en $C_4$—$C_0$)-amino-alkyle inférieur en $C_1$—$C_7$, ou 2-(4-morpholino)-alkyle inférieur en $C_1$—$C_7$, l'un des symboles $R_2$ et $R_3$ représente un groupe alkyle inférieur en $C_1$—$C_4$ et l'autre l'hydrogène, un groupe alkyle inférieur en $C_1$—$C_7$, hydroxyalkyle inférieur en $C_1$—$C_7$, halogénoalkyle inférieur en $C_1$—$C_7$, (alcoxy inférieur en $C_1$—$C_7$)-carbonyl-inférieur en $C_1$—$C_7$, cyano(alkyle inférieur en $C_1$—$C_7$), di-(alkyle inférieur en $C_1$—$C_7$)-amino-alkyle inférieur en $C_1$—$C_7$, (alcoxy inférieur en $C_1$—$C_7$)-carbonyle, cyano, amino, 4-morpholino-(alkyle inférieur en $C_1$—$C_7$)-amino, (alkylène inférieur en $C_4$—$C_6$)-amino, (2-oxo-1-imidazolidinyl)-pipérazino ou bien 4-(2-oxo-1-imidazolidinyl)-pipérazino ou bien 4-(2-furoyl)-pipérazino, un groupe amino $R_2$ ou $R_3$ pouvant être relié à un groupe alkyle inférieur en $C_1$—$C_7$ $R_1$ et former avec ce dernier un groupe 1-aza-alkylène inférieur dont l'atome d'azote du groupe aza est relié à l'atome de carbone cyclique en position 2 ou 6 du cycle 1,4-dihydropyridine, ou bien, lorsque $R_2$ ou $R_3$ représente un groupe alkyle inférieur en $C_1$—$C_7$, celui-ci peut former avec le radical acyle voisin $Ac_1$ ou $Ac_2$ respectivement un groupe 2-oxa-1-oxo-alkylène inférieur dont le groupe carbonyle est relié à l'atome de carbone cyclique en position 3 ou 5 du cycle 1,4-dihydropyridine et chacun des symboles $Ac_1$ et $Ac_2$ représente, indépendamment l'un de l'autre, un groupe alcanoyle inférieur, (alkyle inférieur en $C_1$—$C_7$)-sulfonyle, (alcoxy inférieur en $C_1$—$C_7$)-carbonyle, hydroxy-(alcoxy inférieur en $C_1$—$C_7$)-carbonyle, (alcoxy inférieur en $C_1$—$C_7$)-(alcoxy inférieur en $C_1$—$C_7$)-carbonyle, di-(alkyle inférieur en $C_1$—$C_7$)-amino-(alcoxy inférieur en $C_1$—$C_7$)-carbonyle, N-(alkyle inférieur en $C_1$—$C_7$)-N-phényl-(alkyle inférieur en $C_1$—$C_7$)-amino-(alcoxy inférieur en $C_1$—$C_7$-carbonyle, (alkylène inférieur en $C_4$—$C_6$-amino-(alcoxy inférieur en $C_1$—$C_7$)-carbonyle, (4-morpholino)-(alcoxy inférieur en $C_1$—$C_7$)-carbonyle, carbamoyle, N-(alkyle inférieur en $C_1$—$C_7$)-carbamoyle, N,N-di-(alkylène inférieur en $C_1$—$C_7$)-carbamoyle, N,N-(alkylène inférieur en $C_4$—$C_5$)-carbamoyle ou 4-morpholinocarbonyle, les groupes alkyle inférieurs, alcoxy inférieurs ou alcanoyle inférieurs contenant jusqu'à 4 atomes de carbone inclus, les groupes alkylène inférieurs 4 ou 5 atomes de carbone dans la chaîne, les groupes 1-aza-alkylène inférieurs 2 ou 3 atomes de carbone dans la chaîne et les groupes 2-oxa-1-oxo-alkylène inférieurs 2 atomes de carbone dans la chaîne, ou les sels de ces composés portant des groupes aptes à une alification.

4. Composés de formule I selon la revendication 1, dans laquelle Py représente un groupe N-oxydopyridyle, $R_1$ représente l'hydrogène, un groupe alkyle inférieur en $C_1$—$C_4$, 2-(di-(alkyle inférieur en $C_1$—$C_7$)-amino)-alkyle inférieur en $C_1$—$C_4$, 2-(alkylène inférieur en $C_4$—$C_6$)-amino)-alkyle inférieur en $C_1$—$C_4$ ou 2-(4-morpholino)-alkyle inférieur en $C_1$—$C_4$, l'un des symboles $R_2$ et $R_3$ représente un groupe alkyle inférieur en $C_1$—$C_4$ et l'autre un groupe alkyle inférieur en $C_1$—$C_4$, hydroxyalkyle inférieur en $C_1$—$C_7$, halogénoalkyle inférieur en $C_1$—$C_7$, 2-(di(alkyle inférieur en $C_1$—$C_7$)-amino)-alkyle inférieur en $C_1$—$C_4$, (alcoxy inférieur en $C_1$—$C_7$)-carbonyle, cyano, amino, (4-morpholino)-(alkyle inférieur en $C_1$—$C_7$)-amino, (alkylène inférieur en $C_4$—$C_6$)-amino, (2-oxo-1-imidazolidinyl)-(alkylène inférieur en $C_4$—$C_6$)-amino ou 4-(2-furoyl)-pipérazino, un groupe amino $R_2$ ou $R_3$ pouvant être relié à un groupe alkyle inférieur en $C_1$—$C_4$ $R_1$ et former avec celui-ci un

groupe 1-aza-(alkylène inférieur en $C_2$—$C_3$) dont l'atome d'azote du groupe aza est relié à l'atome de carbone cyclique en position 2 ou 6 du cycle 1,4-dihydropyridine, ou bien, lorsque $R_2$ ou $R_3$ représente un groupe alkyle inférieur en $C_1$—$C_7$, celui-ci peut former avec le radical acyle voisin $Ac_1$ ou $Ac_2$ respectivement un groupe 2-oxa-1-oxo-(alkylène inférieur en $C_2$—$C_3$) dont le groupe carbonyle est relié à l'atome de carbone cyclique en position 3 ou 5 du cycle 1,4-dihydropyridine, et chacun des symboles $Ac_1$ et $Ac_2$ représente, indépendamment de l'autre, un groupe alcanoyle inférieur en $C_1$—$C_4$, alkylsulfonyle inférieur en $C_1$—$C_7$, (alcoxy inférieur en $C_1$—$C_7$)-carbonyle, 2-(alcoxy inférieur en $C_1$—$C_4$)-(alcoxy inférieur en $C_1$—$C_4$)-carbonyle, di-(alkyle inférieur en $C_1$—$C_7$)-amino-(alcoxy inférieur en $C_1$—$C_7$)-carbonyle ou N-(alkyle inférieur en $C_1$—$C_4$)-N-phényl-(alkyle inférieur en $C_1$—$C_4$)-amino-(alcoxy inférieur en $C_1$—$C_7$)-carbonyle, carbamoyle, N-(alkyle inférieur en $C_1$—$C_4$)-carbamoyle, N,N-di-(alkyle inférieur en $C_1$—$C_7$)-carbamoyle ou 4-morpholino-carbonyle, les groupes alkyle inférieurs, alcoxy inférieurs et alcanoyle inférieurs contenant jusqu'à 4 atomes de carbone incus, les groupes alkylène inférieurs 4 ou 5 atomes de carbone dans la chaîne, les groupes 1-aza-alkylène inférieurs 2 ou 3 atomes de carbone dans la chaîne et le groupe 2-oxa-1-oxo-alkylène inférieur 2 atomes de carbone dans la chaîne, ou les sels de ces composés portant des groupes basiques salifiables.

5. Composés de formule I selon la revendication 1, dans laquelle Py représente un groupe N-oxydopyridyle, $R_1$ représente l'hydrogène ou un groupe 2-(4-morpholino)-éthyle, l'un des symboles $R_2$ et $R_3$ représente un groupe méthyle et l'autre un groupe méthyle, hydroxyméthyle, (alcoxy inférieur en $C_1$—$C_4$)-carbonyle, par exemple méthoxycarbonyle, éthoxycarbonyle, n-propyloxycarbonyle, isopropyloxycarbonyle, n-butyloxycarbonyle, ou isobutyloxycarbonyle, cyano ou amino, et chacun des symboles $Ac_1$ et $Ac_2$ représente, indèpendamment de l'autre, un groupe alcanoyle inférieur, par exemple acétyle, alkylsulfonyle, inférieur en $C_1$—$C_7$, par exemple éthylsulfonyle, (alcoxy inférieur)-carbonyle, par exemple méthoxycarbonyle, éthoxycarbonyle, n-propyloxycarbonyle, isopropyloxycarbonyle, n-butyloxycarbonyle ou isobutyloxycarbonyle, carbamoyle, N-(alkyle inférieur)-carbamoyle, par exemple N-mèthylcarbamoyle, ou N,N-di-(alkyle inférieur)-carbamoyle, par exemple N,N-diméthylcarbamoyle, les groupes alkyle inférieurs, alcoxy inférieurs et alcanoyle inférieurs contenant jusqu'à 4 atomes de carbone inclus, ou les sels de ces composés portant des groupes basiques salifiables.

6. Le 2,6 - diméthyl - 4 - (1 - oxydo - 3 - pyridyl) - 1,4 - dihydro - pyridine - 3,5 - dicarboxylate de diméthyle.

7. Le 2,6 - diméthyl - 4 - (1 - oxydo - 3 - pyridyl) - 1,4 - dihydro - pyridine - 3,5 - dicarboxylate de diéthyle.

8. Le 2,6 - diméthyl - 4 - (1 - oxydo - 3 - pyridyl) - 1,4 - dihydro - pyridine - 3 - carboxylate d'isobutyle, 5 - carboxylate de méthyle.

9. Le 2,6 - diméthyl - 4 - (1 - oxydo - 2 - pyridyl) - 1,4 - dihydro - pyridine - 3,5 - dicarboxylate de diéthyle.

10. Le 2,6 - diméthyl - 4 - (1 - oxydo - 4 - pyridyl) - 1,4 - dihydro - pyridine - 3,5-dicarboxylate de diéthyle.

11. Le 2,6 - diméthyl - 4 - (1 - oxydo - 3 - pyridyl) - 1,4 - dihydro - pyridine - 3,5 - dicarboxylate de di - n - propyle.

12. Le 2,6 - diméthyl - 4 - (2 - méthyl - 1 - oxydo - 3 - pyridyl) - 1,4 - dihydro - pyridine - 3,5 - dicarboxylate de diéthyle.

13. Le 2,6 - diméthyl - 4 - (2 - chloro - 1 - oxydo - 3 - pyridyl) - 1,4 - dihydro - pyridine - 3,5 - dicarboxylate de diéthyle.

14. Le 2,6 - diméthyl - 4 - (1 - oxydo - 3 - pyridyl) - 1,4 - dihydro - pyridine - 3 - carboxylate d'isopropyl,5 - carboxylate de 2 - méthoxyéthyle.

15. Le 2,6 - diméthyl - 4 - (1 - oxydo - 3 - pyridyl) - 1,4 - dihydro - pyridine - 3 - carboxylate de méthyle, 5 - carboxylate de 2 - (N - méthyl - N - benzyl - amino) - éthyle.

16. Le 6 - méthoxyméthyl - 2 - méthyl - 4 - (1 - oxydo - 3 - pyridyl) - 1,4 - dihydro - pyridine - 3 - carboxylate d'éthyle,5 - carboxylate de méthyle.

17. Le 2 - amino - 6 - méthyl - 4 - (1 - oxydo - 3 - pyridyl) - 1,4 - dihydro - pyridine - 3,5 - pyridine - dicarboxylate de diéthyle.

18. Le 2,6 - diméthyl - 4 - (2 - méthylthio - 1 - oxydo - 3 - pyridyl) - 1,4 - dihydro - pyridine - 3,5 - dicarboxylate de diéthyle.

19. Le 2,6 - diméthyl - 4 - (2 - méthyl - 1 - oxydo - 3 - pyridyl) - 1,4 - dihydro - pyridine - 3 - carboxylate d'isobutyle, 5 - carboxylate de méthyle.

20. Le 6 - hydroxyméthyl - 2 - méthyl - 4 - (1 - oxydo - 2 - méthyl - 3 - pyridyl) - 1,4 - dihydro - pyridine - 3,5 - dicarboxylate de diéthyle.

21. Le 6 - cyano - 2 - méthyl - 4 - (1 - oxydo - 2 - méthyl - 3 - pyridyl) - 1,4 - dihydro - pyridine - 3,5 - dicarboxylate de diéthyle.

22. Le 2,6 - diméthyl - 4 - (1 - oxydo - 2 - méthoxy - 3 - pyridyl) - 1,4 - dihydro - pyridine - 3,5 - dicarboxylate de diéthyle.

23. Le 2,6 - diméthyl - 4 - (2 - méthylsulfinyl - 1 - oxydo - 3 - pyridyl) - 1,4 - dihydro - pyridine - 3,5 - dicarboxylate de diéthyle.

24. Le 2 - méthyl - 4 - (1 - oxydo - 3 - pyridyl) - 5 - oxo - 1,4,5,7 - tétrahydrofuro[3,4 - b]pyridine - 3 - carboxylate d'éthyle.

25. Le 2 - méthyl - 4 - (1 - oxydo - 2 - chloro - 3 - pyridyl) - 5 - oxo - 1,4,5,7 - tétrahydrofuro[3,4 - b]pyridine - 3 - carboxylate d'éthyle.

26. Le 2 - méthyl - 4 - (1 - oxydo - 2 - méthyl - 3 - pyridyl) - 5 - oxo - 1,4,5,7 - tétrahydrofuro[3,4 - b]pyridine - 3 - carboxylate d'éthyle.

27. Le 2 - méthyl - 4 - (1 - oxydo - 2 - méthylthio - 3 - pyridyl) - 5 - oxo - 1,4,5,7 - tétrahydrofuro-[3,4 - b]pyridine - 3 - carboxylate d'éthyle.

28. Les composés des revendications 1 à 16 en tant que composés possédant une activité pharmacologique, en particulier dans le domaine cardiovasculaire.

29. Les composés des revendications 2, 6 à 15 et 17, en tant que composés possédant une activité pharmacologique, en particulier dans le domaine cardiovasculaire.

30. Les composés des revendications 1, 3 à 5 et 17 à 27, en tant que composés possédant une activité pharmacologique, en particulier dans le domaine cardiovasculaire.

31. Les composés des revendication 1, à l'exception de ceux dans lesquels $R_2$ ou $R_3$ représente un groupe alkyle inférieur en $C_1$—$C_7$, et celui-ci forme avec le radical acyle voisin $Ac_1$ ou $Ac_2$ respectivement un groupe 2-oxa-1-oxo-(alkylène inférieur en $C_2$—$C_3$) dont le groupe carbonyle est relié à l'atome de carbone cyclique en position 3 ou 5 du cycle 1,4-dihydropyridine, et les composés de la revendication 16, en tant que composants possédant une activité pharmacologique, en particulier hypotensive.

32. Les composés de la revendication 2, à l'exception de ceux dans lesquels $R_2$ ou $R_3$ représente un groupe alkyle inférieur en $C_1$—$C_7$ et cleui-ci forme avec le radical acyle voisin $Ac_1$ ou $Ac_2$ respectivement un groupe 2-oxa-1-oxo-(alkylène inférieur en $C_2$—$C_3$) dont le groupe carbonyle est relié à l'atome de carbone cyclique en position 3 ou 5 du cycle 1,4-dihydropyridine, ainsi que les composés des revendications 6 à 15 et 17, en tant que composés possédant une activité pharmacologique, en particulier hypotensive.

33. Les composés des revendications 1 et 3 à 5 à l'exception de ceux dans lesquels $R_2$ ou $R_3$ représente un groupe alkyle inférieur en $C_1$—$C_7$ ou méthyle, et celui-ci forme avec le radical acyle voisin $Ac_1$ oui $Ac_2$ respectivement un groupe 2-oxa-1-oxo-(alkylène inférieur en $C_2$—$C_3$) ou un groupe 2-oxa-1-oxo-1,3-propylène respectivement, dont le groupe carbonyle est relié dans chaque cas à l'atome de carbone cyclique en position 3 ou 5 respectivement du cycle 1,4-dihydropyridine, ainsi que les composés des revendications 18 à 23, en tant que composés possédant une activité pharmacologique, en particulier hypotensive.

34. Les composés de la revendication 1, dans lesquels $R_2$ ou $R_3$ représente un groupe alkyle inférieur en $C_1$—$C_7$ et celui-ci forme avec le radical voisin $Ac_1$ ou $Ac_2$ respectivement un groupe 2-oxa-1-oxo-(alkylène inférieur en $C_2$—$C_3$) dont le groupe carbonyle est relié à l'atome de carbone cyclique en position 3 ou 5 du cycle 1,4-dihydropyridine, ainsi que les composés de la revendication 16, en tant que composés possédant une activité pharmacologique et en particulier augmentant la contractilité myocardiaque.

35. Le composé de la revendication 2, dans lesquels $R_2$ ou $R_3$ représente un groupe alkyle inférieur en $C_1$—$C_7$ et celui-ci forme avec le radical acyle voisin $Ac_1$ ou $Ac_2$ respectivement un groupe 2-oxa-1-oxo-(alkylène inférieur en $C_2$—$C_3$) dont le groupe carbonyle est relié à l'atome de carbone cyclique en position 3 ou 5 respectivement du cycle 1,4-dihydropyridine, ainsi que les composés des revendications 6 à 15 et 17, en tant que composés possédant une activité pharmacologique et en particulier augmentant la contractilité myocardiaque.

36. Les composés de la revendications 1 et 3 à 5, dans lesquels $R_2$ ou $R_3$ représente un groupe alkyle inférieur en $C_1$—$C_7$ et celui-ci forme avec le radical acyle voisin $Ac_1$ ou $Ac_2$ respectivement un groupe 2-oxa-1-oxo(alkylène inférieur en $C_2$—$C_3$) dont le groupe carbonyle est relié à l'atome de carbone cyclique en position 3 ou 5 respectivement du cycle 1,4-dihydropyridine, ainsi que les composés des revendications 26 à 27 en tant que composés possédant une activité pharmacologique, et en particulier augmentant la contractilité myocardiaque.

37. Le composé de la revendication 16, pour le traitement de l'organisme humain ou animal.

38. Les composés de la revendications 2, 6 à 15 et 17, pour le traitement de l'organisme humain ou animal.

39. Les composés de la revendication 1, 3 à 7 et 18 à 27, pour le traitement de l'organisme humain ou animal.

40. Utilisation du composé de la revendication 16 ou de sels de ce composé acceptables pour l'usage pharmaceutique, pour la préparation de compositions pharmaceutiques pour le traitement prophylactique et/ou thérapeutique de l'hypertonie d'une part et d'autres maladies cardiovasculaires d'autre part.

41. Utilisation des composés des revendications 2, 6 à 15 et 17, ou de sels de ces composés acceptables pour l'usage pharmaceutique pour la préparation de compositions pharmaceutiques pour le traitement prophylactique et/ou thérapeutique de l'hypertonie d'une part et d'autres maladies cardiovasculaires d'autre part.

42. Utilisation des composés des revendications 1, 3 à 5 et 18 à 23, ou de leurs sels acceptables pour l'usage pharmaceutique, pour la préparation de compositions pharmaceutiques pour le traitement prophylactique et/ou thérapeutique de l'hypertonie d'une part et d'autres maladies cardiovasculaires d'autre part.

43. Compositions pharmaceutiques contenant le composé de la revendication 16.

44. Compositions pharmaceutiques contenant l'un des composés des revendications 2, 6 à 15 et 17.

45. Compositions pharmaceutiques contenant l'un des composés des revendications 1, 3 à 5 et 18 à 27.

46. Utilisation du composés de la revendication 16 pour la préparation dė compositions pharmaceutiques.

47. Utilisation des composés des revendications 2, 6 à 15 et 17 pour la préparation de compositions pharmaceutiques.

48. Utilisation des composés des revendications 1, 3 à 5 et 18 à 27 pour la préparation de compositions pharmaceutiques.

49. Procédé de préparation de compositions pharmaceutiques, caractérisé en ce que l'on met le composé de la revendication 16, éventuellement en mélange avec des produits auxiliaires, sous la forme de compositions pharmaceutiques.

50. Procédé de préparation de compositions pharmaceutiques, caractérisé en ce que l'on met des composés des revendications 2, 6 à 15 et 17, éventuellement en mélange avec des produits auxiliaires, sous la forme de compositions pharmaceutiques.

51. Procédé de préparation de compositions pharmaceutiques, caractérisé en ce que l'on met des composés des revendications 1, 3 à 5 et 18 à 27, éventuellement en mélange avec des produits auxiliaires, sous la forme de compositions pharmaceutiques.

52. Procédé de préparation des composés de formule I définis dans les revendications 1 et 16, caractérisé en ce que:

a) on cyclise un composé de formule

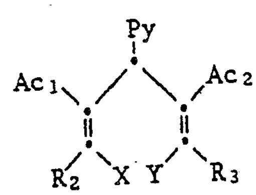

(II)

dans laquelle l'un des symboles X et Y représente le groupe de formule —NH—$R_1$ et l'autre un groupe hydroxy ou le groupe de formule —NH—$R_1$, ou un tautomère d'un tel composé, ou un mélange des tautomères correspondants, ou bien

b) on fait réagir un composé de formule Py—CHO (III) ou un dérivé fonctionnel réactif de ce composé avec un composé de formule

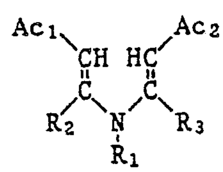

(IV)

ou un tautomère d'un tel composé ou un mélange de tautomères correspondants, ou bien

c) dans un composé de formule

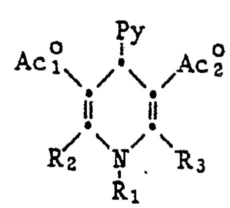

(V)

dans laquelle l'un des symboles $Ac_1^o$ et $Ac_2^o$ représente un reste convertible respectivement en le reste $Ac_1$ ou $Ac_2$, et l'autre représente $Ac_1$ ou $Ac_2$ respectivement ou un reste convertibe en $Ac_1$ ou $Ac_2$ respectivement, on convertit le reste $Ac_1^o$ et/ou $Ac_2^o$ en un reste $Ac_1$ et/ou $Ac_2$, ou bien

d) dans un composé de formule

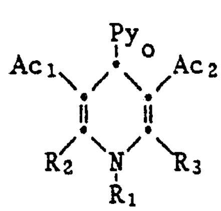

(VI)

dans laquelle $Py_o$ représente un reste pyridyle non substitué ou substitué, on oxyde le reste $Py_o$ en le reste N-oxydo-pyridyle correspondant,
la formation des composés de formule I dans laquelle $R_2$ ou $R_3$, étant un groupe alkyle inférieur en $C_1$—$C_7$, forme avec le radical acyle voisin $Ac_1$ ou $Ac_2$ respectivement, un reste 2-oxa-1-oxo-alkylène inférieur en $C_2$—$C_3$ dont le groupe carbonyle est relié avec l'atome de carbone cyclique en position 3 ou 5 du cycle 1,4-dihydropyridine, à partir de composés de départ de formules II, IV, V et VI, dans lesquelles $R_2$ ou $R_3$ respectivement représente un groupe alkyle inférieur contenant un groupe hydroxy libre ou estérifié, et $Ac_1$ ou $Ac_2$, ou bien $Ac_1^o$ et $Ac_2^o$ représente, dans les composés de départ de formule V, un groupe carboxyle estérifié, pouvant être effectuée directement dans les conditions de réaction de l'un des procédés a) à d), les symboles Py, $R_1$, $R_2$, $R_3$, $Ac_1$ et $Ac_2$ ayant, dans les composés de départ ci-dessus de formules II à IV, lesquels, lorsqu'ils sont capables de former des sels, peuvent également être utilisés à l'état de sels, les significations indiquées en référence à la formule I, et si on le désire, on convertit un composé obtenu répondant à la formule I en un autre composé de formule I, et/ou, si on le désire, on convertit un sel obtenu en le composé libre ou en un autre sel, et/ou, si on le désire, on convertit un composé de formule I obtenu à l'état libre et capable de former des sels, en un sel, et/ou, si on le désire, on sépare un mélange d'isomères obtenu en les isomères individuels.

53. Procédé de préparation des composés de formule I définis dans les revendications 2, 6 à 15 et 17, caractérisé en ce que l'on met en oeuvre les procédés définis dans la revendication 52 avec les produits de départ correspondants.

54. Procédé de préparation des composés de formule I définis dans les revendications 1, 3 à 5 et 18 à 27, caractérisé en ce que l'on met en oeuvre les procédés définis dans la revendication 52 avec les produits de part correspondants.

**Revendications pour l'Etat Contractant: AT**

1. Procédé de préparation des composés de formule I

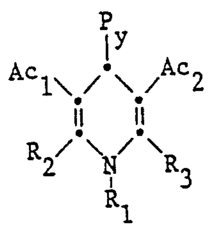

(I),

dans laquelle Py représente un groupe N-oxydopyridyle non substitué ou substitué par des groupes alkyle inférieurs en $C_1$—$C_7$, alcoxy inférieurs en $C_1$—$C_7$, alkylthio inférieurs en $C_1$—$C_7$, alkylsulfinyle inférieurs en $C_1$—$C_7$, alkylsulfonyle inférieurs en $C_1$—$C_7$ et/ou des halogènes, $R_1$ représente l'hydrogène, un groupe alkyle inférieur en $C_1$—$C_7$, di-(alkyle inférieur en $C_1$—$C_7$)-amino-alkyle inférieur en $C_1$—$C_7$, (alkylène inférieure en $C_4$—$C_6$)-amino-alkyle inférieur en $C_1$—$C_7$, morpholino-alkyle inférieur en $C_1$—$C_7$, thiomorpholino-alkyle inférieur en $C_1$—$C_7$, pipérazino-alkyle inférieur en $C_1$—$C_7$ ou 4-(alkyle inférieur en $C_1$—$C_7$)-pipérazino-alkyle inférieur en $C_1$—$C_7$, l'un des symboles $R_2$ et $R_3$ représente un groupe alkyle inférieur en $C_1$—$C_7$ et l'autre l'hydrogène, un groupe alkyle inférieur en $C_1$—$C_7$, hydroxyalkyle inférieur en $C_1$—$C_7$, (alcoxy inférieur en $C_1$—$C_7$)-alkyle inférieur en $C_1$—$C_7$, gem-di-(alcoxy inférieur en $C_1$—$C_7$)-alkyle inférieur en $C_1$—$C_7$, halogéno-alkyle inférieur en $C_1$—$C_7$, oxo-alkyle inférieur en $C_1$—$C_7$, (alcoxy inférieur en $C_1$—$C_7$)-carbonylalkyle inférieur en $C_1$—$C_7$, carbamoyl-(alkyle inférieur en $C_1$—$C_7$), N-(alkyle inférieur en $C_1$—$C_7$)-carbamoylalkyle inférieur en $C_1$—$C_7$, N,N-di-(alkyle inférieur en $C_1$—$C_7$)-carbamoyl-alkyle inférieur en $C_1$—$C_7$, cyano-(alkyle inférieur en $C_1$—$C_7$), aminoalkyle inférieur en $C_1$—$C_7$, (alkyle inférieur en $C_1$—$C_7$)-amino-alkyle inférieur en $C_1$—$C_7$, di-(alkyle inférieur en $C_1$—$C_7$)-amino-alkyle inférieur en $C_1$—$C_7$, (alkylène inférieur en $C_4$—$C_6$)-amino-alkyle inférieur en $C_1$—$C_7$, morpholino-alkyle inférieur en $C_1$—$C_7$, thiomorpholino-alkyle inférieur en $C_1$—$C_7$, pipérazino-alkyle inférieur en $C_1$—$C_7$, 4-(alkyle inférieur en $C_1$—$C_7$)-pipérazino-alkyle inférieur en $C_1$—$C_7$, (alcoxy inférieur en $C_1$—$C_7$)-imino-alkyle inférieur en $C_1$—$C_7$ dans lequel le groupe alcoxy inférieur en $C_1$—$C_7$ et le groupe imino sont substituants du même atome de carbone, hydroxyimino-alkyle inférieur en $C_1$—$C_7$, (alcoxy inférieur en $C_1$—$C_7$)-imino-alkyle inférieur en $C_1$—$C_7$, (alcoxy inférieur en $C_1$—$C_7$)-carbonyle, carbamoyle, N-(alkyle inférieur en $C_1$—$C_7$)-carbamoyle, N,N-di-(alkyle inférieur en $C_1$—$C_7$)-carbamoyle, cyano, amino, (alkyle inférieur en $C_1$—$C_7$)-amino, di-(alkyle inférieur en $C_1$—$C_7$)-amino-(alkyle inférieur en $C_1$—$C_7$)-amino, (alkylène inférieur en $C_4$—$C_6$)-amino-(alkyle inférieur en $C_1$—$C_7$)-amino, morpholino-(alkyle inférieur en $C_1$—$C_7$)-amino, di-(alkyle inférieur en $C_1$—$C_7$)-amino, (alkylène inférieur en $C_4$—$C_6$)-amino, (2-oxo-1-imidazolidinyl)-(alkylène inférieur en $C_4$—$C_6$)-amino, morpholino, thiomorpholino, pipérazino, 4-(alkyle inférieur en $C_1$—$C_7$)-pipérazino, 4-(alcanoyle inférieur en $C_1$—$C_7$)-pipérazino, 4-benzoyl-pipérazino, 4-furoyl-pipérazino ou 4-thiénoyl-pipérazino, un groupe amino $R_2$ ou $R_3$ pouvant également être relié à un groupe alkyle inférieur $R_1$ et former avec ce dernier un groupe 1-aza-(alkylène inférieur en $C_3$—$C_4$) dont l'atome d'azote est relié à l'atome de carbone cyclique en position 2 ou 6 du cycle 1,4-dihydropyridine, ou bien, lorsque $R_2$ ou $R_3$ représente un groupe alkyle inférieur en

$C_1$—$C_7$, celui-ci peut former avec le radical acyle voisin $Ac_1$ ou $Ac_2$ respectivement, un groupe 2-oxa-1-oxo-(alkylène inférieur en $C_2$—$C_3$) dont le groupe carbonyle est relié à l'atome de carbone cyclique en position 3 ou 5 du cycle 1,4-dihydropyridine, et chacun des symboles $Ac_1$ et $Ac_2$ représente, indépendamment de l'autre, un groupe alcanoyle inférieur en $C_1$—$C_7$, benzoyle non substitué ou substitué par un groupe alkyle inférieur en $C_1$—$C_7$, alcoxy inférieur en $C_1$—$C_7$ et/ou un halogène, un groupe alkylsulfonyle inférieur en $C_1$—$C_7$, un groupe phénylsulfonyle non substitué ou substitué par un groupe alkyle inférieur en $C_1$—$C_7$, alcoxy inférieur en $C_1$—$C_7$ et/ou un halogène, un groupe (alkoxy inférieur en $C_1$—$C_7$)-carbonyle, hydroxy-(alcoxy inférieur en $C_1$—$C_7$)-carbonyle, (alcoxy inférieur en $C_1$—$C_7$)-(alcoxy inférieur en $C_1$—$C_7$)-carbonyle, amino-(alcoxy inférieur en $C_1$—$C_7$)-carbonyle, (alkyle inférieur en $C_1$—$C_7$)-amino-(alcoxy inférieur en $C_1$—$C_7$)-carbonyle, di-(alkyle inférieur en $C_1$—$C_7$)-amino-(alcoxy inférieur en $C_1$—$C_7$)-carbonyle, N-(alkyle inférieur en $C_1$—$C_7$)-N-phényl-(alkyle inférieur en $C_1$—$C_7$)-amino-(alcoxy inférieur en $C_1$—$C_7$)-carbonyle, (alkylène inférieur en $C_4$—$C_6$)-amino-(alcoxy inférieur en $C_1$—$C_7$)-carbonyle, morpholino-(alcoxy inférieur en $C_1$—$C_7$)-carbonyle, thiomorpholino-(alcoxy inférieur en $C_1$—$C_7$)-carbonyle, pipérazino-(alcoxy inférieur en $C_1$—$C_7$)-carbonyle, 4-(alkyle inférieur en $C_1$—$C_7$)-pipérazino-(alcoxy inférieur en $C_1$—$C_7$)-carbonyle, phényl-(alcoxy inférieur en $C_1$—$C_7$)-carbonyle non substitué ou substitué par des groupes alkyle inférieur en $C_1$—$C_7$, alcoxy inférieurs en $C_1$—$C_7$ et/ou des halogènes, carbamoyle, N-(alkyle inférieur en $C_1$—$C_7$)-carbamoyle, N,N-di-(alkyle inférieur en $C_1$—$C_7$)-carbamoyle, N,N-(alkylène inférieur en $C_4$—$C_6$)-carbamoyle, morpholinocarbonyle, thiomorpholinocarbonyle, pipérazinocarbonyle ou 4-(alkyle inférieur en $C_1$—$C_7$)-pipérazinocarbonyle, ou des sels de ces composés portant des groupes salifiables, caractérisé en ce que:

a) on cyclise un composé de formule

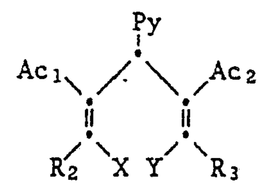

(II)

dans laquelle l'un des symboles X et Y représente le groupe de formule —NH—$R_1$ et l'autre un groupe hydroxy ou le groupe de formule —NH—$R_1$, ou un tautomère d'un tel composé, ou un mélange des tautomères correspondants, ou bien

b) on fait réagir un composé de formule Py—CHO (III) ou un dérivé fonctionnel réactif de ce composé avec un composé de formule

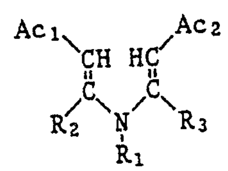

(IV)

ou un tautomère d'un tel composé ou un mélange de tautomères correspondants, ou bien

c) dans un composé de formule

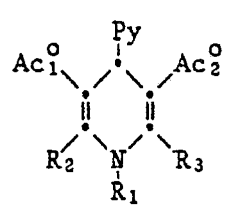

(V)

dans laquelle l'un des symboles $Ac_1^o$ et $Ac_2^o$ représente un reste convertible respectivement en le reste $Ac_1$ ou $Ac_2$, et l'autre représente $Ac_1$ ou $Ac_2$ respectivement ou un reste convertibe en $Ac_1$ ou $Ac_2$ respectivement, on convertit le reste $Ac_1^o$ et/ou $Ac_2^o$ en un reste $Ac_1$ et/ou $Ac_2$, ou bien

d) dans un composé de formule

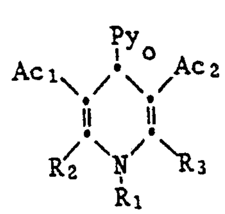

(VI)

dans laquelle $Py_o$ représente un reste pyridyle non substitué ou substitué, on oxyde le reste $Py_o$ en le reste N-oxydo-pyridyle correspondant,
la formation des composés de formule I dans laquelle $R_2$ ou $R_3$, étant un groupe alkyle inférieur en $C_1$—$C_7$, forme avec le radical acyle voisin $Ac_1$ ou $Ac_2$ respectivement, un reste 2-oxa-1-oxo-alkylène inférieur en $C_2$—$C_3$ dont le groupe carbonyle est relié avec l'atome de carbone cyclique en position 3 ou 5 du cycle 1,4-dihydropyridine, à partir de composés de départ de formules II, IV, V et VI, dans lesquelles $R_2$ ou $R_3$ respectivement représente un groupe alkyle inférieur contenant un groupe hydroxy libre ou estérifié, et $Ac_1$ ou $Ac_2$, ou bien $Ac_1^o$ et $Ac_2^o$ représente, dans les composés de départ de formule V, un groupe carboxyle estérifié, pouvant être effectuée directement dans les conditions de réaction de l'un des procédés a) à d), les symboles Py, $R_1$, $R_2$, $R_3$, $Ac_1$ et $Ac_2$ ayant, dans les composés de départ ci-dessus de formules II à IV, lesquels, lorsqu'ils sont capables de former des sels, peuvent également être utilisés à l'état de sels, les significations indiquées en référence à la formule I, et si on le désire, on convertit un composé obtenu répondant à la formule I en un autre composé de formule I, et/ou, si on le désire, on convertit un sel obtenu en le composé libre ou en un autre sel, et/ou, si on le désire, on convertit un composé de formule I obtenu à l'état libre et capable de former des sels, en un sel, et/ou, si on le désire, on sépare un mélange d'isomères obtenu en les isomères individuels.

2. Procédé selon la revendication 1, pour préparer des composés de formule I dans laquelle Py, $R_1$, $Ac_1$ et $Ac_2$ ont les significations indiquées dans la revendication 1, et l'un des symboles $R_2$ et $R_3$ représente un groupe alkyle inférieur en $C_1$—$C_7$ et l'autre l'hydrogène, un groupe alkyle inférieur en $C_1$—$C_7$, hydroxyalkyle inférieur en $C_1$—$C_7$, (alcoxy inférieur en $C_1$—$C_7$)-alkyle inférieur en $C_1$—$C_7$, halogénoalkyle inférieur en $C_1$—$C_7$, (alcoxy inférieur en $C_1$—$C_7$)-carbonyl-alkyle inférieur en $C_1$—$C_7$, carbamoyl-(alkyle inférieur en $C_1$—$C_7$), N-(alkyle inférieur en $C_1$—$C_7$)-carbamoyl-(alkyle inférieur en $C_1$—$C_7$)-, N,N-di-(alkyle inférieur en $C_1$—$C_7$)-carbamoyl-(alkyle inférieur en $C_1$—$C_7$), cyano-(alkyle inférieur en $C_1$—$C_7$), aminoalkyle inférieur en $C_1$—$C_7$, (alkyle inférieur en $C_1$—$C_7$)-amino-alkyle inférieur en $C_1$—$C_7$, di-(alkyle inférieur en $C_1$—$C_7$)-amino-alkyle inférieur en $C_1$—$C_7$, (alkylène inférieur en $C_4$—$C_6$)-amino-alkyle inférieur en $C_1$—$C_7$, morpholino-alkyle inférieur en $C_1$—$C_7$, thiomorpholino-alkyle inférieur en $C_1$—$C_7$, pipérazino-alkyle inférieur en $C_1$—$C_7$, 4-(alkyle inférieur en $C_1$—$C_7$)-pipérazino-alkyle inférieur en $C_1$—$C_7$, (alcoxy inférieur en $C_1$—$C_7$)-carbonyle, carbamoyle, N-(alkyle inférieur en $C_1$—$C_7$)-carbamoyle, N,N-di-(alkyle inférieur en $C_1$—$C_7$)-carbamoyle, cyano, amino, alkylamino inférieur en $C_1$—$C_7$, di-(alkyle inférieur en $C_1$—$C_7$)-amino-(alkyle inférieur en $C_1$—$C_7$)-amino, (alkylène inférieur en $C_4$—$C_6$)-amino-(alkyle inférieur en $C_1$—$C_7$)-amino, morpholino-(alkyle inférieur en $C_1$—$C_7$)-amino, di-(alkyle inférieur en $C_1$—$C_7$)-amino, (alkylène inférieur en $C_4$—$C_6$)-amino, (2-oxo-1-imidazolidinyl)-pipéridino, morpholino, thiomorpholino, pipérazino, 4-(alkyle inférieur en $C_1$—$C_7$)-pipérazino, 4-(alcanoyle inférieur en $C_1$—$C_7$)-pipérazino, 4-benzoyl-pipérazino, 4-furoyl-pipérazino ou 4-thiénoyl-pipérazino, un groupe amino $R_2$ ou $R_3$ pouvant également être relié avec un groupe alkyle inférieur en inférieur en $C_1$—$C_7$ $R_1$ et former avec ce dernier un groupe 1-aza-(alkylène inférieur en $C_3$—$C_4$) dont l'atome d'azote est relié à l'atome de carbone cyclique en position 2 ou 6 respectivement du cycle 1,4-dihydropyridine, ou bien, lorsque $R_2$ ou $R_3$ représente un groupe alkyle inférieur en $C_1$—$C_7$, celui-ci peut former avec le radical acyle voisin $Ac_1$ ou $Ac_2$ respectivement un groupe 2-oxa-1-oxo-(alkylène inférieur en $C_2$—$C_3$) dont le groupe carbonyle est relié à l'atome de carbone cyclique en position 3 ou 5 respectivement du cycle 1,4-dihydropyridine, ou des sels de ces composés portant des groupes aptes à une salification.

3. Procédé selon la revendication 1, pour préparer les composés de formule I dans laquelle Py représente un groupe N-oxydopyridyle non substitué ou substitué par un groupe alkyle inférieur en $C_1$—$C_4$ ou alkylsulfinyle inférieur en $C_1$—$C_7$, $R_1$ représente l'hydrogène, un groupe alkyle inférieur en $C_1$—$C_4$, 2-di-(alkyle inférieur en $C_1$—$C_7$)-amino-alkyle inférieur en $C_1$—$C_7$, 2-(alkylène inférieur en $C_4$—$C_6$)-amino-alkyle inférieur en $C_1$—$C_7$, ou 2-(4-morpholino)-alkyle inférieur en $C_1$—$C_7$, l'un des symboles $R_2$ et $R_3$ représente un groupe alkyle inférieur en $C_1$—$C_4$ et l'autre l'hydrogène, un groupe alkyle inférieur en $C_1$—$C_7$, hydroxyalkyle inférieur en $C_1$—$C_7$, halogénoalkyle inférieur en $C_1$—$C_7$, (alcoxy inférieur en $C_1$—$C_7$)-carbonylalkyl inférieur en $C_1$—$C_7$, cyano(alkyle inférieur en $C_1$—$C_7$), di-(alkyle inférieur en $C_1$—$C_7$)-amino-alkyle inférieur en $C_1$—$C_7$, (alcoxy inférieur en $C_1$—$C_7$)-carbonyle, cyano, amino, 4-morpholino-(alkyle inférieur en $C_1$—$C_7$)-amino, (alkylène inférieur en $C_4$—$C_6$)-amino, (2-oxo-1-imidazolidinyl)-pipérazino ou bien 4-(2-oxo-1-imidazolidinyl)-pipérazino ou bien 4-(2-furoyl)-pipérazino, un groupe amino $R_2$ ou $R_3$ pouvant être relié à un groupe alkyle inférieur en $C_1$—$C_7$ $R_1$ et former avec ce dernier un groupe 1-aza-alkylène inférieur dont l'atome d'azote du groupe aza est relié à l'atome de carbone cyclique en position 2 ou 6 du cycle 1,4-dihydropyridine, ou bien, lorsque $R_2$ ou $R_3$ représente un groupe alkyle inférieur en $C_1$—$C_7$, celui-ci peut former avec le radical acyle voisin $Ac_1$ ou $Ac_2$ respectivement un groupe 2-oxa-1-oxo-alkylène inférieur dont le groupe carbonyle est relié à l'atome de carbone cyclique en position 3 ou 5 du cycle 1,4-dihydropyridine et chacun des symboles $Ac_1$ et $Ac_2$ représente, indépendamment l'un de l'autre, un groupe alcanoyle inférieur, (alkyle inférieur en $C_1$—$C_7$)-sulfonyle, (alcoxy inférieur en $C_1$—$C_7$)-carbonyle, hydroxy-(alcoxy inférieur en $C_1$—$C_7$)-carbonyle, (alcoxy inférieur en $C_1$—$C_7$)-(alcoxy inférieur en $C_1$—$C_7$)-carbonyle, di-(alkyle inférieur en $C_1$—$C_7$)-amino-(alcoxy inférieur en $C_1$—$C_7$)-carbonyle, N-(alkyle inférieur en $C_1$—$C_7$)-N-phényl-(alkyle inférieur en $C_1$—$C_7$)-amino-(alcoxy inférieur en $C_1$—$C_7$-carbonyle, (alkylène inférieur en $C_4$—$C_6$-amino-(alcoxy inférieur en $C_1$—$C_7$)-carbonyle, (4-morpholino)-(alcoxy inférieur en $C_1$—$C_7$)-carbonyle, carbamoyle, N-(alkyle inférieur en $C_1$—$C_7$)-carbamoyle, N,N-di-(alkyle inférieur en $C_1$—$C_7$)-carbamoyle, N,N-(alkylène inférieur en $C_4$—$C_5$)-carbamoyle ou 4-morpholinocarbonyle, les groupes alkyle inférieurs, alcoxy inférieurs ou alcanoyle inférieurs contenant jusqu'à 4 atomes de carbone inclus, les

groupes alkylène inférieurs 4 ou 5 atomes de carbone dans la chaîne, les groupes 1-aza-alkylène inférieurs 2 ou 3 atomes de carbone dans la chaîne et les groupes 2-oxa-1-oxo-alkylène inférieurs 2 atomes de carbone dans la chaîne, ou des sels de ces composés portant des groupes aptes à une alification.

4. Procédé selon la revendication 1, pour préparer les composés de formule I dans laquelle Py représente un groupe N-oxydopyridyle, $R_1$ représente l'hydrogène, un groupe alkyle inférieur en $C_1$—$C_4$, 2-(di-(alkyle inférieur en $C_1$—$C_7$)-amino)-alkyle inférieur en $C_1$—$C_4$, 2-(alkylène inférieur en $C_4$—$C_6$)-amino)-alkyle inférieur en $C_1$—$C_4$ ou 2-(4-morpholino)-alkyle inférieur en $C_1$—$C_4$, l'un des symboles $R_2$ et $R_3$ représente un groupe alkyle inférieur en $C_1$—$C_4$ et l'autre un groupe alkyle inférieur en $C_1$—$C_4$, hydroxy-alkyle inférieur en $C_1$—$C_7$, halogénoalkyle inférieur en $C_1$—$C_7$, 2-(di(alkyle inférieur en $C_1$—$C_7$)-amino)-alkyle inférieur en $C_1$—$C_4$, (alcoxy inférieur en $C_1$—$C_7$)-carbonyle, cyano, amino, (4-morpholino)-(alkyle inférieur en $C_1$—$C_7$)-amino, (alkylène inférieur en $C_4$—$C_6$)-amino, (2-oxo-1-imidazolidinyl)-(alkylène inférieur en $C_4$—$C_6$)-amino ou 4-(2-furoyl)-pipérazino, un groupe amino $R_2$ ou $R_3$ pouvant être relié à un groupe alkyle inférieur en $C_1$—$C_4$ $R_1$ et former avec celui-ci un groupe 1-aza-(alkylène inférieur en $C_2$—$C_3$) dont l'atome d'azote du groupe aza est relié à l'atome de carbone cyclique en position 2 ou 6 du cycle 1,4-dihydro-pyridine, ou bien, lorsque $R_2$ ou $R_3$ représente un groupe alkyle inférieur en $C_1$—$C_7$, celui-ci peut former avec le radical acyle voisin $Ac_1$ ou $Ac_2$ respectivement un groupe 2-oxa-1-oxo-(alkylène inférieur en $C_2$—$C_3$) dont le groupe carbonyle est relié à l'atome de carbone cyclique en position 3 ou 5 du cycle 1,4-dihydropyridine, et chacun des symboles $Ac_1$ et $Ac_2$ représente, indépendamment de l'autre, un groupe alcanoyle inférieur en $C_1$—$C_4$, alkylsulfonyle inférieur en $C_1$—$C_7$, (alcoxy inférieur en $C_1$—$C_7$)-carbonyle, 2-(alcoxy inférieur en $C_1$—$C_4$)-(alcoxy inférieur en $C_1$—$C_4$)-carbonyle, di-(alkyle inférieur en $C_1$—$C_7$)-amino-(alcoxy inférieur en $C_1$—$C_7$)-carbonyle ou N-(alkyle inférieur en $C_1$—$C_4$)-N-phényl-(alkyle inférieur en $C_1$—$C_4$)-amino-(alcoxy inférieur en $C_1$—$C_7$)-carbonyle, carbamoyle, N-(alkyle inférieur en $C_1$—$C_4$)-carbamoyle, N,N-di-(alkyle inférieur en $C_1$—$C_7$)-carbamoyle ou 4-morpholino-carbonyle, les groupes alkyle inférieurs, alcoxy inférieurs et alcanoyle inférieurs contenant jusqu'à 4 atomes de carbone incus, les groupes alkylène inférieurs 4 ou 5 atomes de carbone dans la chaîne, les groupes 1-aza-alkylène inférieurs 2 ou 3 atomes de carbone dans la chaîne et le groupe 2-oxa-1-oxo-alkylène inférieur 2 atomes de carbone dans la chaîne, ou des sels de ces composés portant des groupes basiques salifiables.

5. Procédé selon la revendication 1, pour préparer des composés de formule I dans laquelle Py représente un groupe N-oxydopyridyle, $R_1$ représente l'hydrogène ou un groupe 2-(4-morpholino)-éthyle, l'un des symboles $R_2$ et $R_3$ représente un groupe méthyle et l'autre un groupe méthyle, hydroxyméthyle, (alcoxy inférieur en $C_1$—$C_4$)-carbonyle, par exemple méthoxycarbonyle, éthoxycarbonyle, n-propyloxycarbonyle, isopropyloxycarbonyle, n-butyloxycarbonyle, ou isobutyloxycarbonyle, cyano ou amino, et chacun des symboles $Ac_1$ et $Ac_2$ représente, indèpendamment de l'autre, un groupe alcanoyle inférieur, par exemple acétyle, alkylsulfonyle, inférieur en $C_1$—$C_7$, par exemple éthylsulfonyle, (alcoxy inférieur)-carbonyle, par exemple méthoxycarbonyle, éthoxycarbonyle, n-propyloxycarbonyle, isopropyloxycarbonyle, n-butyloxycarbonyle ou isobutyloxycarbonyle, carbamoyle, N-(alkyle inférieur)-carbamoyle, par exemple N-mèthylcarbamoyle, ou N,N-di-(alkyle inférieur)-carbamoyle, par exemple N,N-diméthyl-carbamoyle, les groupes alkyle inférieurs, alcoxy inférieurs et alcanoyle inférieurs contenant jusqu'à 4 atomes de carbone inclus, ou des sels de ces composés portant des groupes basiques salifiables.

6. Procédé selon la revendication 1, pour préparer le 2,6 - diméthyl - 4 - (1 - oxydo - 3 - pyridyl) - 1,4 - dihydro - pyridine - 3,5 - dicarboxylate de diméthyle.

7. Procédé selon la revendication 1, pour préparer le 2,6 - diméthyl - 4 - (1 - oxydo - 3 - pyridyl) - 1,4 - dihydro - pyridine - 3,5 - dicarboxylate de diéthyle.

8. Procédé selon la revendication 1, pour préparer le 2,6 - diméthyl - 4 - (1 - oxydo - 3 - pyridyl) - 1,4 - dihydro - pyridine - 3 - carboxylate d'isobutyle, 5 - carboxylate de méthyle.

9. Procédé selon la revendication 1, pour préparer le 2,6 - diméthyl - 4 - (1 - oxydo - 2 - pyridyl) - 1,4 - dihydro - pyridine - 3,5 - dicarboxylate de diéthyle.

10. Procédé selon la revendication 1, pour préparer le 2,6 - diméthyl - 4 - (1 - oxydo - 4 - pyridyl) - 1,4 - dihydro - pyridine - 3,5-dicarboxylate de diéthyle.

11. Procédé selon la revendication 1, pour préparer le 2,6 - diméthyl - 4 - (1 - oxydo - 3 - pyridyl) - 1,4 - dihydro - pyridine - 3,5 - dicarboxylate de di - n - propyle.

12. Procédé selon la revendication 1, pour préparer le 2,6 - diméthyl - 4 - (2 - méthyl - 1 - oxydo - 3 - pyridyl) - 1,4 - dihydro - pyridine - 3,5 - dicarboxylate de diéthyle.

13. Procédé selon la revendication 1, pour préparer le 2,6 - diméthyl - 4 - (2 - chloro - 1 - oxydo - 3 - pyridyl) - 1,4 - dihydro - pyridine - 3,5 - dicarboxylate de diéthyle.

14. Procédé selon la revendication 1, pour préparer le 2,6 - diméthyl - 4 - (1 - oxydo - 3 - pyridyl) - 1,4 - dihydro - pyridine - 3 - carboxylate d'isopropyl,5 - carboxylate de 2 - méthoxyéthyle.

15. Procédé selon la revendication 1, pour préparer le 2,6 - diméthyl - 4 - (1 - oxydo - 3 - pyridyl) - 1,4 - dihydro - pyridine - 3 - carboxylate de méthyle, 5 - carboxylate de 2 - (N - méthyl - N - benzyl - amino) - éthyle.

16. Procédé selon la revendication 1, pour préparer le 6 - méthoxyméthyl - 2 - méthyl - 4 - (1 - oxydo - 3 - pyridyl) - 1,4 - dihydro - pyridine - 3 - carboxylate d'éthyle,5 - carboxylate de méthyle.

17. Procédé selon la revendication 1, pour préparer le 2 - amino - 6 - méthyl - 4 - (1 - oxydo - 3 - pyridyl) - 1,4 - dihydro - pyridine - 3,5 - pyridine - dicarboxylate de diéthyle.

18. Procédé selon la revendication 1, pour préparer le 2,6 - diméthyl - 4 - (2 - méthylthio - 1 -

oxydo - 3 - pyridyl) - 1,4 - dihydro - pyridine - 3,5 - dicarboxylate de diéthyle.

19. Procédé selon la revendication 1, pour préparer le 2,6 - diméthyl - 4 - (2 - méthyl - 1 - oxydo - 3 - pyridyl) - 1,4 - dihydro - pyridine - 3 - carboxylate d'isobutyle, 5 - carboxylate de méthyle.

20. Procédé selon la revendication 1, pour préparer le 6 - hydroxyméthyl - 2 - méthyl - 4 - (1 - oxydo - 2 - méthyl - 3 - pyridyl) - 1,4 - dihydro - pyridine - 3,5 - dicarboxylate de diéthyle.

21. Procédé selon la revendication 1, pour préparer le 6 - cyano - 2 - méthyl - 4 - (1 - oxydo - 2 - méthyl - 3 - pyridyl) - 1,4 - dihydro - pyridine - 3,5 - dicarboxylate de diéthyle.

22. Procédé selon la revendication 1, pour préparer le 2,6 - diméthyl - 4 - (1 - oxydo - 2 - méthoxy - 3 - pyridyl) - 1,4 - dihydro - pyridine - 3,5 - dicarboxylate de diéthyle.

23. Procédé selon la revendication 1, pour préparer le 2,6 - diméthyl - 4 - (2 - méthylsulfinyl - 1 - oxydo - 3 - pyridyl) - 1,4 - dihydro - pyridine - 3,5 - dicarboxylate de diéthyle.

24. Procédé selon la revendication 1, pour préparer le 2 - méthyl - 4 - (1 - oxydo - 3 - pyridyl) - 5 - oxo - 1,4,5,7 - tétrahydrofuro[3,4 - b]pyridine - 3 - carboxylate d'éthyle.

25. Procédé selon la revendication 1, pour préparer le 2 - méthyl - 4 - (1 - oxydo - 2 - chloro - 3 - pyridyl) - 5 - oxo - 1,4,5,7 - tétrahydrofuro[3,4 - b]pyridine - 3 - carboxylate d'éthyle.

26. Procédé selon la revendication 1, pour préparer le 2 - méthyl - 4 - (1 - oxydo - 2 - méthyl - 3 - pyridyl) - 5 - oxo - 1,4,5,7 - tétrahydrofuro[3,4 - b]pyridine - 3 - carboxylate d'éthyle.

27. Procédé selon la revendication 1, pour préparer le 2 - méthyl - 4 - (1 - oxydo - 2 - méthylthio - 3 - pyridyl) - 5 - oxo - 1,4,5,7 - tétrahydrofuro[3,4 - b]pyridine - 3 - carboxylate d'éthyle.

28. Procédé de préparation de compositions pharmaceutiques, caractérisé en ce que l'on met des composés préparés selon les revendications 1 à 27, éventuellement en mélangeant des produits auxilaires, sous la forme de compositions pharmaceutiques.